# EUROPEAN PATENT SPECIFICATION

(11) **EP 3 943 492 B1**
(45) Date of publication and mention of the grant of the patent: **08.05.2024**
(21) Application number: 20461551.2
(22) Date of filing: 24.07.2020
(51) Int. Cl.: C07D 413/14, C07D 401/14, C09K 11/06, H05B 33/10, C09B 57/00, C09B 19/00, H10K 85/60, H10K 101/20

(54) **TADF MATERIALS COMPRISING 4-(3-(2-(10H-PHENOXAZIN-10-YL)PYRIDIN-5-YL)-9H-CARBAZOL-9-YL)BENZONITRILE DERIVATIVES AND RELATED COMPOUNDS FOR USE IN OLEDS**
TADF MATERIALIEN UMFASSEND 4-(3-(2-(10H-PHENOXAZIN-10-YL)PYRIDIN-5-YL)-9H-CARBAZOL-9-YL)BENZONITRIL-DERIVATE UND ÄHNLICHE VERBINDUNGEN ZUR VERWENDUNG IN OLEDS
MATÉRIAUX TADF COMPRENANT DÉRIVÉS DE 4-(3-(2-(10H-PHENOXAZIN-10-YL)PYRIDIN-5-YL)-9H-CARBAZOL-9-YL)BENZONITRILE ET COMPOSÉS SIMILAIRES À UTILISÉ DANS DES OLEDS

(43) Date of publication of application: 26.01.2022
(73) Proprietor: Noctiluca S.A., 81-100 Torun (PL)
(72) Inventor: Trzaska, Piotr, 87-134 Zlawies Wielka (PL); Bosiak, Mariusz, 87-100 Torun (PL); Zielinska, Alicja, 87-313 Brzozie (PL); Rakowiecki, Marcin, 87-100 Torun (PL); Wolan, Andrzej, 87-100 Torun (PL)
(74) Representative: Kowalkiewicz, Zuzanna

(56) References cited:
- EP-A1- 3 018 727
- WO-A1-2013/162284
- WO-A1-2015/016498
- WO-A1-2016/068446
- WO-A1-2020/042626
- CN-A- 106 883 240
- CN-A- 106 938 999
- CN-A- 110 790 782
- CN-A- 110 835 340
- JP-A- 2020 007 239
- KR-A- 20150 093 995
- KR-A- 20150 105 201
- KR-A- 20190 071 971
- HWANG JINHYO ET AL: "Structural isomers of 9-(pyridin-2-yl)-9H-carbazole in combination with 9'H-9,3':6',9''-tercarbazole and their application to high efficiency solution processed green TADF OLEDs", DYES AND PIGMENTS, ELSEVIER APPLIED SCIENCE PUBLISHERS BARKING, GB, vol. 179, 4 April 2020 (2020-04-04), XP086147085, ISSN: 0143-7208, DOI: 10.1016/J.DYEPIG.2020.108403 [retrieved on 2020-04-04]

## Description

### TECHNICAL FIELD

The present invention relates to donor structures suitable for use in organic thermally activated delayed fluorescence (TADF) material(s).

### BACKGROUND

During the electrical excitation of fluorescent emitter only 25% of excitons have singlet state and are able to radiative decay. The other 75% of excitons are trapped in triplet states from which radiative emission is forbidden by selection rules. The second generation of OLED emitters, phosphorescent ones, containing in their structures iridium or platinum, are able to harvest triplet excitons due to intersystem crossing (ISC) but they have also some drawbacks such as burn-in effect, lack of efficient and stable blue emitter or environmental pollution with heavy metals.

Thermally activated delayed fluorescence (TADF) is one of the most intriguing discoveries towards realization of light-emitting devices such as highly efficient organic light-emitting diodes (OLED) utilizing small organic molecules as emitters.

There are known materials exhibiting the thermally activated delayed fluorescence, called herein interchangeably TADF molecules, TADF compounds, TADF emitters or TADF materials, bearing different design concepts which have been developed with the aim to boost their light-emitting characteristic. Among various emitters, the TADF compounds are used i.a. in OLED applications due to their near 100% internal efficiency in these diodes.

Typically, the TADF compounds are of a type comprising one or more donor moiety(ies) and one or more acceptor moiety(ies) linked together via a chemical bond. Typically, the TADF compounds are of a donor-acceptor type or a donor-acceptor-donor type (abbrev. D-A or D-A-D).

The TADF compounds need to have considerably small energy gap (ΔE_{ST}) of singlet-triplet excited state to allow effective transfer from excited triplet state to singlet (reverse intersystem crossing) from which radiated emission occurs. To achieve good TADF effect ΔE_{ST} between S₁ and T₁ should be substantially minimal, preferably below 0.1 eV (ΔE_{ST} < 0.1 eV) and oscillator strength substantially maximal, preferably over 0.2.

A small ΔE_{ST} can be accomplished by spatially separating the highest occupied molecular orbital (HOMO) and the lowest unoccupied molecular orbital (LUMO) of the molecule. Nonetheless, localizing the HOMO and LUMO on different parts of the molecule decreases the oscillator strength (f) of the transition between excited S₁ state and S₀ state, where S₀ is a ground state of the molecule, i.e. the ground energy level of the molecule. The lower the oscillator strength (f) value, the smaller the probability of the radiative emission of TADF compound, and thereby limited quantum performance of the TADF material. Hence, designing efficient TADF molecules requires careful molecular engineering.

Form the prior art, there are known various structures comprised in TADF compounds. Among them, aromatic and heterocyclic amines, such as diphenylamine, carbazole, phenoxazine, phenothiazine 5,10-dihydrophenazine, 9,10-dihydro-9,9-dimethylacridine and their derivatives, constitute preferred groups used to build up TADF materials. Alone, the above-mentioned groups usually reveal low efficiency in decreasing the ΔE_{ST} parameter of the TADF molecule, which translates into limited quantum efficiency of TADF emitters based on them, because some excitons remain trapped in the excited triplet state and return to the ground state by non-radiative decay, thereby without the expected certain radiative emission.

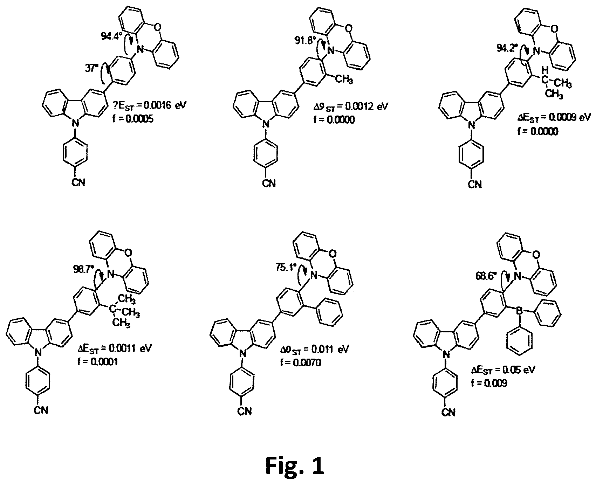

Further, it is known that preferred lower levels of ΔE_{ST} can be achieved by combining several of the above-mentioned aromatic and heterocyclic amine molecules in the donor structure of the emitter, as shown in Fig.1. Such systems are of substantially low ΔE_{ST} values due to good separation of the HOMO and LUMO orbits. Nevertheless, this causes a strong decrease in the value of the oscillator strength (*f*) parameter, which is responsible for the probability of radiative emission, hence, this also lowers the emission.

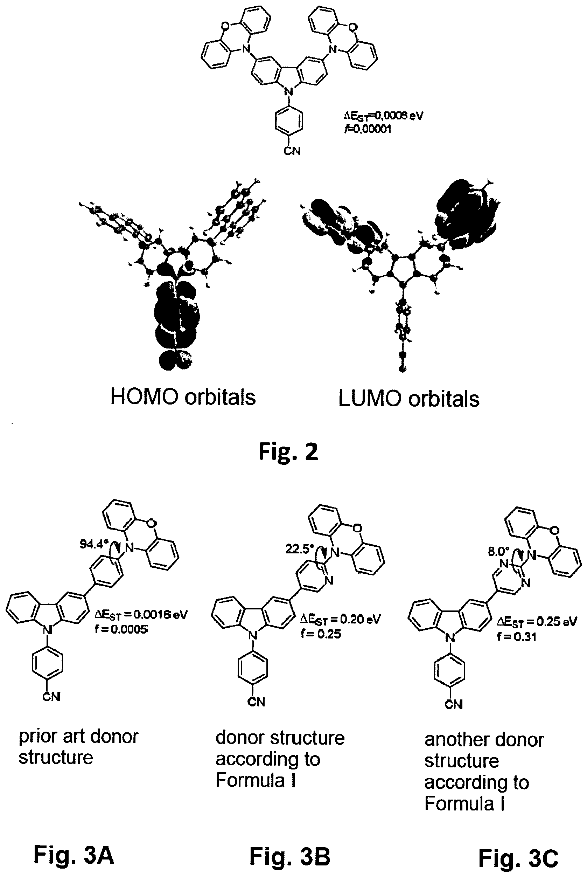

Moreover, it is assumed that the most probable reason for the complete separation of the HOMO and LUMO orbitals, and hence the decrease in the oscillator strength value (f), is large torsion, i.e. dihedral angle, between the heterocycle moieties in the molecule. For example, as schematically shown in Fig. 2, the dihedral angle between phenoxazine and carbazole moieties in 4-(3,6-di(10*H*-Phenoxazin-10-yl)-9*H-*carbazol-9-yl)benzonitrile is about 90°; the left structure represents HOMO orbitals of this molecule and the right structure represents LUMO orbitals of this molecule. Nonetheless, a separation of heterocyclic amines by a phenylene ring only slightly improves oscillator strength, which can be derived upon a comparison of Fig. 1 and Fig. 2 - the compounds with and without phenylene ring, respectively. Also, the introduction of bulky alkyl or aromatic groups on the phenylene ring to reduce the dihedral angle does not significantly improve the oscillator strength parameter, as follows from data of ΔE_{ST} and f in Fig. 1.

Form the patent literature there are also known various TADF emitters.

A Korean patent publication KR2011079402 describes an organic light emitting compound of the structure consisting of carbazole with a heterocyclic substituent selected from oxazolyl group, thiazolyl, triazolyl group, tetrazolyl, oxadiazolyl group, pyridinyl group, pyridazinyl group, pirymidynyl and pyrazinyl system. Nonetheless, in this compound, the position of the aryl substituent is not determined as providing any substantial effect.

An international patent application WO2018080067 describes organic compounds exhibiting improved electroluminescence and stability. The compounds comprise a structure in which a basic skeleton is formed by phenoxazine or phenothiazine bonded with carbazole moiety. The HOMO and LUMO energy levels of the molecule can be controlled by selection of substituents being heterocyclic moieties comprising nitrogen, such as pyridine, pyrimidine or triazine, bounded to the basic skeleton. This provides an increase in interaction between holes and electrons in the material when used as emitter in emitting diodes. Thus, in these compounds, neither pyridine nor pyrimidine moiety is used as a linker/spacer between phenoxazine and carbazole.

An international WO2019046734 patent application describes compounds being TADF emitters. The compounds consist of donor moiety and acceptor moiety to provide the electron transfer from HOMO to LUMO orbital of the acceptor moiety. These compounds are Adachi type emitters containing in center of the structure pyridine ring, instead of benzene, and several donors. The central pyridine modified with electron withdrawing group serves as an integral part of electron acceptor which is multiply substituted at any possible position. Thereby, the pyridine ring within these compounds is not an integral part of the donor structure.

A Chinese patent publication CN109494308 describes a display of layered structure comprising donor material, acceptor material, and donor-acceptor material. The acceptor material consists of compounds comprising a heterocyclic moiety linked to the carbazole through its atom number 2. This chemical configuration provides an increase in the degree of freedom in the selection of the exciplex material.

Moreover, the patent application CN110835340A provides a novel organic electroluminescent material and an organic electroluminescent device using the same. The source of light is carbazole as a donor group that guarantees a good stability and lifting performance. Another Chinese patent publication CN106938999A presents a triazole compound and a light emitting device using the triazole compound.

The European patent application EP3018727A1 reveals the organic light emitting display device comprises an anode, an organic layer on the anode, and a cathode on the organic layer, wherein the organic layer comprises a compound including a carbazole group having hole characteristics and a heteroaryl group having electron characteristics.

Moreover, the Korean patent application KR 20150105201A1 shows an organic optical device and an organic optical compound comprises a carbazole group and a heteroaryl group having.

KR 20150093995A presents a polymer compound comprising carbazole to improve light emission efficiency and reliability of an element and to extend the lifespan of the element, to an organic electronic element using the same, and to an electronic device thereof. The document WO 2015/016498 A1 describes heterocyclic compound comprising carbazole and an organic light-emitting device comprising the same.

Furthermore, WO 2020/042626 A1 reveals an organic electroluminescent device and a manufacturing method therefor, and a display device. The organic electroluminescent device comprises an organic light-emitting layer comprising a carbazole structure, wherein the organic light-emitting layer comprises a host material, a sensitizer material, and a resonant thermally activated delayed fluorescent material; the host material is a wide-bandgap material.

The Chinese patent application CN 110790782 A shows the dark blue organic light-emitting material containing the B/Bi-N host structure of the present invention achieves the separation of HOMO and LUMO by enhancing the resonance effect between atoms in the material, so that it has a very narrow emission spectrum, and at the same time It has TADF properties. Thus, its colour purity of blue light is improved, and its stability is enhanced.

The Japanese patent application JP 2020007239 A provides a pyrimidine derivative as a thermal activation delayed fluorescence (TADF) material capable of performing efficiently up-conversion from a triplet exciton to a singlet exciton.

A patent application KR 20190071971A reveals an organic light emitting diode including: a light emitting material layer including first to third layers; a positive electrode located at one side of the light emitting material layer; and a negative electrode disposed on the other side of the light emitting material layer to face the positive electrode. The first layer includes a TADF dopant, the second layer includes a first fluorescent dopant, and the third layer includes a TADF dopant.

CN 106883240A discloses a tri-s-triazine compound that possess a D-pion-A structure and keeps TADF performance. Furthermore, substituent groups contain aromatic heterocyclic structures. The molecules have asymmetrical structures, thus, are not prone to crystallizing and have good film forming property. The invention further provides a luminescent device using the tri-s-triazine compound.

The article of HWANG JINHYO ET AL titled "Structural isomers of 9-(pyridin-2-yl)-9H- carbazole in combination with 9'H-9,3':6',9"-tercarbazole and their application to high efficiency solution processed green TADF OLEDs" (DYES AND PIGMENTS, ELSEVIER APPLIED SCIENCE PUBLISHERS BARKING, GB, vol. 179, 4 April 2020 (2020-04-04), XP086147085, ISSN: 0143-7208, DOI: 10.1016/J.DYEPIG.2020.108403) states that pyridine can be the electron-deficient core within CzPy₂TCz and CzPy₃TCz derivatives as electron-rich units.

The international patent application WO 2016/068446 A1 provides is an organic optoelectronic diode and a display device comprising the organic optoelectronic diode, the organic optoelectronic diode comprising: an anode and a cathode facing each other; an emission layer located between the anode and the cathode; an electron transport layer located between the cathode and the emission layer; and an auxiliary electron transport layer located between the electron transport layer and the emission layer.

The patent application WO 2013/162284 A1 relates to a novel organic electroluminescent compound and an organic electroluminescent device comprising the same. The organic electroluminescent compound comprising carbazole structure having high green luminous efficiency and superior material lifespan characteristics, compared with conventional phosphorescent host materials, and thus can provide an organic electroluminescent device which is excellent in operational lifespan, and induces increased power efficiency and improves power consumption.

As follows from the above-mentioned patent literature, donor structures, as well as acceptor structures, building up TADF compounds, undergo continuous development in order to achieve better characteristics of the TADF materials to be used in various applications, e.g. light-emitting devices, such as diodes including OLEDs.

Therefore, it would be desirable to further develop the donor structures to be used for the production of TADF emitters with improved characteristics.

### SUMMARY OF THE DISCLOSURE

A thermally activated delayed fluorescence (TADF) compound comprising one structure of donor-acceptor type or donor-acceptor-donor type selected from the group consisting of: 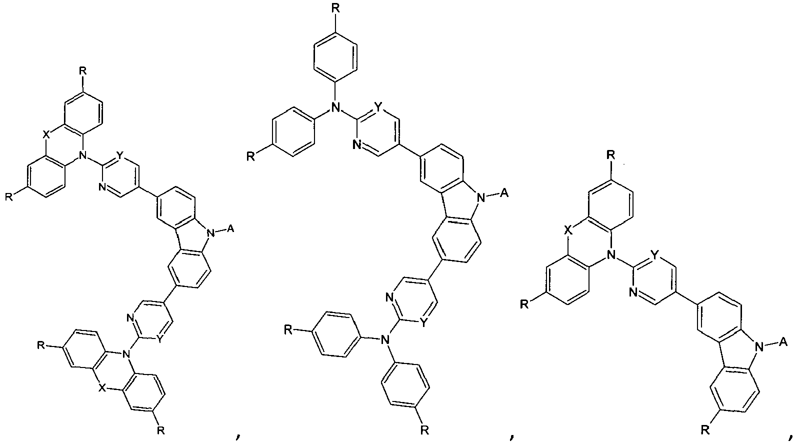 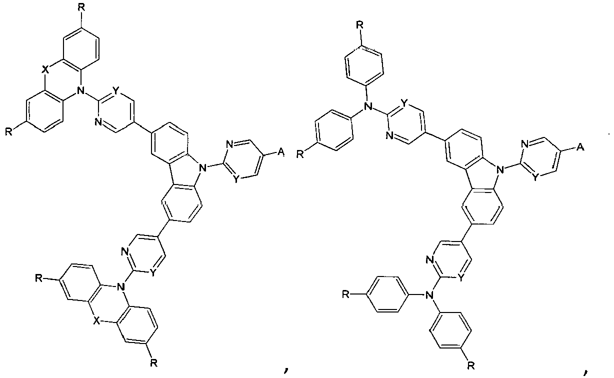 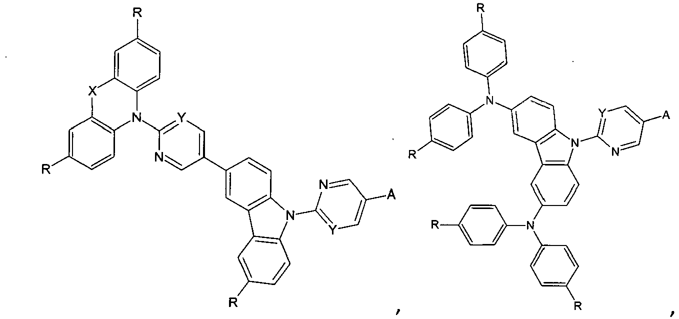 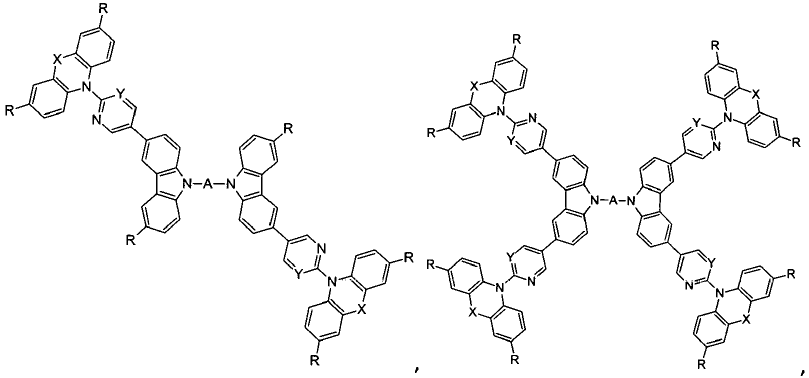 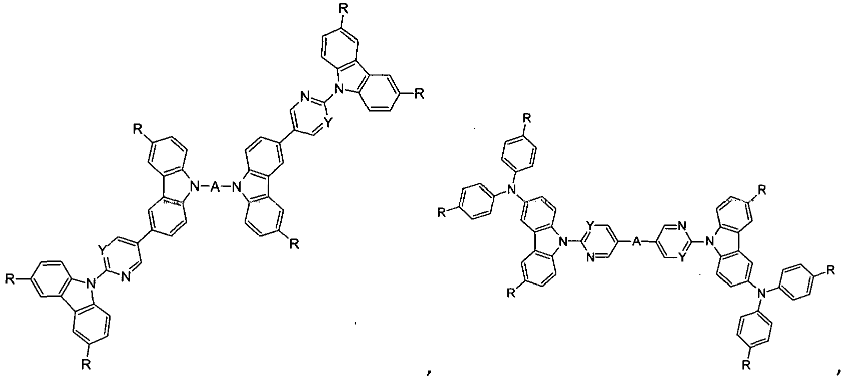 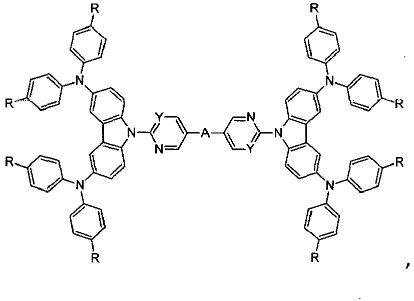 wherein:
Y is independently selected from carbon and nitrogen atom,
R is independently selected from the group consisting of H, alkyl, alkenyl, alkynyl, aryl and heteroaryl,
X is independently selected from the group consisting of O, S, N(R) and C(CH₃)₂. A is the acceptor moiety A for the donor-acceptor type selected from the group consisting of: cyano (CN) group, benzonitrile, phthalonitrile, isophthalonitrile, terephthalonitrile isonicotinonitrile, benzene-1,3,5-tricarbonitrile, diphenyl sulfone, benzophenone, 1,4-dibenzoylbenzene, 1,3-bis(phenylsulfonyl)benzene, 1,4-bis(phenylsulfonyl)benzene, 2,4,6-triphenyl-1,3,5-triazine, pyrimidine, 2-phenylpyrimidine, 2-methylpyrimidine, pyridine-3,5-dicarbonitrile, 4H-124-triazole, 1,3,4-oxadiazole, 1,3,4-thiadiazole, benzo[d]thiazole, benzo[1,2-d:4,5-d']bis(oxazole), benzo[1,2-d:5,4-d']bis(oxazole), quinoxaline, 1H-benzo[d]imidazole, dibenzo[f,h]quinoxaline, anthracene-9,10-dione, 9H-thioxanthene-9-one 10,10-dioxide, 9,9-dimethyl-9H-thioxanthene 10,10-dioxide, 10,10-dimethylanthracen-9(10H)-one, 5H-cyclopenta[1,2-b:5,4-b']dipyridine, 9H-fluorene-2,7-dicarbonitrile, dibenzo[f,h]quinoxaline-2,3-dicarbonitrile, thianthrene 5,5,10,10-tetraoxide, pyrazino[2,3-f][1,10]phenanthroline-2,3-dicarbonitrile, triphenylborane, tris(2,6-dimethylphenyl)borane and trimesitylborane;
A is the acceptor moiety A for the donor-acceptor-donor type selected from the group consisting of: benzonitrile, phthalonitrile, isophthalonitrile, terephthalonitrile isonicotinonitrile, benzene-1,3,5-tricarbonitrile, diphenyl sulfone, benzophenone, 1,4-dibenzoylbenzene, 1,3-bis(phenylsulfonyl)benzene, 1,4-bis(phenylsulfonyl)benzene, 2,4,6-triphenyl-1,3,5-triazine, pyrimidine, 2-phenylpyrimidine, 2-methylpyrimidine, pyridine-3,5-dicarbonitrile, 4H-124-triazole, 1,3,4-oxadiazole, 1,3,4-thiadiazole, benzo[d]thiazole, benzo[1,2-d:4,5-d']bis(oxazole), benzo[1,2-d:5,4-d']bis(oxazole), quinoxaline, 1H-benzo[d]imidazole, dibenzo[f,h]quinoxaline, anthracene-9,10-dione, 9H-thioxanthene-9-one 10,10-dioxide, 9,9-dimethyl-9H-thioxanthene 10,10-dioxide, 10,10-dimethylanthracen-9(10H)-one, 5H-cyclopenta[1,2-b:5,4-b']dipyridine, 9H-fluorene-2,7-dicarbonitrile, dibenzo[f,h]quinoxaline-2,3-dicarbonitrile, thianthrene 5,5,10,10-tetraoxide, pyrazino[2,3-f][1,10]phenanthroline-2,3-dicarbonitrile, triphenylborane, tris(2,6-dimethylphenyl)borane and trimesitylborane.

Preferably, the TADF compound is selected from the group consisting of

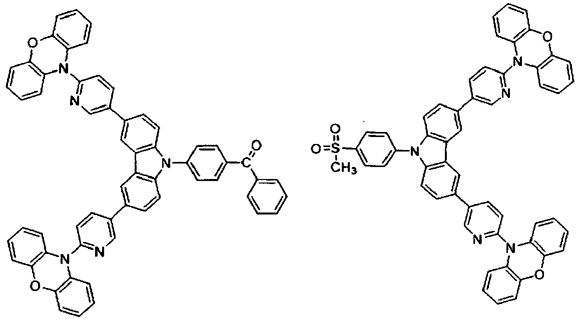

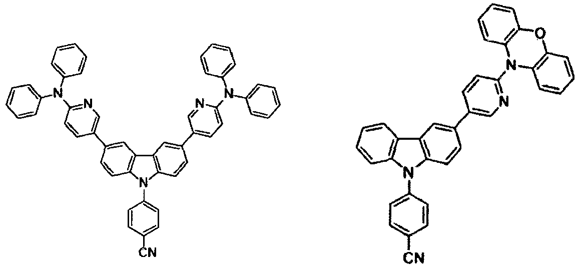

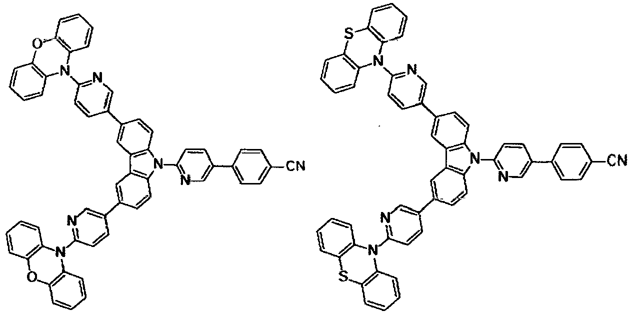

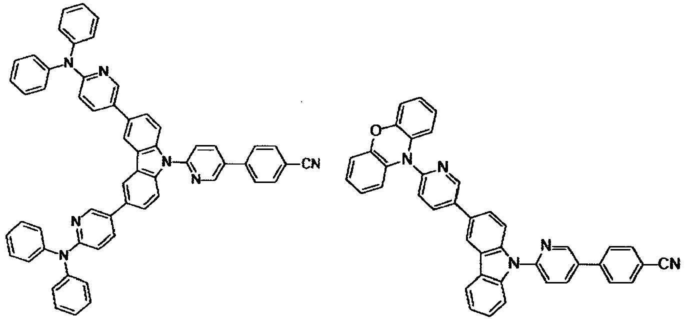

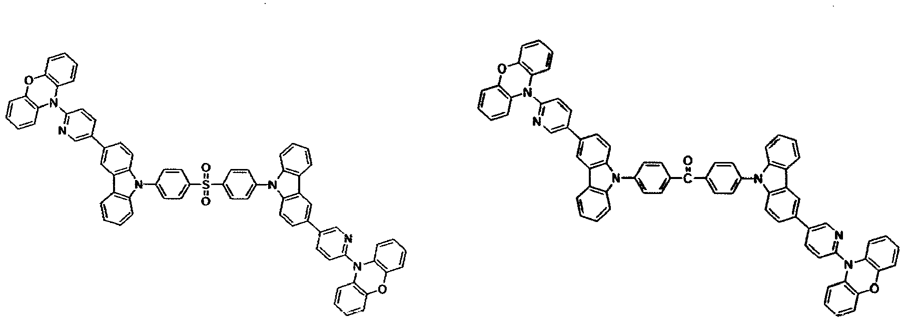

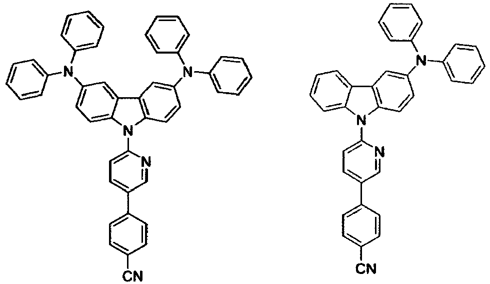

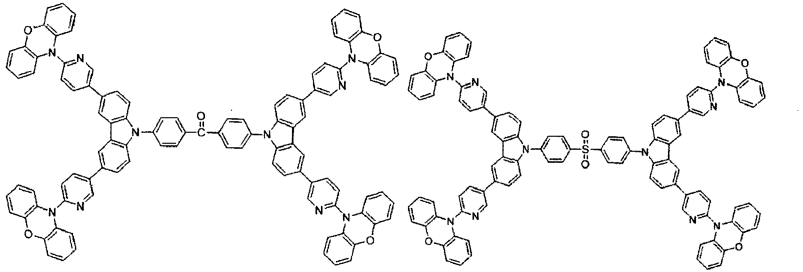

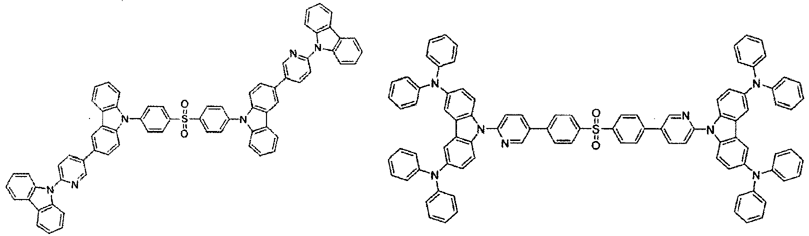

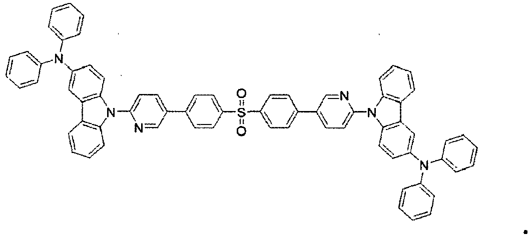

There is further disclosed a light emitting device comprising the compound for TADF as described above.

Preferably, the light emitting device comprises an emissive layer for emitting light comprising the TADF compound as described above comprising the donor structure with the acceptor moiety.

Preferably, the device comprises an anode, a cathode, an electron transport layer and a hole transport layer, and wherein at least one of the electron transport layer and the hole transport layer comprises the TADF compound as described above.

Preferably, the device comprises at least one layer selected from the group consisting of an electron injection layer, a hole injection layer, a barrier layer, a hole barrier layer and an electron barrier layer, and wherein at least one of said layers comprises the TADF compound as described above.

The donor structure according to the present disclosure features reduced dihedral angle and increased oscillator strength (f). Furthermore, upon combining the donor structure with an acceptor structure, hence providing TADF material, it emits light of a wavelength range from 380 nm to 900 nm, depending on the type of connected acceptor structure. Therefore, the present disclosure further relates to a light-emitting device comprising the developed donor structure and the TADF material that comprises this donor structure.

There is further disclosed the use of the TADF compound as described above as a material for thermally activated delayed fluorescence (TADF) in organic light-emitting devices (OLED).

### BRIEF DESCRIPTION OF DRAWINGS

The object of the present disclosure is shown by means of exemplary embodiments in a drawing, wherein:
Fig. 1 presents prior art TADF compounds comprising donor structures with a phenylene internal spacer and an impact of various substituents at the phenylene ring on the value of dihedral angle, ΔE_{ST} and oscillator strength (f) of the donor structures;
Fig. 2 presents prior art TADF compound comprising structure without a phenylene internal spacer and an impact of its absence on the value of dihedral angle, ΔE_{ST} and oscillator strength (f) of the donor structures;
Fig. 3 presents a comparison between the value of dihedral angle, ΔE_{ST} and oscillator strength (f) of two embodiments of TADF compounds comprising donor structures according to the present disclosure and a corresponding prior art TADF compound comprising known donor structure (the effect of heteroatom in the phenylene internal spacer is visible);
Fig. 4A presents embodiments of TADF structures comprising the donor structures, according to the present disclosure; the structures being of donor-acceptor (D-A) type;
Figs 4B - 4C present embodiments of TADF structures comprising the donor structures, according to the present disclosure; the structures being of donor-acceptor-donor (D-A-D) type;
Figs 4D - 4E schematically present arrangements of pyridine and pyrimidine aromatic rings with respect to carbazole moiety;
Figs. 5A - 5C present three embodiments of architectures of light-emitting devices comprising donor structures for TADF material or TADF material comprising the donor structures, according to the present disclosure;
Fig. 6 presents exemplary acceptor structures A which can be combined with the donor structure of Formula I providing the TADF materials;
Fig. 7 presents comparative data for TADF materials comprising the donor structures of the present disclosure and TADF materials comprising the donor structures of the prior art.

### DETAILED DESCRIPTION

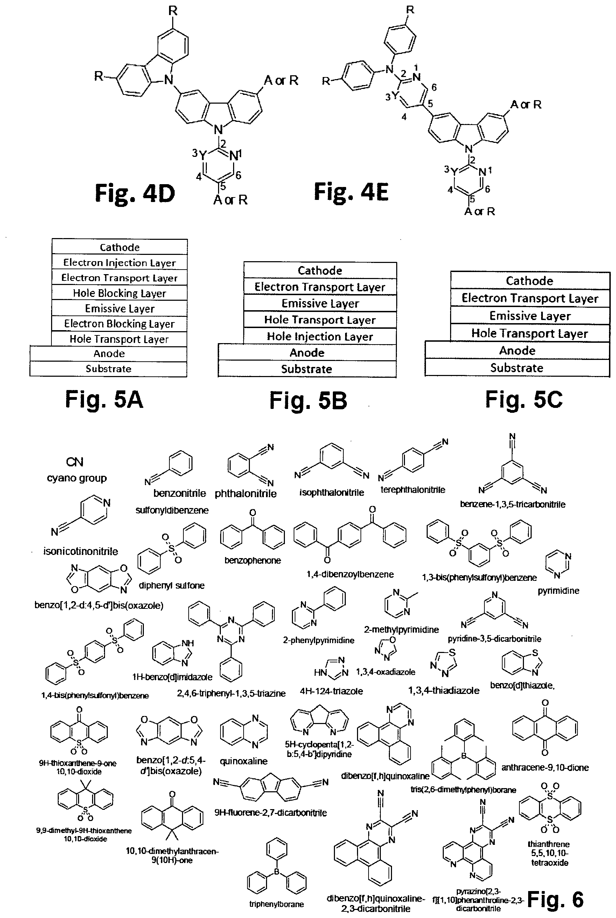

The present disclosure relates to donor structures suitable for use in TADF emitters, comprising within the donor structure a carbazole moiety substituted at its position 3 or positions 3 and 6 with at least one pyridine and/or pyrimidine aromatic ring at their positions 5. To improve the clarity, Figs 4D and 4E presents the atom numbering of pyridine and pyrimidine ring used herein. The pyridine or pyrimidine at their 2 position are further bonded to another amine group being either aromatic or heterocyclic amine - as shown in Fig. 4E, where said amine group is diphenylamine moiety. Optionally, the carbazole moiety can be substituted at its position 9 with the pyridine or pyrimidine ring at its position 2 (Fig. 4D). Thus, the donor structures presented herein are suitable for use in TADF emitters, comprising within the donor structure a carbazole moiety substituted or unsubstituted at its positions 3 and/or 6 and or 9 with pyridine or pyrimidine through their carbon number 5 (Fig. 4E), or through their carbon number 2- as shown in Fig. 4D. Therefore, said amine group in some embodiments of the disclosure, may itself constitute the carbazole moiety - as presented by the example in Fig. 4D.

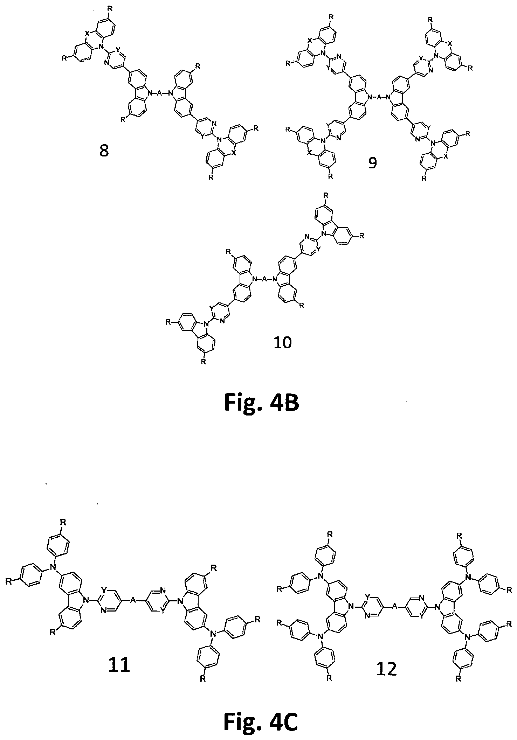

In TADF compounds, the presence of such donor structure, where the carbazole is connected to the other aromatic or heterocyclic amine by the pyridine or pyrimidine spacer at their specified positions, provides an improvement in the value of dihedral angle, ΔE_{ST} and oscillator strength (*f*). Further, the carbazole group can be bonded through its nitrogen atom to another pyridine or pyrimidine rings through their positions 2 respectively, as shown in Fig. 4C and 4D.

The term *donor* mentioned herein relates to a donor of electron(s), i.e. a chemical entity, such as a chemical moiety being a part of molecule, or a separate structure capable of donating its electron(s) to another chemical entity, moiety or structure.

The donor structure according to the present disclosure is especially suitable for use as a donor moiety of TADF materials of various types, e.g. donor-acceptor (D-A) and/or donor-acceptor-donor type (D-A-D). Therefore, the donor structure bonded to respective acceptor may be comprised in the TADF compound and/ or TADF material in a form that is suitable for being immediately used in various light-emitting devices, e.g. of D-A or D-A-D type, therefore capable for thermally activated delayed fluorescence, or it may have a form of compound comprising the donor structure that is suitable to be combined with an acceptor structure, e.g. by substitution or transformation of certain substituent group(s) of the compound comprising the donor structure, as described herein.

The present disclosure provides a material for TADF comprising the donor structure according to the following Formula (I): 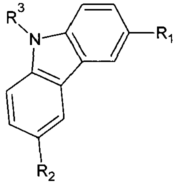 **Formula I** (carbazole moiety with R₁, R₂, R₃ substituents, at the carbazole positions 3, 6, 9)
wherein at least one of the group: R₁, R₂, is represented by Formula II and/or R₃ is represented by Formula III, which are presented below: 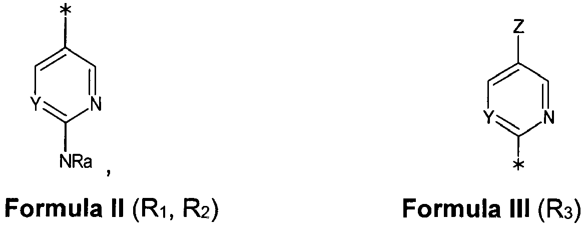 wherein:
Y is independently selected from carbon and nitrogen atom,
NRₐ is amine group independently selected from aromatic amine and heterocyclic amine, wherein N is a trivalent nitrogen atom,
Z is acceptor moiety (A) or another substituent suitable for being transformed or substituted with the acceptor moiety; and the acceptor moiety selected from the group consisting of: cyano (CN) group, benzonitrile, phthalonitrile, isophthalonitrile, terephthalonitrile isonicotinonitrile, benzene-1,3,5-tricarbonitrile, diphenyl sulfone, benzophenone, 1,4-dibenzoylbenzene, 1,3-bis(phenylsulfonyl)benzene, 1,4-bis(phenylsulfonyl)benzene, 2,4,6-triphenyl-1,3,5-triazine, pyrimidine, 2-phenylpyrimidine, 2-methylpyrimidine, pyridine-3,5-dicarbonitrile, 4*H*-124-triazole, 1,3,4-oxadiazole, 1,3,4-thiadiazole, benzo[*d*]thiazole, benzo[1,2-*d*:4,5-*d*']bis(oxazole), benzo[1,2-*d*:5,4-*d*]bis(oxazole), quinoxaline, 1*H*-benzo[*d]*imidazole, dibenzo[*f,h*]quinoxaline, anthracene-9,10-dione, 9*H*-thioxanthene-9-one 10,10-dioxide, 9,9-dimethyl-9*H*-thioxanthene 10,10-dioxide, 10,10-dimethylanthracen-9(10*H*)-one, 5*H*-cyclopenta[1,2-b:5,4-bldipyridine, 9*H*-fluorene-2,7-dicarbonitrile, dibenzo[*f,h*]quinoxaline-2,3-dicarbonitrile, thianthrene 5,5,10,10-tetraoxide, pyrazino[2,3-*f*][1,10]phenanthroline-2,3-dicarbonitrile, triphenylborane, tris(2,6-dimethylphenyl)borane and trimesitylborane. The exemplary acceptor moieties A which may be used are also shown in Fig. 6.

Preferably, NRₐ of Formula II is selected from the group consisting of: 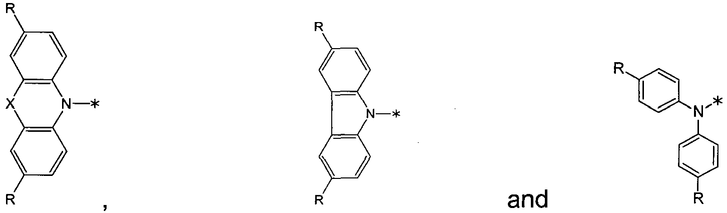 wherein:
R is independently selected from the group consisting of hydrogen (H), alkyl, alkenyl, alkynyl, aryl and heteroaryl,
X is independently selected from the group consisting of O, S, N(R) and C(CH₃)₂. The above-presented amine groups (NRₐ) are especially suitable because of their aromatic or heterocyclic, rigid structures and strong electron donating character.

The group, selected from R₁, R₂ and R₃, that meets Formula II and/or Formula III, respectively, comprises pyridine or pyrimidine moiety that is bounded to trivalent nitrogen atom (N^{III}) of aromatic or heterocyclic amine functional group - for R₁ and/or R₂, and heterocyclic amine being carbazole shown by Formula I - for R₃, through the carbon atom number 2 of the pyridine and/or pyrimidine ring shown by Formula II and Formula III, respectively. Fig. 4C and Fig. 4D schematically show the arrangement of the said pyridine ring of substituent R₁ or R₂ (Fig. 4C) and of substituent R₃ (Fig. 4D).

If R₃ in Formula I is represented by Formula III, the amine group is the carbazole moiety (heterocyclic amine) that is shown by Formula I. In such instance, the donor structure may comprise further (another) aromatic or heterocyclic amine(s) or the donor structure may comprise only said carbazole moiety i.e. the one that is shown by Formula I. Such configuration provides significant reduction of dihedral angle between an acceptor and the carbazole and increases the value of oscillator strength (f).

Nonetheless, if the compound comprising the donor structure is TADF material, Z is an acceptor A structure, the compound exhibits TADF effect. Suitable, non-limiting examples of the acceptor structures A, are shown in Fig. 6.

If R₁ and/or R₂ are (is) represented by Formula II, pyridine and/or pyrimidine ring(s) are (is) substituted to the carbazole moiety, as shown by Formula I. In such case, the amine group NRₐ of Formula II may be either aromatic amine or heterocyclic amine group comprising trivalent nitrogen atom within the amine functional group. Within this configuration, pyridine and/or pyrimidine ring is arranged between two strong electron donating aromatic or heterocyclic amines, one of them being carbazole moiety, the heterocyclic amine that is shown by Formula I, and the other amine constituting the group represented by NRₐ of Formula II. This arrangement of pyridine and/or pyrimidine ring, within the donor structure, provides significant reduction of the dihedral angle between these amines, which in turn leads to a large increase in the value of f parameter and acceptable, i.e. only minimal, increase in ΔE_{ST} value. Thereby, the donor structure, in which R₁ and/or R₂ meet(s) Formula II, exhibits improved values of both parameters, f and ΔE_{ST}. As already mentioned, this simultaneous improvement (f and Δ_{EST}) is due to the presence of pyridine and/or pyrimidine ring specifically arranged between carbazole and the other amine (NRₐ). Furthermore, because carbazole and the other amine NRₐ are both considered as very strong donors, it is presumed that pyridine and/or pyrimidine ring(s), that is (are) included in R₁/R₂ substituents meeting Formula II, are an integral part of the donor structure of Formula I.

The preferred amines denoted as NRₐ by Formula II, are selected from the group consisting of: carbazole and its derivatives (preferably being symmetrical moieties), phenoxazine and/or its derivatives (preferably being symmetrical moieties), phenothiazine and its derivatives (preferably being symmetrical moieties), phenazine and/or its derivatives (preferably being symmetrical moieties) and diphenylamine and/or its derivatives (preferably being symmetrical moieties).

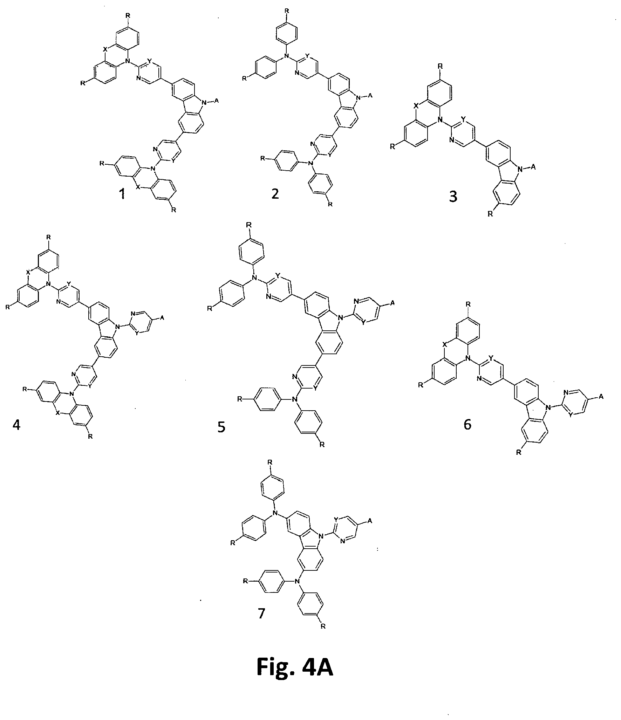

The donor structure of Formula I comprises at least one group selected from R₁, R₂, and R₃ meeting Formula II and/or Formula III, as described above. In one embodiment of the donor structure Formula I comprises only one substituent selected from R₁, R₂, R₃, in another embodiment of the donor structure Formula I comprises two substituents selected from R₁, R₂ and R₃, in yet another embodiment of the donor structure Formula I comprises all the groups: R₁, R₂ and R₃ meeting Formula II and Formula III, respectively. The preferred embodiments of the compounds comprising donor structures according to Formula I are shown in Fig. 4A - 4C. All presented donors can be divided in three groups. First group containing only R₁ and/or R₂ substituents meeting Formula II - compounds 1-3, 8-10 where pyridine or pyrimidine spacer provides decrease in valule of dihedral angle between the carbazole and the other amine (NRₐ) from ca. 90° to 15-45°. Second group, with only R₃ substituent meeting Formula III, comprises pyridine or pyrimidine ring which provides a decrease in value of torsion angle between carbazole and the acceptor moiety A (compounds 7, 11-12). The third group containing R₃ and R₁ and/or R₂ substituents meeting Formula III and Formula II, where pyridine and/or pyrimidine spacer is, thereby, arranged between both carbazole and the other amine (NRₐ), in Formula II, as well as carbazole and acceptor moiety A, in Formula III - compounds 4, 5, 6. These compounds combine all the advantages of both of the aforementioned groups.

The remained substituents R₁, R₂, R₃ i.e. those substituents which are not represented by the Formula II and/or Formula III, respectively, may be of various chemical structure, e.g. selected from the group consisting of H, halogen, hydrocarbon being either saturated or unsaturated, substituted or non-substituted, aryl or heteroaryl. More preferably, as shown in Fig. 4, the structure numbered 7, the substituent, selected from R₁ and R₂ not meeting the Formula II, is independently selected from the group consisting of H, alkyl, alkenyl, alkynyl, aryl, heteroaryl and amine group NRₐ₁. Preferably the amine group NRₐ₁ is selected from the group consisting of heterocyclic amine and aromatic amine. Most preferably the amine group NRₐ₁ constituting R₁ and/or R₂ is selected from: 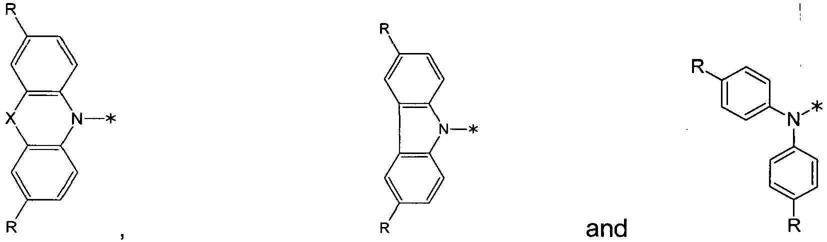 wherein:
R is independently selected from the group consisting of H, alkyl, alkenyl, alkynyl, aryl and heteroaryl.
X is independently selected from the group consisting of O, S, N(R) and C(CH₃)₂.

In the case where both R₁ and R₂ substituents do not meet Formula II, the substituent R₃ meets Formula III, and thereby R₃ is represented by Formula III. The preferred embodiment of such donor structure is shown in Fig. 4, numbered 7. This structure exhibits considerably low ΔE_{ST} parameter as well as high f parameter.

However, if R₃ substituent does not meet Formula III, and thereby R1, and/or R₂ are represented by Formula II, the R₃ substituent is preferably represented by the acceptor moiety A, therefore, the material comprising the donor structure is TADF emitter. The preferred acceptor moieties A are listed above and shown in Fig. 6. Nonetheless, the substituent R₃ that is not represented by Formula III, not necessarily has to constitute an acceptor moiety, and R₃ not meeting Formula III, may be selected from the group suitable for being substituted or transformed into the acceptor moiety. If R₃ is not an acceptor moiety A, the compound comprising the donor moiety is not a TADF material, but it is suitable for being transformed into the TADF material upon combination with the acceptor structure, thereby constituting an intermediate for synthesizing a TADF compound.

Fig. 3 presents a comparison of important parameters of prior art compound comprising donor structure (Fig. 3A) and two compounds comprising donor structures according to Formula I (Fig. 3B, Fig. 3C) in which only the substituent R₁ is represented by the Formula II comprising pyridine ring (Fig. 3B) and pyrimidine ring (Fig. 3C), R₂ is hydrogen (H), and R₃ is benzonitrile moiety. Thereby, neither R₂ meets Formula II nor R₃ meets Formula III in this embodiment of donor structure.

The donor structures of Fig. 3B and Fig. 3C feature significant reduction of dihedral angle, when compared with the corresponding structure known from the art (Fig. 3A). The values of dihedral angles and f values were obtained by DFT calculations in Gaussian program using B3LYP functional.

The observed reduction of dihedral angle, for Fig. 3B and 3C, was achieved by introduction of pyridine (Fig. 3B) and pyrimidine moiety, respectively (each being heterocyclic aromatic ring), between the carbazole and the heterocyclic amine moiety, thereby, providing a configuration in which the donor structure comprises carbazole moiety and a pyridine or pyrimidine ring bonded by its carbon atom number 2 to the trivalent nitrogen of heterocyclic amine group.

As can be seen in Fig. 3, the dihedral angle is reduced from over 94° (Fig. 3A) to 22.5° (Fig. 3B) and 8° (Fig. 3C), respectively. Thus the use of pyridine ring, substituted at its postions 2 and 5 (as shown in Fig. 3B) with carbazole and the other heterocyclic amine, as a spacer between these two amines, provides a significant decrease in the value of dihedral angle and hence an increase in the f parameter, with ΔE_{ST} value constantly not greater than 0.2 eV. The use of pyrimidine provides even further reduction in the value of dihedral angle and hence the increase of f value, together with, an acceptable, minor increase in ΔE_{ST}. This is due to the obtained configuration in which two strong electron-donating heterocyclic amines are separated by pyridine or pyrimidine ring which due to the reduction of dihedral angle between the two amines, contributes to a partial, HOMO and LUMO, orbital overlapping which isresponsible for the above-described reduction of f value almost to zero.

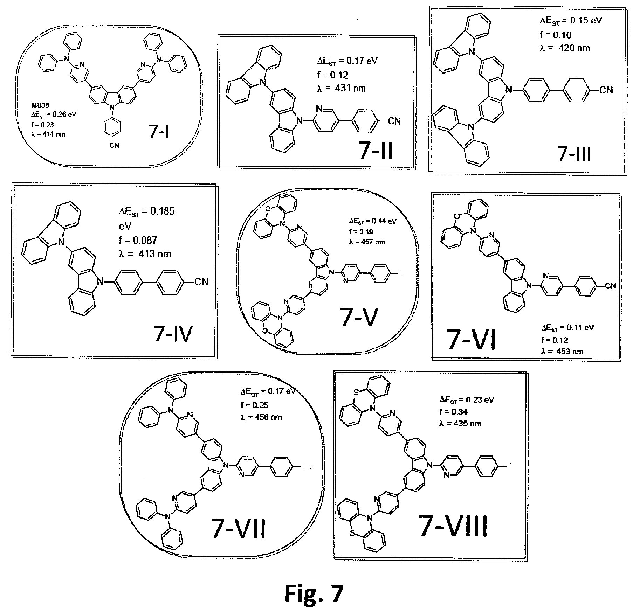

Further, Fig. 7 presents the values f and ΔE_{ST} of the prior art TADF materials: 7-II, 7-III, 7-IV, and TADF material comprising the donor structures according to the present disclosure. Introduction of only one nitrogen atom into phenylene group attached to carbazole nitrogen atom reveals significant improvement of oscillator strength and shift of the emission maximum towards longer wavelengths with an only slight increase of ΔE_{ST}.

In the developed donor structure of Formula I, where at least one substituent R₁, R₂, R₃ is represented by Formula II and/or Formula III, a pyridine/pyrimidine aromatic ring is provided as a spacer that is directly bonded, through the atom number 2 of the pyridine/pyrimidine aromatic ring, to the trivalent nitrogen of the amine group being heterocyclic amine or aromatic amine. The presence of such configuration of the electron orbitals involved in the creation of respective bonds between the atoms, in the donor structure according to Formula I, provides improvement in certain parameters including reduced value of the dihedral angle and increased value of oscillator strength (f) parameter, which are considered to be important for providing higher quantum efficiency of the TADF emitters.

Thus, the donor structure of Formula I is especially suitable to be used as donor moiety in TADF emitter of the structure of donor-acceptor (D-A) or donor-acceptor-donor type (D-A-D). The TADF emitters comprising the donor structure of Formula I exhibit improved parameters including: high TADF effect due to low ΔE_{ST} value and high oscillator strength and stability. The exemplary embodiments of TADF structures are shown in Fig. 4A, where structures 1, 2, 3, 4, 5, 6, 7 are of D-A type, and in Figs 4B where structures 8, 9, 10 are of D-A-D type, wherein two donor moieties D-may be of the same or of different chemical structure, wherein at least on the donor structure meets Formula I, thereby representing the donor moiety according to the present disclosure. In Fig. 4A-C, A denotes acceptor moiety and D denotes any donor moiety.

Further, in Fig. 4A, the molecular skeletons of the donor structures 1, 2, 4, 5, 7 are symmetrical as they comprise R₁ and R₂ substituents of the same amino group bonded to the pyridine/pyrimidine ring. The structures 3, 6 are unsymmetrical as they comprise either R₁ and R₃ substituent of Formula II and Formula III, or only R₁ substituent of Formula II. Preferably, as shown in Fig. 4, the symmetrical and unsymmetrical donor structures (Formula I) are heterocyclic compounds having in their structure, a pyridine and/or pyrimidine ring (Formula II), connected through its carbon atom of aromatic ring in position 5 with carbazole moiety (in its position 3 or in its positions 3 and 6) and in position 2 with nitrogen atom of an aromatic or heterocyclic amine group, wherein most preferably the amine group is selected from the group consisting of diphenylamine, carbazole, 3,6-di-alkyl-9*H*-carbazole, phenoxazine, phenothiazine, 5,10-dihydrophenazine, 9,10-dihydro-9,9-dimethylacridine and their derivatives (as shown in Fig. 4, donor structures 1, 2, 3, 4, 5, 6. Furthermore, the pyridine ring can also be connected via a carbon atom in 2 position with the nitrogen atom of the carbazole moiety and through a carbon atom in position 5 with the acceptor (Fig. 4, donor structures 4, 5, 6, 7).

All the structures of Figs 4A - 4B are of electron-donor nature, and in combination with the prior art acceptor(s) A, show the effect of thermally activated delayed fluorescence (TADF) occurring in conjugated aromatic and heterocyclic systems in which the ΔE_{ST} parameter (energy difference between singlet S₁ and triplet excited state T₁) is substantially low, preferably below 0.2 eV, and the oscillator strength parameter (f) is higher (improved) than those of the known donor structures. The oscillator strength parameter (f) values obtained for the donor structures according to Formula I are preferably above 0.2, whereas ΔE_{ST} is preferably at most of 0.2 eV and more preferably at most of 0.1 eV. This provides high quantum efficiency of the TADF material, of both D-A type and D-A-D type. The introduction of pyridine/pyrimidine ring according to Formula II and/or III provides certain configuration of the atoms and electrons forming respective bonds between them and partial separation of HOMO and LUMO molecular orbitals in the donor structure of Formula I. The obtained separation of orbitals is large enough for the ΔE_{ST} parameter to be less than 0.2 eV but not complete, thus HOMO and LUMO orbitals partially overlap within the donor structure, which is important as it provides the value of oscillator strength parameter f above zero (f>0). Thereby the developed donor structures, when combined with acceptor structures to form TADF compounds, can exhibit particularly good performance when used as the TADF emitter materials in various light-emitting devices such as diodes. Preferably, the TADF emitters comprising the donor structure according to Formula I can be used in organic light-emitting diodes (OLEDs). The emitters comprising the donor structures of Formula I show low ΔE_{ST} values (below 0.2 eV) and high oscillator strength (f) and can emit light of a wavelength range from 380 nm to 900 nm depending on the type of connected acceptor structure. Figs. 5A - 5C show schematically three embodiments of the diode architectures with the material for TADF comprising the donor structure according to Formula I.

As shown in Fig. 5 the diode may be of layered structure comprising: emissive layer being the light-emitting layer, cathode, anode, substrate, and optionally further at least one material selected from the group consisting of electron injection material, hole injection material, electron transport material, hole transport material, electron blocking material and hole blocking material, depending on the diode architecture. The preferred diode architectures are shown schematically in Figs. 5A, 5B and 5C.

The light-emitting layer also called herein *'emissive layer'* according to Figs. 5A, 5B and 5C may comprise one or more TADF compound(s) comprising one or more than one donor structure(s) according to Formula I. More preferably the emissive layer comprises one or more of the donor structures according to Fig. 4, wherein A group constitutes the acceptor structures, preferably selected from the group shown in Fig. 6.

The diode of Fig. 5 comprises the emissive layer comprising emitter molecules. Holes or electrons injected from anode or cathode recombine and excites the emitter molecules to form excitons. The excitons return to their basic state by emitting light. The emitter molecules may be solely used as the emissive layer, or the emissive layer may further contain a host material, beside the emitter molecules.

The emitter molecules, being TADF compound(s), used in the emissive layer of the diode, according to Fig. 5, may comprise one or more than one donor structure(s) according to Formula I, and more preferably one or more than one donor structure(s) shown schematically in Fig. 4 (numbered 1, 2, 3, 4, 5, 6, 7). However, it is important that the recombination, mentioned above, occurs within the emissive layer of the diode in order to obtain high emission efficiency.

The light emitted by the diode contains both fluorescent emission and delayed fluorescent emission. As the emission in the emissive layer proceeds by the Thermally Activated Delayed Fluorescence mechanism, a host material is preferably used to avoid the efficiency roll-off of the emitter and a part of the emitted light may contain light emitted from the host material.

The amount of TADF emitter in host is 0.1%-50% by weight, preferably 5-20% by weight. The host material is preferably an organic compound that has both hole transporting function and electron transporting function and singlet and triplet excited state energy higher than the singlet and triplet excited state energy of the emitter. The host materials that can be used in the diode with TADF emitter(s) comprising the donor structure according to Formula I are preferably selected from the group consisting of bis[2-(diphenylphosphino)phenyl]ether oxide (DPEPO), 1,3-bis[2-(4-tert-butylphenyl)-1,3,4-oxadiazo-5-yl]benzene (OXD-7), 1,4-Bis(triphenylsilyl)benzene (UGH-2), 2,8-Bis(diphenyl-phosphoryl)-dibenzo[b,d]thiophene (PPT), diphenyl[4-(triphenylsilyl)phenyl]phosphine oxide (TSPO1), 2,2',2"-(1,3,5-Benzinetriyl)-tris(1-phenyl-1-H-benzimidazole) (TPBi), tris(4-carbazoyl-9-ylphenyl)amine (TCTA), 1,1-Bis[(di-4-tolylamino)phenyl]cyclohexane (TAPC), bis-4-(N-carbazolyl)phenyl)phenylphosphine oxide (BCPO), and their derivatives, and/or carbazole-based hosts selected form the group consisting of: 4,4'-Bis(N-carbazolyl)-1,1'-biphenyl (CBP), 1,3-Bis(N-carbazolyl)benzene (mCP), mCPCN, 3,3'-Di(9*H-*carbazol-9-yl)-1,1'-biphenyl (mCBP), CPCB, 9-(4-tert-Butylphenyl)-3,6-bis(triphenylsilyl)-9*H*-carbazole (CzSi), 9-Phenyl-3,6-bis(9-phenyl-9*H*-carbazol-3-yl)-9*H*-carbazole (Tris-PCz), 9,9'-Diphenyl-9*H*,9'*H*-3,3'-bicarbazole (BCzPh), LK-3-7, ZDZ, SiCzPy1, SiCzPy2, SiCzPy3 and 5-(4,6-Diphenyl-1,3,5-triazin-2-yl)benzene-1,3-dinitrile (DCzDCN). Nonetheless, other host materials, e.g. those known from the art may be used as the host materials as well.

The substrate of the diode (Fig. 5A-5C) may be made of various materials, which are commonly used in organic electroluminescent devices, preferably transparent or translucent, such as glass, quartz, silicon, transparent polymer or polymers mixtures.

The anode of the diode may be preferably made of an electroconductive compound or a mixture thereof, such as a metal, metal alloy preferably of large work function i.e. above or equal 4 eV (≥4 eV). The anode material may include indium tin oxide (ITO), SnO₂, ZnO, FTO, Cul, IDIXO (In₂O₃-ZnO), MoOs, Au. The anode may be for example a thin film of the material prepared by vapor deposition or sputtering and can be patterned by a known patterning method, for example, photolithography. Alternatively, wet methods, such as printing or a coating, may be used to obtain the anode. The anode preferably may have a transmittance of more than 10%, for example, more than 20%, or more than 50% or more than 70% or more than 90%. Preferably, the anode may exhibit a sheet resistance of several hundred Ohm per square or less. The thickness of the anode film preferably may be in the range of 10 nm to 1 µm, and more preferably in the range of 10 to 300 nm, depending on the material used.

The cathode of the diode may be preferably made of a metal, an alloy or an electroconductive compound or mixture thereof, preferably having a small work function, more preferably below or equal 4 eV (≤4 eV). For example, the cathode may comprise aluminum, magnesium, calcium, a magnesium-copper mixture, a magnesium-silver mixture, a magnesium-aluminum mixture, a magnesium-indium mixture, an aluminum-aluminum oxide (Al₂O₃) mixture, indium, a lithium-aluminum mixture, sodium, lithium, a sodium-potassium alloy, or a rare earth metal. The cathode may be produced, for example, by forming the cathode material into a thin film, e.g. by vapor deposition or sputtering. The cathode preferably has a sheet resistance of several hundred Ohm per square or less, and the thickness from 10 nm to 5 µm, and preferably from 50 to 200 nm. The cathode may also be prepared from electroconductive transparent materials of a small work function (≤4 eV) to give a transparent or translucent cathode, therefore, in combination with a transparent anode, a fully transparent device may be produced.

The hole transport layer of the diode may be made of semiconductor capable of transporting holes injected from anode toward the emissive layer. Preferably, the hole transporting material may also have the property of electron blocking. The hole transport materials that may be used in the diode include triphenylamine-based materials, aromatic tertiary amines, carbazole derivatives, fluorenone derivatives, phenylenediamine derivatives, arylamine derivatives, spiro compounds, transition metal oxides of n-type, e.g., V₂O₅, WO₃, MoOs, or p-type, e.g., NiOx, as well as silazane derivatives, indolocarbazole derivatives, triazole derivatives, oxadiazole derivatives, imidazole derivatives, polyarylalkane derivatives, pyrazoline derivatives, pyrazolone derivatives, amino-substituted chalcone derivatives, oxazole derivatives, anthracene derivatives, hydrazone derivatives, stilbene derivatives, aniline copolymers, or electroconductive polymers such as polithiophenes and thiophene oligomers.

The electron transport layer of the diode may be made of semiconductor capable of transporting electrons. Preferably, the electron transport material may also have the property of hole blocking. The electron transport materials that may be used in the diode include metal chelates, oxadiazole compounds, polymeric oxadiazoles, azole-based materials, triazines, polibenzodiazoles, benzothiadiazole polymers, imine compounds (N=C) containing quinoline, anthrazoline, phenanthraline, polypyridines, quinoline derivatives, polyquinolines, quinoxaline derivatives, anthrazolines, phenanthroline derivatives, perfluorinated oligo(p-phenylene)s, and fluorene derivative.

Optionally, the diode may comprise the hole injection layer provided between the anode and the hole transport layer to decrease the driving voltage and enhance light emission.

Optionally, the diode may comprise the electron injection layer which is provided between the cathode and the electron transport layer to decrease the driving voltage and enhance light emission.

Optionally the diode may comprise the hole blocking layer to trap holes before reaching the electron transporting layer and hence enhances the probability of electrons and holes recombination in the emissive layer. Preferably, the electron transporting layer materials may be used as the materials of the hole blocking layer.

Optionally, the diode may comprise the electron blocking layer to trap electrons before reaching the hole transporting layer in order to enhance the probability of electrons and holes recombination in the emissive layer. Preferably, the hole transporting layer materials may be used as the material for the electron blocking layer.

The diode may comprise TADF emitter(s) comprising the donor structure(s) according to Formula I within at least one layer selected from the group consisting of injection layer, barrier layer, hole barrier layer, electron barrier layer, hole transport layer or electron transport layer as well as yet another layer of the diode, preferably of the function similar to those of mentioned layers. The said layer(s) with the TADF emitter(s) comprising the donor structure(s) according to Formula I may be made of the material(s) listed above. The layers of the diode with the TADF emitter(s) comprising the donor structure(s) according to Formula I may be produced by various techniques, such as those known from the art, e.g. including dry processes such as for example, physical vapor deposition (PVD), and wet processes such as for example coating, or ink-jet printing.

Method of synthesis the donor structures and donor-acceptor structures according to the present disclosure:

### EMBODIMENT 1 - synthesis of 10-(5-(9H-Carbazol-3-yl)pyridin-2-yl)-10H-phenoxazine and 3,6-bis(2-(10H-phenoxazin-10-yl)pyridin-5-yl)-9H-carbazole

The above 10-(5-bromopyridin-2-yl)-10*H*-phenoxazine, shown also below: 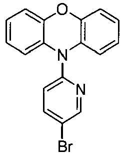 was synthesized by the following method:
phenoxazine (1.83 g; 10 mmol), potassium tert-butoxide (1.68 g; 15 mmol) and 5-bromo-2-fluoropyridine (1.76 g; 10 mmol) were mixed in dry DMF (50 mL), stirred at 100°C for 24 h and cooled to RT. The reaction was quenched with water (50 mL) and extracted with chloroform (3×50 mL). The combined extracts were washed with water (100 mL), brine (100 mL) and dried with anhydrous magnesium sulfate. The solvent was removed by rotary evaporation to give 3.436 g of dark powder. The crude product was adsorbed onto silica gel and purified by flash column chromatography AcOEt/PE - 5:95. The pure product was obtained as a yellow powder, product mass: m=2.567 g, (yield: 76%), melting temperature: mp = 108.3-111.3°C.

The data of ¹H NMR spectrum of the obtained product:
1H NMR (CDCl3, 700 MHz), δ ppm: 6.71 (d, J=8 Hz, 2H, 2CHAr); 6.78-6.83 (m, 2H, 2CHAr); 6.84-6.88 (m, 4H, 4CHAr); 7.24 (d, J=8.7 Hz, 1H, CHAr); 7.86 (dd, J1 =8.7 Hz, J2 = 2.6 Hz, 1H, CHAr); 8.64 (d, J=2.7 Hz, 1H, CHAr).

The above 3-bromo-9H-carbazole, shown also below: 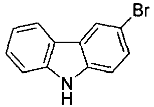 was synthesized by the following method:
In a 500 mL two-necked flask equipped with a reflux condenser and dropping funnel, carbazole (20 g; 0.12 mol; 1 eq.) and DMF (200 mL) were placed and stirred for 10 minutes until a homogeneous solution was obtained. N-Bromosuccinimide (22.43 g; 0.126 mol 1.05 eq.) in DMF (50 mL) was added dropwise and the mixture was stirred at RT overnight. The mixture was poured into water (1000 mL), stirred for 30 minutes and the product was filtered off on a sintered glass funnel, washed with water (3×150 mL), petroleum ether (2×150 mL) and air-dried. The precipitate (28.6 g) was placed in a single-necked flask equipped with a reflux condenser and petroleum ether (450 mL) and acetone (50mL) were added. The mixture was heated to 60°C and stirred at this temperature for 10 minutes. The hot mixture was filtered, the precipitate was washed with another portion of petroleum ether (45 mL) + acetone (5 mL) and air-dried to obtain product mass m = 21.2 g (yield: 72%) of the product as a white powder, which was taken to the next step without purification; melting temperature: mp = 192-194°C.

The above 3,6-dibromo-9*H*-carbazole shown also below: 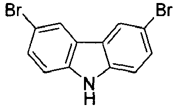 was synthesized by the following method:
carbazole (10 g; 0.06 mol; 1 eq.) was placed in a 500 mL two-necked flask equipped with a reflux condenser and dropping funnel and DMF (200 mL) was added. The mixture was stirred 10 minutes to obtain a homogeneous solution and N-bromosuccinimide (22.43 g; 0.126 mol 2.1 eq.) in DMF (70 mL) was added dropwise. Mixture was stirred at RT for 3 hours and then poured into water (1000 mL), stirred for 30 minutes and the product was filtered off on a sintered glass funnel. It was washed with water (3×150 mL), petroleum ether (2×150 mL) and air dried to give 18.68 g (96%) of a light beige product. The product without purification was taken to the next stage. mp = 200-204 °C.

The data of ¹H NMR and IR spectra of the obtained product:
¹H NMR (DMSO-d₆, 700 MHz), δ ppm: 11.59 (s, 1H, NH), 8.43 (d, *J*=2.0 Hz, 2H, 2xCHAr), 7.53 (d, *J*=2.0 Hz, 1H, CHAr), 7.52 (d, *J*=2.0 Hz, H, CHAr) 7.48 (s, H, CHAr), 7.47 (s, H, CHAr).

IR vₘₐₓ cm⁻¹: 3413.67(s), 3068.88(w), 2919.59(m), 2858.04(w), 1869.36(w), 1737.17(w), 1660.75(w), 1604.40(m), 1562.03(w), 1469.04(s), 1.431.85(s), 1378.92(w), 1324.81(w), 1281.24(s), 1237.33(s), 1126.99(w), 1051.24(m), 1015.41(w), 898.76(w), 866.98(m), 800.55(s), 732.25(w), 685.93(m), 635.95(m), 561.45(s), 490.28(w).

The above 3-(4,4,5,5-tetramethyl-1 ,3,2-dioxaborolan-2-yl)-9*H*-carbazole, shown also below by two formulas of the same meaning: 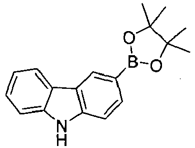 was synthesized by the following method:
in a Schlenk flask equipped with a magnetic stirring bar and reflux condenser, 3-bromocarbazole (1.230 g; 5 mmol), Pd(dppf)Cl₂ (0.075 g; 0.1 mmol) and potassium acetate (0.98 g; 10 mmol) were added under argon atmosphere, followed by dry degassed dioxane (25 mL). After 23 hours at 80°C, it was cooled to RT (room temperature) and water (250 mL) was added. Mixture was extracted with chloroform (3×100 mL), the combined organic extracts were washed with water (250 mL) and brine (200 mL) and dried with anhydrous magnesium sulfate. Solvent was removed by rotary evaporation to give 1.94 g of light oil (crystallizes after cooling) - the product. The crude product was adsorbed on silica gel and purified by flash chromatography (DCM) to give 1.173 g of the product (yield: 80%) of a light yellow powder, mp = 195.6-196.9°C.

The data of ¹H NMR and IR spectra of the obtained product:
¹H NMR (CDCl₃, 700 MHz), δ ppm: 1.40 (s, 12H, 12CH3); 7.22-7.27 (m, 1H, CHAr); 7.39-7.44 (m, 3H, 3CHAr); 7.88 (dd, J1=8.1 Hz, J2=1.0 Hz, 1H, CHAr); 8.11 (d, J=7.6 Hz, 1H, CHAr); 8.14 (s, br, 1H, NH); 8.58 (s, 1H, CHAr).

IR vₘₐₓ cm⁻¹: 3284.80(m), 2975.43(m), 2646.49(w), 2281.76(w), 1883.89(w), 1753.03(w), 1701.64(w), 1604.52(m), 1442.97(m), 1350.99(s), 1240.08(s), 1138.89(s), 1070.60(s), 963.22(m), 895.11(m), 854.86(s), 810.46(m), 731.60(s), 675.13(s), 580.05(m), 527.97(m), 501.70(m), 450.53(m), 419.74(m).

The above 3,6-bis(4,4,5,5-tetramethyl-1,3,2-dioxaborolan-2-yl)-9*H*-carbazole, shown also below: 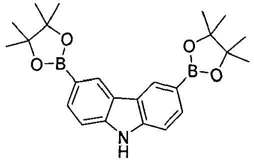 was synthesized by the following method:
3,6-Dibromocarbazole (1.63 g; 5 mmol), bis(pinacolato)diboron (5.08 g; 20 mmol), Pd(dppf)Cl₂ (0.10 g; 0.14 mmol) and potassium acetate (1.93 g; 20 mmol) were mixed in dry 1 ,4-dioxane (20 mL) under a nitrogen atmosphere. After 20 h at 80°C, the mixture was cooled to RT and water (100 mL) was added. Extracted with dichloromethane (3×80 mL). The combined organic extracts were washed with water (100 mL), brine (100 mL) and dried with anhydrous magnesium sulfate. Solvent was evaporated to dryness on a rotary evaporator to give 1.30 g of a light oil (crystallizes after cooling). The crude product was adsorbed on silica gel and purified by flash chromatography (AcOEt:PE- 15:85) to give 1.10 g (53%) of a light yellow powder. mp=96-98°C.

The data of ¹H NMR and IR spectra of the obtained product:
¹H NMR (CDCl₃, 700 MHz), δ ppm: 1.39 (s, 24H, 24CH₃); 7.40 (d, *J*=8.0 Hz, 2H, 2CH_{Ar}); 7.86 (dd, *J₁*=8.0 Hz, *J₂*= 1.1 Hz, 2H, 2CH_{Ar}); 8.22 (br, 1H, NH_{Ar}); 8.65 (s, 2H, 2CH_{Ar}).

IR vₘₐₓ cm⁻¹: 3406.87(m), 3305.85(w), 2977.61(m), 2934.61(w), 1907.75(w), 1695.17(m), 1602.13(m), 1459.79(m), 1347.41(s), 1247.57(m), 1138.07(s), 1074.28(s), 1024.16(m), 961.20(m), 909.26(m), 855.14(m), 815.70(m), 746.07(m), 673.71(s), 635.41(m), 576.99(m), 420.50(m).

The above 10-(5-(9*H*-carbazol-3-yl)pyridin-2-yl)-10*H*-phenoxazine, shown also below: 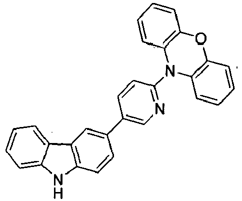 was synthesized by the following method:
3-(4,4,5,5-Tetramethyl-1,3,2-dioxaborolane-2-yl)-9*H*-carbazole (0.586 g; 2 mmol), 10-(5-bromopyridin-2-yl)-10*H*-phenoxazine (0.678 g; 2 mmol) and Pd(dppf)Cl₂ (0.035 g; 0.05 mmol) were mixed in a Schlenk flask under a nitrogen atmosphere in degassed 1,4-dioxane (5 mL). K3PO4 (3M aqueous, 5mmol, 1.7 mL) was added and the flask was inserted into an oil bath at T=80°C. After 20 hours the mixture was cooled to RT, poured into water (50 mL) and extracted with chloroform (3×20 mL). The combined organic extracts were washed with water (50 mL), brine (50 mL) and dried with anhydrous magnesium sulfate. The solvent was evaporated to dryness to give 0.540 g of dark green powder. The crude product was adsorbed on silica gel and purified by flash column chromatography (AcOEt:PE, 25→50%) to give the pure product (light yellow powder), m=0.450 g (53%), mp = 254-256°C.

The data of ¹H NMR and IR spectra of the obtained product:
¹H NMR: (CDCl3, 700 MHz), δ ppm: 6.50-6.63 (m, 2H, 2CHAr); 6.73-6.85 (m, 6H, 6CHAr); 7.29 (td, J1=7.1 Hz, J2=1.2 Hz, 1H, CHAr); 7.44-7.50 (m, 3H, 3CHAr); 7.56 (d, J=8.2 Hz, 1H, CHAr); 7.68 (d, J=8.2 Hz, 1H, CHAr); 8.14 (d, J=7.6 Hz, 1H, CHAr); 8.18 (d, J=8.1 Hz, 1H, CHAr); 8.27 (s, 1H, br, NH); 8.32 (s, 1H, CHAr); 9.03 (s, 1H, CHAr).

IR vₘₐₓ cm⁻¹: 3415.64(m), 3050.63(w), 1730.43(w), 1585.31(m), 1485.71(s), 1330.45(s), 1271.68(s), 1042.81(m), 935.35(m), 865.35(m), 811.30(m), 732.91(s), 615.64(m), 569.32(m), 520.65(m), 421.23(s).

The above 3,6-bis(2-(10*H*-phenoxazin-10-yl)pyridin-5-yl)-9*H*-carbazole shown also below: 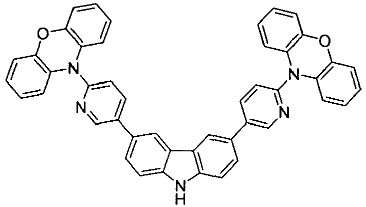 was synthesized by the following method:
3-(4,4,5,5-Tetramethyl-1,3,2-dioxaborolan-2-yl)-9*H*-carbazole (0.156 g, 0.37 mmol), 10-(5-bromopyridin-2-yl)-10*H*-phenoxazine (0.251 g; 0.74 mmol) and Pd(dppf)Cl₂ (0.027 g; 0.037 mmol) were mixed in degassed 1,4-dioxane (5 mL) under an argon atmosphere. K₃PO₄ (3M aqueous, 1.0 mL, 3 mmol) was added the mixture was heated at T=80°C for 2 hours. It was cooled to RT, water (20 mL) was added and extracted with chloroform (3×20 mL). The combined organic extracts were washed with water (50 mL), brine (50 mL) and dried with anhydrous magnesium sulfate. The solvent was removed to give 0.351 g of a brown powder. The crude product was adsorbed on silica gel and purified by flash chromatography (AcOEt:PE - 25:75) to give 0.178 g (70%) of the pure product (light yellow powder), mp=143.4-144.6°C.

The data of ¹H NMR and IR spectra of the obtained product:
¹H NMR: (CDCl₃, 700 MHz), δ ppm: 6.52 (d, *J*=8.2 Hz, 4H, 4CH_{Ar}); 6.73-6.83 (m, 12H, 12CH_{Ar}); 7.48 (d, *J*=8.3 Hz, 2H, 2CH_{Ar}); 7.62 (d, *J*=8.3 Hz, 2H, 2CH_{Ar}); 7.75 (dd, *J₁*=8.2 Hz, *J₂*=1.6 Hz, 2H, 2CH_{Ar}); 8.18 (dd, *J₁*=8.2 Hz, *J₂*=2.3 Hz, 2H, 2CH_{Ar}); 8.39 (s, 1H, br, NH); 8.41 (s, 2H, 2CHAr); 9.03 (d, *J*=2.3 Hz, 2H, 2CH_{Ar}).

IR vₘₐₓ cm⁻¹: 29922.57(w), 2226.86(w), 1887.63(w), 1732.90(w), 1596.32(m), 1481.93(s), 1372.26(m), 1327.36(m), 1261.44(s), 1151.22(m), 1039.70(m), 927.48(m), 808.84(m), 737.04(s), 612.26(m), 581.54(m), 426.81(m).

**EMBODIMENT 2** - method for synthesizing of TADF compounds comprising the donor structure of Formula I. The obtained TADF compounds are as follows:
4-(3-(2-(10*H*-Phenoxazin-10-yl)pyridin-5-yl)-9*H*-carbazol-9-yl)benzonitrile, 10,10'-(((Sulfonylbis(4,1-phenylene))bis(9*H*-carbazole-9,3-diyl))bis(pyridine-5,2-diyl))bis(10*H*-phenoxazine),
Bis(4-(3-(2-(10*H*-phenoxazin-10-yl)pyridin-5-yl)-9*H*-carbazol-9-yl)phenyl)methanone, 10-(5-(9-(4-((4-(9H-Carbazol-9-yl)phenyl)sulfonyl)phenyl)-9*H*-carbazol-3-yl)pyridin-2-yl)-10*H*-phenoxazine,
1,4-Phenylenebis((4-(3-(2-(10*H*-phenoxazin-10-yl)pyridin-5-yl)-9*H*-carbazol-9-yl)phenyl)methanone).

Compounds IV and V are not encompassed by the wording of the claims but are considered as useful for understanding the invention.

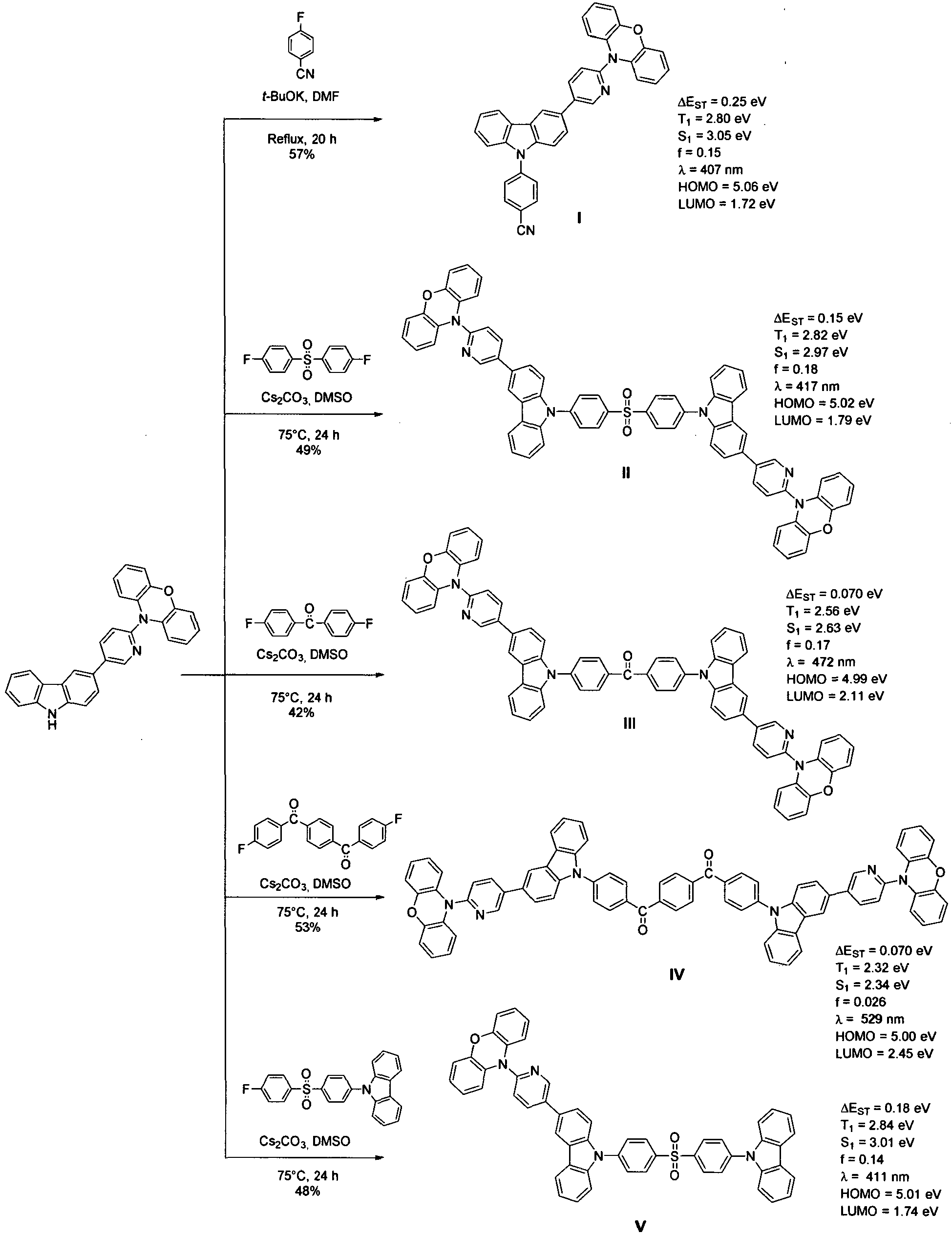

The 10-(5-(9*H*-carbazol-3-yl)pyridin-2-yl)-10*H*-phenoxazine obtained in EMBODIMENT 1 served as substrate for synthesis according to EMBODIMENT 2.

The above 4-(3-(2-(10*H*-phenoxazin-10-yl)pyridin-5-yl)-9*H*-carbazol-9-yl)benzonitrile shown also below: 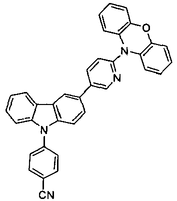 was synthesized by the following method:
10-(5-(9*H*-carbazole-3-yl)pyridin-2-yl)-10*H*-phenoxazine (0.426 g; 1 mmol), 4-fluorobenzonitrile (0.242 g; 2 mmol) and potassium *tert*-butoxide (0.224 g; 2 mmol) were mixed in dry DMF (5 mL) and heated to reflux. After 20 h it was cooled to RT and poured into water (50 mL) and extracted with chloroform (3×30 mL). The combined extracts were washed with water (2×100 mL) and brine (100 mL), dried with anhydrous magnesium sulfate and evaporated to dryness. The crude product was adsorbed on silica gel and purified by flash column chromatography (DCM:PE, 70→80%) to give 0.300 g (57%) of a light yellow powder. Melting point mp=272.3-274.0°C.

The data of ¹H NMR and IR spectra of the obtained product:
¹H NMR (CDCl3, 700 MHz), δ ppm: 6.55 (d, J=7.9 Hz, 2H, 2CH_{Ar}); 6.74-6.84 (m, 6H, 6CHAr); 7.37-7.42 (m, 1H, 2CHAr); 7.47 (d, *J*=8.4 Hz, 1H, CHAr); 7.48-7.51 (m, 2H, 2CH_{Ar}); 7.58 (d, 1H, CH_{Ar}); 7.71 (dd, *J₁*=8.5 Hz, *J₂*=1.8 Hz, 1H, CH_{Ar}); 7.79 (d, *J*=8.6 Hz, 2H, 2CH_{Ar}); 7.95 (d, *J*=8.6 Hz, 2H, 2CH_{Ar}); 8.15 (dd, *J₁*=8.3 Hz, *J₂*=2.6 Hz, 1H, CH_{Ar}); 8.22 (d, *J*=7.8 Hz, 1H, CH_{Ar}); 8.39 (d, *J*=1.5 Hz, 1H, CH_{Ar}); 9.01 (d, *J*=2.6 Hz, 1H, CH_{Ar}).

IR vₘₐₓ cm⁻¹: 3064.69(w), 2924.15(w), 2611.12(w), 2283.52(w), 2224.06(m), 2060.40(w), 1985.52(w), 1915.30(w) 1831.27(w), 1659.2(w), 1597.49(s), 1483.68(s), 1454.96(s), 1329.02(s), 1269.44(s), 1230.48(s), 1160.80(m), 116.66(m), 1039.21(m), 921.5(w), 849.20(m), 804.69(m), 726.95(s), 623.31(m), 582.00(m), 547.44(m), 416.03(m).

The above 10,10'-(((sulfonylbis(4,1-phenylene))bis(9*H*-carbazole-9,3-diyl))bis(pyridine-5,2-diyl))bis(10*H*-phenoxazine) shown also below: 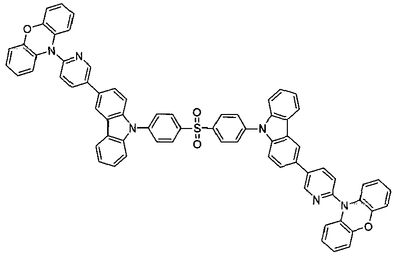 was synthesized by the following method:
4,4'-Sulfonylbis(fluorobenzene) (0.058 g; 0.23 mmol), 10-(5-(9*H*-carbazol-3-yl)pyridin-2-yl)-10*H*-phenoxazine (0.300 g; 0.71 mmol) and cesium carbonate (0.232 g; 0.71 mmol) were mixed in dry DMSO and heated to 75°C. After 24h at this temperature, the reaction mixture was cooled to RT, poured onto water (50 mL) and the precipitate was filtered off. The precipitate was washed with water (30 mL), methanol (30 mL) and diethyl ether (30 mL), dried and weighed to give 0.243 g of light powder. The crude product was adsorbed on silica gel and purified by flash column chromatography (DCM:PE, 50→75%) to give 0.121 g (49%) of a yellow powder. Melting point: mp> 330 °C.

The data of ¹H NMR and IR spectra of the obtained product:
¹H NMR (CDCl₃, 700 MHz), δ ppm: 6.56 (d, *J*=8.1 Hz, 4H, 4CH_{Ar}); 6.72-6.85 (m, 12H, 12CH_{Ar}); 7.40 (t, *J*=7.6 Hz, 2H,2CH_{Ar}); 7.45-7.57 (m, 6H, 6CH_{Ar}); 7.62 (d, *J*=8.7 Hz, 2H, 2CH_{Ar}); 7.70 (dd, *J₁*=8.5 Hz, *J₂*=1.8 Hz, 2H, 2CH_{Ar}); 7.89 (d, *J*=8.6 Hz, 4H, 4CH_{Ar}); 8.15 (dd, *J₁*=8.3 Hz, *J₂*=2.4 Hz, 2H, 2CH_{Ar}); 8.22 (d, *J*=7.7 Hz, 2H, 2CH_{Ar}); 8.34 (d, *J*=8.7 Hz, 4H, 4CH_{Ar}); 8.39 (s, 2H, 2CH_{Ar}); 9.01 (d, *J*=2.3 Hz, 2H, 2CH_{Ar}).

IR vₘₐₓ cm⁻¹: 3042.71(w), 1703.00(w), 1591.12(m), 1472.55(s), 1452.78(s), 1367.82(m), 1315.90(s), 1265.57(s), 129.37(m), 1152.94(m), 1103.15(m), 1026.12(m), 927.45(m), 806.75(m), 739.61(s), 603.03(m), 571.43(m), 419.52(m).

The above bis(4-(3-(2-(10*H*-phenoxazin-10-yl)pyridin-5-yl)-9*H*-carbazol-9-yl)phenyl)methanone, shown also below: 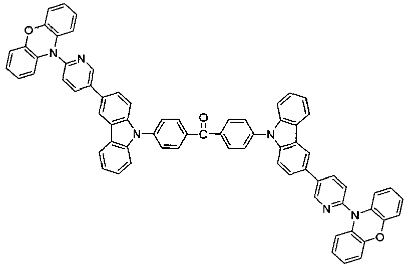 was synthesized by the following method:
Bis(4-fluorophenyl)methanone (0.041 g; 0.19 mmol), 10-(5-(9*H*-carbazole-3-yl)pyridin-2-yl)-10*H*-phenoxazine (0.200 g; 0.47 mmol) and cesium carbonate (0.153 g; 0.47 mmol) were mixed in dry DMSO (5 mL), and heated to 75°C. After 24h at this temperature, the reaction mixture was cooled to RT, water (50 mL) was added and the precipitate was filtered off. The precipitate was washed with water (30 mL), methanol (30 mL), diethyl ether (30 mL) and air-dried to give 0.171 g of light powder. The crude product was adsorbed on silica gel and purified by flash chromatography (DCM:PE, 50→75%) to give 0.082 g (42%) of a yellow powder. Melting point: mp=176-178°C.

The data of ¹H NMR and IR spectra of the obtained product:
¹H NMR (CDCl₃, 700 MHz), δ ppm: 6.54 (d, *J*=8.1 Hz, 4H, 4CH_{Ar}); 6.72-6.84 (m, 12H, 12CH_{Ar}); 7.41 (d, *J*=7.3 Hz, 2H, 2CH_{Ar}); 7.48 (d, *J*=8.2 Hz, 2H, 2CH_{Ar}); 7.52 (t, *J*=7.6 Hz, 2H, 2CH_{Ar}); 7.61 (d, *J*=8.5 Hz, 2H, 2CH_{Ar}); 7.68 (d, *J*=8.6 Hz, 2H, 2CH_{Ar}); 7.74 (dd, *J₁*=8.6 Hz, *J₂*=1.8 Hz, 2H, 2CH_{Ar}), 7.86 (d, *J*=8.5 Hz, 4H, 4CH_{Ar}); 8.18 (dd, *J₁*=8.5 Hz, *J₂*=2.5 Hz, 2H, 2CHAr); 8.21-8.27 (m, 6H, 6CHAr); 8.43 (d, *J*=1.5 Hz, 2H, 2CHAr); 9.04 (d, *J*=2.6 Hz, 2H, 2CHAr).

IR vₘₐₓ cm⁻¹: 3054.80(w), 2922.29(w), 1939.59(w), 1653.78(m), 1597.36(m), 1484.00(s), 1454.95(s), 1366.33(m), 1325.39(m), 1269.51(s), 1159.14(m), 1034.68(m), 928.37(m), 861.97(m), 805.11(m), 743.71(s), 686.99(m), 620.60(m), 581.50(m), 456.63(m), 421.96(m).

The above 9-(4-((4-fluorophenyl)sulfonyl)phenyl)-9H-carbazole shown also below: 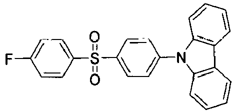 was synthesized by the following method:
Sodium hydride (60% dispersion in oil, 10 mmol; 0.400 g) was washed with dry petroleum ether and mixed with dry DMF (5 mL) under nitrogen. Carbazole (0.835 g, 5 mmol) dissolved in DMF (10 mL) was added dropwise and stirred for 30 min at RT. Then 4,4'-sulfonylbis(fluorobenzene) (2.54 g, 10 mmol) was added and stirred for 15 hours. Water (50 mL) was added and the resulting precipitate was filtered off, washed with methanol (20 mL) and dried to give 1.200 g (57%) of a white powder, which was taken to the next step without purification.

The data of ¹H NMR spectrum of the obtained product:
¹H NMR (CDCl₃, 400 MHz): 7.26 (t, *J*=8.9 Hz, 2H, CH_{Ar}); 7.32 (dd, *J₁*=7.4 Hz, *J₂*=1.4 Hz, 2H, CH_{Ar}); 7.41 (td, *J₁*=8.3 Hz, *J₂*=1.3 Hz, 2H, CH_{Ar}); 7.44 (d, *J*=8.2 Hz, 2H, CH_{Ar}); 7.76 (d, *J*=8.7 Hz, 2H, CH_{Ar}); 8.05-8.10 (m, 2H, CH_{Ar}); 8.13 (d, *J*=7.5 Hz, 2H, CHAr); 8.16 (d, *J*=8.6 Hz, 2H, CHAr).

The above 10-(5-(9-(4-((4-(9*H*-carbazol-9-yl)phenyl)sulfonyl)phenyl)-9*H*-carbazol-3-yl)pyridin-2-yl)-10*H*-phenoxazine shown also below: 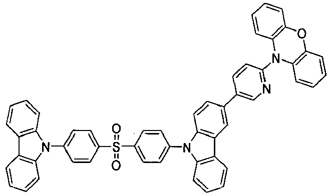 was synthesized by the following method:
9-(4-((4-fluorophenyl)sulfonyl)phenyl)-9*H*-carbazole (0.120 g; 0.30 mmol) from the previous step was mixed with 10-(5-(9*H*-carbazole-3-yl)pyridine-2-yl)-10H-phenoxazine (0.150 g; 0.35 mmol) and cesium carbonate (0.196 g; 0.60 mmol) in dry DMSO (5 mL) and heated to 75°C. After 24 h the reaction mixture was cooled to RT, water (50 mL) was added and the precipitate was filtered off. The precipitate was washed with water (30 mL), methanol (30 mL) and diethyl ether (30 mL) and air-dried to give 0.175 g of light powder. The crude product was adsorbed on silica gel and purified by flash chromatography (DCM:PE, 50→75%) to give 0.117 g (48%) of a yellow powder, mp=166-169°C.

The data of 1H NMR and IR spectrum of the obtained product:
¹H NMR (CDCl₃, 400 MHz), δ ppm: 6.58 (d, *J*=8.0 Hz, 2H, 2CH_{Ar}); 6.74-6.84 (m, 6H, 6CH_{Ar}); 7.34 (t, *J*=7.5 Hz, 2H, 2CH_{Ar}); 7.38-7.54 (m, 8H, 8CH_{Ar}); 7.61 (d, *J*=8.4 Hz, 1H, CH_{Ar}); 7.70 (d, *J*=8.8 Hz, 1H, CH_{Ar}); 7.86 (t, *J*=8.4 Hz, 4H, 4CH_{Ar}); 8.12-8.18 (m, 3H, 3CH_{Ar}); 8.22 (d, *J*=7.7 Hz, 1H, CH_{Ar}); 8.31 (t, *J*=8.9 Hz, 4H, 4CH_{Ar}); 8.39 (s, 1H, CH_{Ar}); 9.02 (d, *J*=2.2 Hz, 1H, CH_{Ar}).

IR vₘₐₓ cm⁻¹: 3065.10(w), 2341.12(m), 2226.42(w), 1645.97(m), 1594.65(s), 1488.29(s), 1452.96(m), 1402.90(m), 1271.58(s), 1151.16(m), 1104.33(m), 1041.04(m), 927.49(m), 841.46(m), 739.91(s), 685.45(m), 601.99(m), 566.89(m), 510.10(m), 420.48(m).

The above 1,4-phenylenebis((4-fluorophenyl)methanone) show also below: 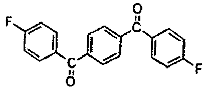 was synthesized by the following method:
Terephthaloyl chloride (2.54 g; 12.5 mmol) and fluorobenzene (2.80 g, 29 mmol) were mixed at RT, AlCl₃ (3.47 g; 26 mmol) was added in small portions and stirred for 20 h. Then poured onto crushed ice, the resulting precipitate was filtered off, washed with water (30 mL), methanol (20 mL), diethyl ether (20 mL) and dried to give 3.46 g (86%) of white powder. mp = 221.9-223.9°C.

The data of 1H NMR and IR spectra of the obtained product:
¹H NMR (CDCl3, 700 MHz), δ ppm: 7.20 (t, *J*=8.6 Hz, 4H, 4CH_{Ar}); 7.87 (s, 4H, 4CH_{Ar}); 7.87-7.91 (m, 4H, 4CHAr).

IR vₘₐₓ cm⁻¹: 3069.21(w), 2924.53(w), 2605.78(w), 2225.92(w), 1791.53(w), 1643.34(s), 1592.11(s), 1494.57(s), 1398.12(m), 1272.96(s), 1228.77(s), 1150.75(s), 1013.12(m), 968.25(m), 921.00(s), 840.82(s), 810.60(m), 740.03(s), 685.76(s), 624.30(m), 555.84(s), 506.22(s), 429.57(m).

The above 1,4-phenylenebis((4-(3-(2-(10*H*-phenoxazin-10-yl)pyridin-5-yl)-9*H-*carbazol-9-yl)phenyl)methanone) shown also below: 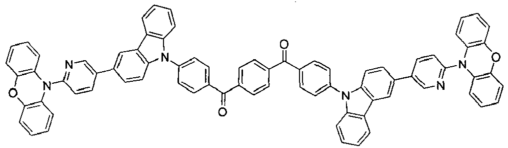 was synthesized by the following method:
4-Phenylene-bis((4-fluorophenyl)methanone) (0.106 g; 0.33 mmol), 10-(5-(9*H-*carbazol-3-yl)pyridin-2-yl)-10*H*-phenoxazine (0.426 g; 1 mmol) and cesium carbonate (0.326 g; 1 mmol) were mixed in dry DMSO (5 mL) and heated to 75°C. After 24 h at this temperature, the reaction mixture was cooled to RT, water (50 mL) was added and the precipitate was filtered off. The precipitate was washed with water (30 mL), methanol (30 mL), diethyl ether (30 mL) and dried to give 0.470 g of a green-yellow powder. The crude product was adsorbed on silica gel and purified by flash chromatography (DCM:PE, 50%→100%DCM) to obtain 0.198 g (53%) of pure product as a dark yellow powder. mp> 330°C.

The data of ¹H NMR and IR spectra of the obtained product:
¹H NMR (CDCl₃, 700 MHz), δ ppm: 6.55 (s, br, 4H, 4CH_{Ar}); 6.72-6.86 (m, 12H, 12CHAr); 7.40 (t, *J*=7.4 Hz, 2H, 2CHAr); 7.48 (d, *J*=8.1 Hz, 2H, 2CHAr); 7.52 (t, *J*=7.7 Hz, 2H, 2CH_{Ar}); 7.59 (d, *J*=8.3 Hz, 2H2CH_{Ar}); 7.67 (d, *J*=8.4 Hz, 2H, 2CH_{Ar}); 7.73 (d, *J*=8.5 Hz, 2H, 2CH_{Ar}); 7.83 (d, *J*=8.5 Hz, 4H, 4CH_{Ar}); 8.08 (s, 4H, 4CH_{Ar}); 8.18 (d, *J*=8.4 Hz, 6H, 6CH_{Ar}); 8.24 (d, *J*=7.7 Hz, 2H, 2CH_{Ar}); 8.42 (s, 2H, 2CH_{Ar}); 9.04 (brs, 2H, 2CH_{Ar}).

IR vₘₐₓ cm⁻¹: 3061.82(w), 2983.12(w), 2615.01(w), 2226.57(w), 1657.67(m), 1597.06(m), 1485.15(s), 1453.53(s), 1329.27(m), 1268.95(s), 1220.09(m), 1154.64(m), 1040.50(m), 922.84(m), 860.49(m), 838.88(m), 806.02(m), 735.17(s), 692.34(m), 619.09(m), 583.12(m), 462.20(m), 422.61(m).

**EMBODIMENT 3** - method for synthesizing of TADF compounds comprising the donor structure of Formula I. The obtained TADF compounds are as follows:
4-(3,6-Bis(2-(10*H*-phenoxazin-10-yl)pyridin-5-yl)-9*H*-carbazol-9-yl)benzonitrile,
(4-(3,6-Bis(2-(10*H*-phenoxazin-10-yl)pyridin-5-yl)-9*H*-carbazol-9-yl)phenyl)(phenyl)methanone,
10,10'-((9-(4-(Methylsulfonyl)phenyl)-9*H*-carbazole-3,6-diyl)bis(pyridine-5,2-diyl))bis(10*H*-phenoxazine),
Bis(4-(3,6-bis(2-(10*H*-phenoxazin-10-yl)pyridin-5-yl)-9*H*-carbazol-9-yl)phenyl)methanone,
10,10',10",10‴-(((Sulfonylbis(4,1-phenylene))bis(9*H*-carbazole-9,3,6-triyl))tetrakis(pyridine-5,2-diyl))tetrakis(10*H*-phenoxazine),
10,10'-((9-(4-((4-(9*H*-Carbazol-9-yl)phenyl)sulfonyl)phenyl)-9*H*-carbazole-3,6-diyl)bis(pyridine-5,2-diyl))bis(10*H*-phenoxazine),

Compounds VI and X are not encompassed by the wording of the claims but are considered as useful for understanding the invention.

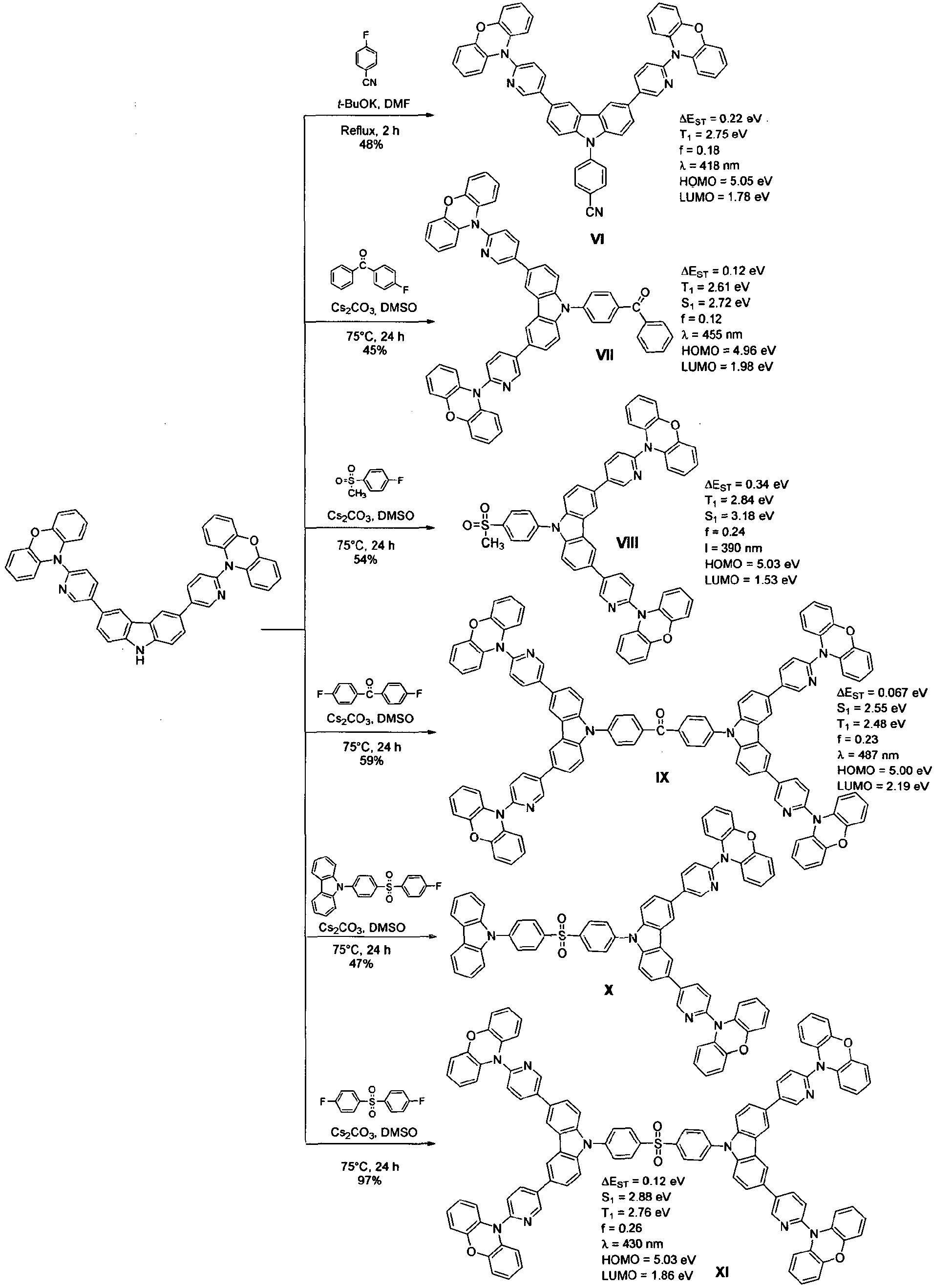

The obtained in Embodiment 1 3,6-bis(2-(10*H*-phenoxazin-10-yl)pyridin-5-yl)-9*H-*carbazole served as a substrate for synthesis according to EMBODIMENT 3.

The above 4-(3,6-bis(2-(10*H*-phenoxazin-10-yl)pyridin-5-yl)-9*H*-carbazol-9-yl)benzonitrile shown also below: 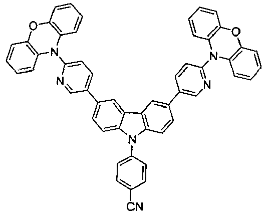 was synthesized by the following method:
3,6-Bis(2-(10*H*-phenoxazine-10-yl)pyridine-5-yl)-9*H*-carbazole (0.250 g; 0.37 mmol), 4-fluorobenzonitrile (0.90 g; 0.74 mmol) and potassium tert-butoxide (0.083 g; 0.74 mmol) in dry DMF (5 mL) were heated to reflux for 20 hours. Cooled to RT and water (10 mL) was added, extracted with chloroform (3×20 mL). The combined extracts were washed with water (2×50 mL), brine (50 mL), dried with anhydrous magnesium sulfate and the solvent was removed. The crude product was adsorbed on silica gel and purified by flash chromatography (DCM) to give 0.138 g (48%) of a light yellow powder. mp> 310°C.

The data of 1H NMR and IR spectra of the obtained product:
¹H NMR (CDCl3, 700 MHz), δ ppm: 6.69 (d, J=7.5 Hz, 4H4CHAr); 6.79-6.88 (m, 12H, 12CHAr); 7.49 (d, J=8.4 Hz, 2H, 2CHAr); 7.61 (d, J=8.5 Hz, 2H, 2CHAr); 7.75 (d, J=8.5 Hz, 2H, 2CHAr); 7.82 (d, J=8.6 Hz, 2H, 2CHAr); 7.99 (d, J=8.5 Hz, 2H, 2CHAr); 8.19 (dd, J1=8.3 Hz, J2=2.5 Hz, 2H, 2CHAr); 8.47 (d, J=1.6 Hz, 2H, 2CHAr); 9.04 (d, J=2.6 Hz, 2H, 2CHAr).

IR vₘₐₓ cm⁻¹: 3052.50(w), 2924.20(w), 2228.32(w), 1596.12(m), 1483.80(s), 1369.81(m), 1331.76(m), 1271.14(s), 1231.82(m), 1038.63(m), 925.35(w), 866.89(m), 813.15(m), 738.85(s), 644.31(w), 611.77(m), 583.42(m), 546.84(m), 424.66(m).

The above (4-(3,6-bis(2-(10*H*-phenoxazin-10-yl)pyridin-5-yl)-9*H*-carbazol-9-yl)phenyl)(phenyl)methanone shown also below: 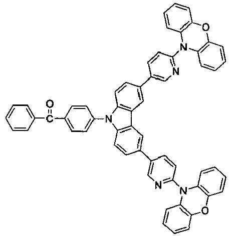 was synthesized by the following method:
(4-Fluorophenyl)(phenyl)methanone (0.120 g; 0.60 mmol), 3,6-bis(2-(10*H*-phenoxazine-10-yl)pyridine-5-yl)-9*H*-carbazole (0.173 g; 0.20 mmol) and cesium carbonate (0.196 g; 0.60 mmol) were mixed in dry DMSO (5 mL) and heated to 75°C. After 24 h the reaction mixture was cooled to RT, water (50 mL) was added and extracted with chloroform (3×20 mL). The combined extracts were washed with water (3×50 mL), brine (50 mL), dried with anhydrous magnesium sulfate and evaporated to dryness. The crude product was adsorbed on silica gel and purified by flash chromatography (DCM:PE, 1:1) to give 0.075 g (45%) of a yellow powder. mp=156-158°C.

The data of ¹H NMR and IR spectra of the obtained product:
¹H NMR (CDCl₃, 400 MHz), δ ppm: 6.60 (d, *J*=8.0 Hz, 4H, 4CH_{Ar}); 6.75-6.85 (m, 12H, 12CHAr); 7.49 (d, *J*=8.1 Hz, 2H, 2CHAr); 7.58 (d, *J*=7.7 Hz, 2H, 2CHAr); 7.65-7.70 (m, 3H, 3CHAr); 7.77 (d, *J*=8.6 Hz, 2H, 2CHAr); 7.81 (d, *J*=8.4 Hz, 2H, 2CH_{Ar}); 7.94 (d, *J*=8.0 Hz, 2H, 2CH_{Ar}); 8.15 (d, *J*=8.4 Hz, 2H, 2CH_{Ar}); 8.19 (d, *J*=8.5 Hz, 2H, 2CH_{Ar}); 8.49 (s, 2H, 2CHAr); 9.05 (s, 2H, 2CHAr).

IR vₘₐₓ cm⁻¹: 3058.34(w), 2923.21(w), 2225.03(w), 1903.31(w), 1654.34(m), 1595.90(m), 1482.27(s), 1370.10(m), 1325.79(m), 1266.93(s), 1168.00(m), 1037.55(m), 924.36(m), 855.47(m), 803.97(m), 735.07(s), 696.99(m), 590.91(m), 464.12(m), 413.66(m).

The above 10,10'-((9-(4-(methylsulfonyl)phenyl)-9*H*-carbazole-3,6-diyl)bis(pyridine-5,2-diyl))bis(10H-phenoxazine) shown also below: 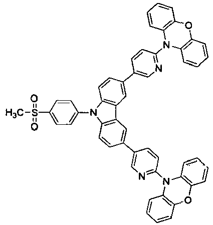 was synthesized by the following method:
1-Fluoro-4-(methylsulfonyl)benzene (0.105 g; 0.60 mmol), 3,6-bis(2-(10*H*-phenoxazine-10-yl)pyridine-5-yl)-9*H*-carbazole (0.173 g; 0.20 mmol) and cesium carbonate (0.196 g; 0.60 mmol) in dry DMSO (5 mL) were heated to 75°C. After 24 h, the reaction mixture was cooled to RT, water (50 mL) was added and extracted with chloroform (3×20 mL). The combined extracts were washed with water (3×50 mL), brine (50 mL), dried with anhydrous magnesium sulfate and evaporated to dryness. The crude product was adsorbed on silica gel and purified by flash chromatography (chloroform) to give 0.090 g (54%) of a dark yellow powder, mp=142-144°C.

The data of ¹H NMR and IR spectrum of the obtained product:
¹H NMR (CDCl₃, 700 MHz), δ ppm: 3.22 (s, 3H, 3CH₃); 6.66 (d, J=7.8 Hz, 4H, 4CH_{Ar}); 6.78-6.87 (m, 12H, 12CH_{Ar}); 7.49 (d, *J*=8.2 Hz, 2H, 2CH_{Ar}); 7.63 (d, *J*=8.4 Hz, 2H, 2CH_{Ar}); 7.76 (dd, *J₁*=8.4 Hz, *J₂*=1.9 Hz, 2H, 2CH_{Ar}); 7.91 (d, *J*=8.5 Hz, 2H, 2CH_{Ar}); 8.19 (dd, *J₁*=8.2 Hz, *J₂*=2.7 Hz, 2H, 2CH_{Ar}); 8.28 (d, *J*=8.4 Hz, 2H, 2CH_{Ar}); 8.48 (d, *J*=1.6 Hz, 2H, 2CHAr); 9.04 (d, *J*=2.4 Hz, 2H, 2CHAr).

IR vₘₐₓ cm⁻¹: 3058.34(w), 2923.21(w), 2225.03(w), 1903.31(w), 1654.34(m), 1595.90(m), 1482.27(s), 1370.10(m), 1325.79(m), 1266.93(s), 1168.00(m), 1037.55(m), 924.36(m), 855.47(m), 803.97(m), 735.07(s), 696.99(m), 590.91(m), 464.12(m), 413.66(m).

The above bis(4-(3,6-bis(2-(10*H*-phenoxazin-10-yl)pyridin-5-yl)-9*H*-carbazol-9-yl)phenyl)methanone shown also below: 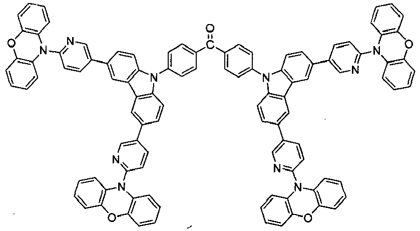 was synthesized by the following method:
Bis(4-fluorophenyl)methanone (0.052 g; 0.24 mmol), 3,6-bis(2-(10*H*-phenoxazine-10-yl)pyridine-5-yl)-9*H*-carbazole (0.400 g; 0.60 mmol) and cesium carbonate (0.196 g; 0.60 mmol) in dry DMSO (5 mL) were heated to 75°C. After 24h the reaction mixture was cooled to RT, water (50 mL) was added and the precipitate was filtered off. The precipitate was washed with water (30 mL), methanol (30 mL), diethyl ether (30 mL) and air dried to give 0.307 g of light green powder. The crude product was adsorbed on silica gel and purified by flash chromatography (DCM:PE, 1:1) to give 0.220 g (59%) of pure product as a light green powder, mp=209-211°C.

The data of 1H NMR and IR spectra of the obtained product:
¹H NMR (CDCl₃, 400 MHz), δ ppm: 6.59 (d, *J*=7.9 Hz,8H, 8CH_{Ar}); 6.74-6.86 (m, 24H, 24CHAr); 7.50 (d, *J=*8.3 Hz, 4H, 4CH_{Ar}); 7.73 (d, *J*=8.5 Hz, 4H, 4CHAr); 7.79 (dd, *J₁*=8.6 Hz, *J₂*=1.8 Hz, 4H, 4CH_{Ar}); 7.91 (d, *J*=8.6 Hz, 4H, 4CH_{Ar}); 8.19 (dd, *J₁*=8.3 Hz, *J₂*=2.6 Hz, 4H, 4CHAr); 8.28 (d, *J*=8.5 Hz, 4H, 4CHAr); 8.50 (d, *J*=1.4 Hz, 4H,4CHAr); 9.05 (d, *J*=2.6 Hz, 4H, 4CHAr).

IR vₘₐₓ cm⁻¹: 3059.45(w), 2923.10(w), 2226.51(w), 1654.22(m), 1595.80(m), 1482.47(s), 1455.19(s), 1370.97(m), 1327.20(m), 1266.75(s), 1154.73(m), 1037.38(m), 925.42(m), 857.89(m), 804.89(m), 737.15(s), 581.51(m), 461.31(m).

The above 10,10',10",10‴-(((sulfonylbis(4,1-phenylene))bis(9*H*-carbazole-9,3,6-triyl))tetrakis(pyridine-5,2-diyl))tetrakis(10*H*-phenoxazine) shown also below: 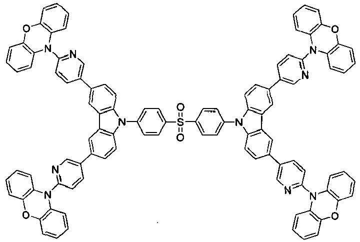 was synthesized by the following method:
4,4'-Sulfonylbis(fluorobenzene) (0.060 g; 0.24 mmol), 3,6-bis(2-(10*H*-phenoxazine-10-yl)pyridine-5-yl)-9*H*-carbazole (0.400 g; 0.60 mmol) and cesium carbonate (0.196 g; 0.60 mmol) were mixed in dry DMSO (5 mL) and heated to 75°C. After 24 h, the reaction mixture was cooled to RT, water (50 mL) was added and the precipitate was filtered off. The precipitate was washed with water (30 mL), methanol (30 mL), diethyl ether (30 mL) and air dried to give 0.388 g (97%) of pure product as a pale yellow powder. mp>330°C.

The data of ¹H NMR and IR spectra of the obtained product:
1H NMR (CDCl3, 400 MHz), δ ppm: 6.61 (d, J=7.8 Hz, 8H, 8CHAr); 6.73-6.87 (m, 24H, 24CHAr); 7.48 (d, J=8.3 Hz, 4H, 4CHAr); 7.66 (d, J=8.7 Hz, 4H, 4CHAr); 7.76 (d, J=8.4 Hz, 4H, 4CHAr); 7.94 (d, J=8.3 Hz, 4H, 4CHAr); 8.16 (d, J=8.3 Hz, 4H, 4CHAr); 8.39 (d, J=8.4 Hz, 4H, 4CHAr); 8.47 (s, 4H, 4CHAr); 9.02 (s, 4H, 4CHAr).

IR vmax cm-1: 2060.97(w), 29922.34(w), 2226.57(w), 1645.63(m), 1594.48(m), 1485.02(s), 1455.06(s), 1371.18(m), 1326.39(m), 1271.96(s), 1153.32(m), 1104.44(m), 1041.44(m), 928.81(m), 864.94(m), 805.10(m), 741.70(s), 692.85(m), 614.21(m), 579.12(m), 461.15(m), 418.24(m).

The above 10,10'-((9-(4-((4-(9*H*-carbazol-9-yl)phenyl)sulfonyl)phenyl)-9*H*-carbazole-3,6-diyl)bis(pyridine-5,2-diyl))bis(10*H*-phenoxazine), shown also below: 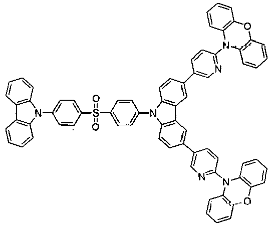 was synthesized by the following method:
9-(4-((4-Fluorophenyl)sulfonyl)phenyl)-9*H-*carbazole (0.120 g; 0.30 mmol), 3,6-bis(2-(10*H*-phenoxazine-10-yl)pyridine-5-yl)-9*H*-carbazole (0.257 g; 0.38 mmol) and cesium carbonate (0.196 g; 0.60 mmol) in dry DMSO (5 mL) were heated to 75°C. After 24 h the reaction mixture was cooled to RT, water (50 mL) was added and the precipitate was filtered off. The precipitate was washed with water (30 mL), methanol (30 mL), diethyl ether (30 mL) and air-dried to give 0.336 g of light powder. The crude product was adsorbed on silica gel and purified by flash chromatography (DCM:PE, 50%→75% DCM) to give 0.148 g (47%) of a yellow powder, mp=293.8-295.9°C.

The data of ¹H NMR and IR spectra of the obtained product:
¹H NMR (CDCl₃, 400 MHz), δ ppm: 6.58 (d, *J*=7.9 Hz, 4H, 4CH_{Ar}); 6.73-6.85 (m, 12H, 12CHAr); 7.34 (t, *J*=7.4 Hz, 2H, 2CH_{Ar}); 7.44 (t, *J*=7.7 Hz, 2H, 2CHAr); 7.46-7.53 (m, 4H, 4CH_{Ar}); 7.65 (d, *J*=8.4 Hz, 2H, 2CH_{Ar}); 7.75 (dd, *J₁*=8.7 Hz, *J₂*=1.7 Hz, 2H, 2CH_{Ar}); 7.87 (d, *J*=8.6 Hz, 2H, 2CHAr); 7.91 (d, J=8.7 Hz, 2H, 2CHAr); 8.13-8.18 (m, 4H, 4CHAr); 8.31 (d, *J*=8.7 Hz, 2H, 2CH_{Ar}); 8.35 (d, J=8.7 Hz, 2H, 2CH_{Ar}); 8.46 (d, *J*=1.6 Hz, 2H, 2CH_{Ar}); 9.01 (d, *J*=2.4 Hz, 2H, 2CHAr).

IR vₘₐₓ cm⁻¹: 3060.70(w), 2927.87(w), 2227.09(w), 1737.35(w), 1592.96(m), 1484.40(s), 1452.90(m), 1328.12(s), 1271.58(s), 1153.09(m), 1103.26(m), 1040.59(m), 927.12(m), 866.11(m), 810.67(m), 740.15(s), 680.77(m), 608.34(m), 574.52(m), 420.23(m).

### EMBODIMENT 4 - method for synthesizing of TADF compounds comprising the donor structure of Formula I: 9,9'-(sulfonylbis(4,1-phenylene))bis(3-(2-(9H-carbazol-9-yl)pyridin-5-yl)-9H-carbazole).

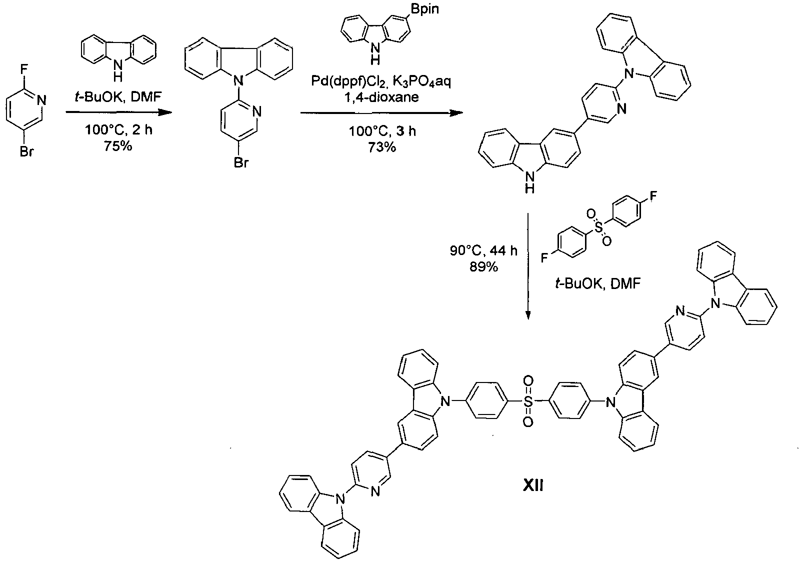

The above 9-(5-bromopyridin-2-yl)-9*H*-carbazole shown also below: 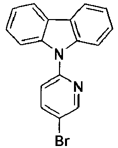 was synthesized by the following method:
Carbazole (1.67 g; 0.01 mol), 5-bromo-2-fluoropyridine (5.28 g; 0.03 mol) and potassium *tert*-butoxide (3.66 g; 0.03 mol) were dissolved in DMF (50 mL) and stirred at 100°C for 2 h. Then the reaction was cooled to RT, water (150 mL) was added and extracted with chloroform (3×100 mL). The combined organic layers were washed with water (3×100 mL), brine (150 mL) and dried with anhydrous magnesium sulfate. The solvent was evaporated on a rotary evaporator to give 8.055 g of crude product. The product was purified by flash chromatography (AcOEt:PE - 5:95) to give 2.414 g (75%) of pure product. mp=110.4-111.0°C.

The data of 1H NMR and IR spectra of the obtained product:
¹H NMR (CDCl₃, 700 MHz), δ ppm: 8.77 (s, 1H, CH_{Ar}), 8.11 (dd, *J*=7.8 Hz, 2H, 2CH_{Ar}), 8.03 (dd, *J*=8.5 Hz, 2.4 Hz, 1H, CH_{Ar}), 7.82 (d, *J*=8.1 Hz, 2H, 2CH_{Ar}), 7.57 (d, *J*=8.4 Hz, 1H, CH_{Ar}), 7.45 (td, *J*=7.8 Hz, 1.4 Hz, 2H, 2CH_{Ar}), 7.33 (t, *J*=7.4 Hz, 2H, 2CH_{Ar}).

IR vₘₐₓ cm⁻¹: 3014.55(w), 2659.58(w), 2276.48(w), 2085.09(w), 1987.41(w), 1892.80-(w), 1845.00(w), 1743.02(s), 1565.72(s), 1440.60(s), 1370.13(m), 1317.43(m), 1217.02(m), 1174.30(m), 1129.78(w), 1089.26(m), 1000.38(m), 969.18(w), 913.77(m), 848.48(s), 817.38(s), 742.46(s), 714.70(s), 670.19(w), 615.26(m), 588.00(w), 568.74(m), 529.08(m), 497.78(m), 440.00(w), 419.87(w).

The above 3-(2-(9*H*-carbazol-9-yl)pyridin-5-yl)-9*H*-carbazole shown also below: 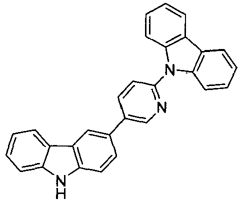 was synthesized by the following method:
3-(4,4,5,5-Tetramethyl-1,3,2-dioxaborolan-2-yl)-9*H*-carbazole (1.465 g; 5 mmol), 9-(5-bromopyridin-2-yl)-9H-carbazole (1.777 g; 5 mmol) and Pd(dppf)Cl₂•DCM (110 mg; 0.15 mmol) were dissolved in 1,4-dioxane (20 mL) under an inert atmosphere. K₃PO₄ (3M aqueous; 18 mL; 54 mmol) was added at RT, heated to 100°C and stirred at this temperature for 3 h. Water (50 mL) was added and extracted with chloroform (3×50 mL). The combined organic layers were washed with water (3×50 mL), brine (100 mL) and dried with anhydrous magnesium sulfate. The solvent was evaporated, yielding 1.90 g of crude product. The product was purified on a flash column (DCM:PE, 75%) to give 1.49 g (73%) of pure product. mp=246-250°C.

The data of ¹H NMR and IR spectra of the obtained product:
1H NMR (DMSO-d6, 700 MHz), δ ppm: 11.42 (s, 1H, NH), 9.17 (s, 1H, CHAr), 8.67 (s, 1H, CHAr), 8.50 (dd, J=8.4 Hz, 2.6 Hz, 1H, CHAr), 8.27 (t, J=7.0 Hz, 3H, 3CHAr), 7.89 (d, J=8.3 Hz, 4H, 4CHAr), 7.66 (d, J=8.7 Hz, 1H, CHAr), 7.52 (q, J=8.1 Hz, 3H, 3CHAr), 7.44 (t, J=7.8 Hz, 1H, CHAr), 7.36 (t, J=7.4 Hz, 2H, 2CHAr), 7.22 (t, J=7.4 Hz, 1H, CHAr). IR vmax cm-1: 3423.60(s), 3051.50(s), 3009.30(w), 2096.83(w), 1920.50(w), 1891.93(w), 1871.30(w), 1737.99(s), 1594.40(m), 1561.86(m), 1450.69(s), 1374.93(m), 1322.92 (m), 1288.25(w), 1222.74(s), 1187.12(w), 1131.71(m), 1001.20(m), 929.26(m), 896.00(w), 864.88(w), 846.95(w), 814.23(s), 741.87(s), 722.21(s), 620.51(s), 568.09(s), 530.16(w), 495.75(w), 423.72(m).

The above 9,9'-(sulfonylbis(4,1-phenylene))bis(3-(2-(9*H*-carbazol-9-yl)pyridin-5-yl)-9*H-*carbazole) shown also below: 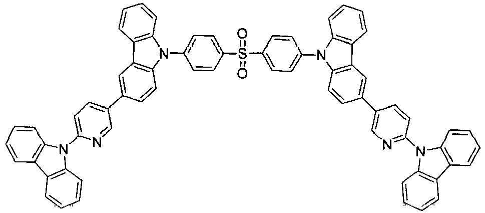 was synthesized by the following method:
3-(2-(9*H*-Carbazol-9-yl)pyridin-5-yl)-9*H*-carbazole (512 mg; 1.25 mmol) was dissolved in anhydrous DMF (5 mL) and potassium *tert*-butoxide (140 mg; 1.25 mmol) was added under argon and stirred at 90°C for 10 min. 4,4'-Sulfonylbis(fluorobenzene) (127 mg; 0.5 mmol) was added and stirred at 90°C for 44 h. The mixture was cooled to RT and poured into methanol (20 mL). The resulting precipitate was filtered off and allowed to dry to give 460 mg (89%) of pure product.

The data of ¹H NMR spectrum of the obtained product:
¹H NMR 400 MHz (CDCl₃): δ ppm: 9.09 (s, 2H, 2CH_{Ar}), 8.47 (s, 2H, 2CH_{Ar}), 8.37 (d, *J*=8.6 Hz, 4H, 4CH_{Ar}), 8.29-8.24 (m, 4H, 4CH_{Ar}), 8.17 (d, *J*=7.7 Hz, 4H, 4CH_{Ar}), 7.96-7.91 (m, 8H, 8CHAr), 7.79 (d, *J*=8.3 Hz, 4H, 4CH_{Ar}), 7.68 (d, *J*=8.6 Hz, 2H, 2CHAr), 7.60-7.47 (m, 8H, 8CH_{Ar}), 7.43 (t, *J*=7.4 Hz, 2H, 2CH_{Ar}), 7.36 (t, *J*=7.5 Hz, 4H, 4CH_{Ar}).

Embodiment 5 is not encompassed by the wording of the claims but are considered as useful for understanding the invention.

### EMBODIMENT 5 - synthesis of 1,4-phenylenebis((4-(3-(2-(9H-carbazol-9-yl)pyridin-5-yl)-9H-carbazol-9-yl)phenyl)methanone)

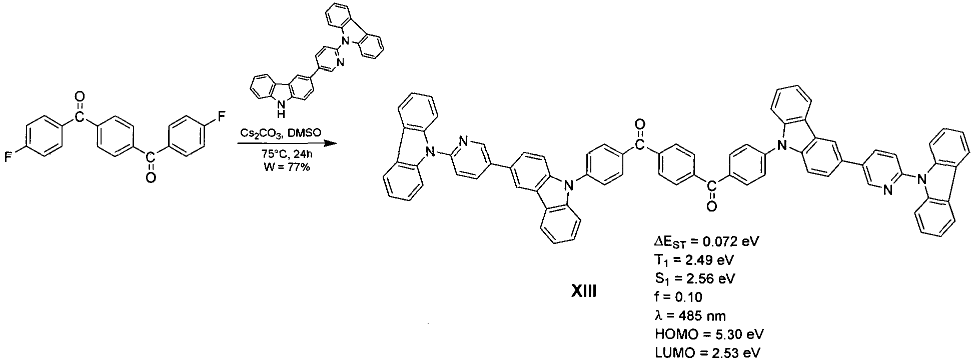

The above 3-(2-(9*H*-zarbazol-9-yl)pyridin-5-yl)-9*H*-carbazole shown also below: 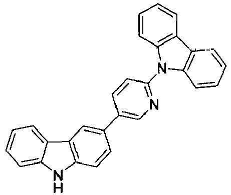 was synthesized by the following method:
3-(4,4,5,5-Tetramethyl-1,3,2-dioxaborolan-2-yl)-9*H*-carbazole (1.465 g; 5 mmol), 9-(5-bromopyridin-2-yl)-9*H*-carbazole (1.777 g; 5m5mol) and Pd(dppf)Cl₂·DCM (110 mg; 0.15 mmol) were dissolved in 1,4-dioxane (20 mL) under an inert atmosphere. K₃PO₄ (3M aqueous; 18 mL; 54 mmol) was added at RT, heated to 100°C and stirred at this temperature for 3 h. Water (50 mL) was added and extracted with chloroform (3×50 mL). The combined organic layers were washed with water (3×50 mL), brine (100 mL) and dried with anhydrous magnesium sulfate. The solvent was evaporated, yielding 1.90 g of crude product. The product was purified on a flash column (DCM:PE, 75%) to give 1.49 g (73%) of pure product. mp=246-250°C.

The data of ¹H NMR and IR spectra of the obtained product:
¹H NMR (DMSO-d₆, 700 MHz), δ ppm: 11.42 (s, 1H, NH), 9.17 (s, 1H, CH_{Ar}), 8.67 (s, 1H, CH_{Ar}), 8.50 (dd, *J*=8.4 Hz, 2.6 Hz, 1H, CH_{Ar}), 8.27 (t, *J*=7.0 Hz, 3H, 3CH_{Ar}), 7.89 (d, *J*=8.3 Hz, 4H, 4CH_{Ar}), 7.66 (d, *J*=8.7 Hz, 1H, CH_{Ar}), 7.52 (q, *J*=8.1 Hz, 3H, 3CH_{Ar}), 7.44 (t, *J*=7.8 Hz, 1H, CH_{Ar}), 7.36 (t, *J*=7.4 Hz, 2H, 2CH_{Ar}), 7.22 (t, *J*=7.4 Hz, 1H, CH_{Ar}). IR vₘₐₓ cm⁻¹: 3423.60(s), 3051.50(s), 3009.30(w), 2096.83(w), 1920.50(w), 1891.93(w), 1871.30(w), 1737.99(s), 1594.40(m), 1561.86(m), 1450.69(s), 1374.93(m), 1322.92 (m), 1288.25(w), 1222.74(s), 1187.12(w), 1131.71(m), 1001.20(m), 929.26(m), 896.00(w), 864.88(w), 846.95(w), 814.23(s), 741.87(s), 722.21(s), 620.51(s), 568.09(s), 530.16(w), 495.75(w), 423.72(m).

The above 1,4-phenylenebis((4-(3-(2-(9*H*-carbazol-9-yl)pyridin-5-yl)-9*H*-carbazol-9-yl)phenyl)methanone): 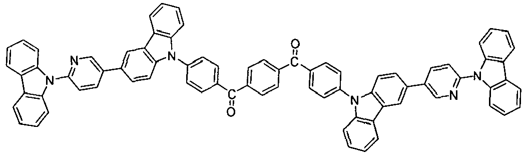 was synthesized by the following method:
1,4-Phenylenebis((4-fluorophenyl)methanone) (0.106 g; 0.33 mmol), 3-(2-(9*H*-carbazole-9-yl)pyridin-5-yl)-9*H*-carbazole (0.410 g; 1 mmol) and cesium carbonate (0.326 g; 1 mmol) in dry DMSO (5 mL) were heated to 75°C. After 24 h the reaction mixture was cooled to RT, water (50 mL) was added and the precipitate was filtered off. The precipitate was washed with water (30 mL), methanol (30 mL), diethyl ether (30 mL) and air dried to give 0.885 g of orange powder. The crude product was adsorbed on silica gel and purified by flash chromatography (DCM:PE, 50%→100% DCM) to give 0.279 g (77%) of the pure product as a light yellow powder. mp=309-312°C.

The data of ¹H NMR and IR spectra of the obtained product:
¹H NMR (CDCl₃, 700 MHz), δ ppm: 7.35 (t, *J*=7.5 Hz, 4H, 4CH_{Ar}); 7.40-7.45 (m, 6H, 6CH_{Ar}); 7.49 (t, *J*=7.7 Hz,4H, 4CH_{Ar}); 7.61 (d, *J*=8.2 Hz, 2H, 2CH_{Ar}); 7.70 (d, *J*=8.4 Hz, 2H, 2CHAr); 7.78 (d, *J*=8.1 Hz, 2H, 2CHAr); 7.85 (d, *J*=8.5 Hz, 4H, 4CH_{Ar}); 7.93 (d, *J*=8.2 Hz, 4H, 4CH_{Ar}); 8.10 (s, 4H, 4CH_{Ar}); 8.15 (d, *J*=7.8 Hz,4H, 4CH_{Ar}); 8.20 (d, *J*=8.2 Hz; 4H, 4CHAr); 8.27 (d, *J*=8.3 Hz, 4H, 4CHAr); 8.47 (d, J=1.7 Hz, 2H, 2CHAr); 9.09 (d, *J*=2.4 Hz, 2H, 2CH_{Ar}).

IR vₘₐₓ cm⁻¹: 3051.50(w), 1920.61(w), 1708.00(m), 1648.61(m), 1593.02(m), 1554.30(m), 1458.68(s), 1371.89(m), 1322.90(m), 1266.77(m), 1228.88(s), 1154.16(m), 1019.01(m), 921.47(m), 809.01(m), 738.99(s), 695.07(m), 619.56(m), 567.13(m), 420.78(m).

### EMBODIMENT 6 - synthesis of Bis(4-(3-(2-(9H-carbazol-9-yl)pyridin-5-yl)-9H-carbazol-9-yl)phenyl)methanone

The Bis(4-(3-(2-(9*H*-carbazol-9-yl)pyridin-5-yl)-9*H*-carbazol-9-yl)phenyl)methanone shown below: 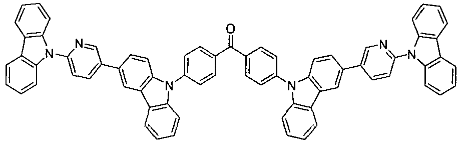 was synthesized by the following method:
3-(2-(9*H*-Carbazol-9-yl)pyridin-5-yl)-9*H*-carbazole (512 mg; 1.25 mmol) was dissolved in anhydrous DMF (5 mL). Potassium tert-butoxide (140 mg; 1.25 mmol) was added and the mixture was stirred at 90°C for 10 min. in an inert gas atmosphere. Bis(4-fluorophenyl)methanone (109 mg; 0.5 mmol) was added and the reaction was stirred at 90°C for 44 h. Cooled to RT and poured into methanol (20 mL). The resulting precipitate was filtered off and allowed to dry to obtain 443 mg of crude product. The product was purified on a flash column using (DCM:PE 50%→100% DCM) to give 222 mg (45%) of pure product. mp=224-360°C.

The data of ¹H NMR, ¹³C NMR and IR spectrum of the obtained product:
¹H NMR (CDCl3, 400 MHz): δ ppm: 9.12 (s, 2H, 2CHAr), 8.50 (s, 2H, 2CHAr), 8.32-8.25 (m, 8H, 8CHAr), 8.18 (d, J=7.8 Hz, 4H, 4CHAr), 7.95 (d, J=8.3 Hz, 4H, 4CHAr), 7.90 (d, J=8.5 Hz, 4H, 4CHAr), 7.81 (t, J=8.5 Hz, 4H, 4CHAr), 7.74 (d, J=8.4 Hz, 2H, 2CHAr), 7.84 (d, J=8.2 Hz, 2H, 2CHAr), 7.56 (t, J=7.7 Hz, 2H, 2CHAr), 7.51 (t, J=7.8 Hz, 4H, 4CHAr), 7.44 (t, J=7.4 Hz, 2H, 2CHAr), 7.37 (t, J=7.6 Hz, 4H, 4CHAr).

¹³C NMR (CDCl3, 100 MHz): δ ppm: 194.33, (C), 150.40 (2CAr), 147.95 (2CHAr), 141.68 (2CAr), 140.88 (2CAr), 140.40 (2CAr), 139.69 (4CAr), 136.87 (2CHAr), 136.10 (2CAr), 134.87 (2CAr), 132.00 (4CHAr), 130.04 (2CAr), 126.86 (2CHAr), 126.52 (4CHAr), 126.24 (4CHAr), 125.44 (2CHAr), 124.80 (2CAr), 124.36 (4CAr), 123.77 (2CAr), 121.14 (2CHAr), 120.93 (4CHAr), 120.70 (2CHAr), 120.25 (4CHAr), 119.05 (2CHAr), 118.92 (2CHAr), 111.22 (4CHAr), 110.60 (2CHAr), 110.11 (2CHAr).

IR vmax cm-1: 3045.38(m), 2927.22(w), 2082.95(w), 1984.51(w), 1933.40(w), 1889.40(w), 1740.00(m), 1672.44(s), 1596.79(s), 1450.91(s), 1381.07(w), 1322.15(m), 1291.00(w), 1266.09(w), 1224.55(m), 1164.52(m), 115.07(w), 1014.57(m), 928.16(s), 848.35(m), 804.92(m), 745.19(s), 724.00(w), 680.47(w), 647.33(w), 615.89(m), 566.62(m), 531.20(m), 504.44(w), 480.00(w), 420.42(s).

Embodiment 7 is not encompassed by the wording of the claims but are considered as useful for understanding the invention.

### EMBODIMENT 7 - synthesis of compounds XV-XVIII, shown below by the semi-structural formulae:

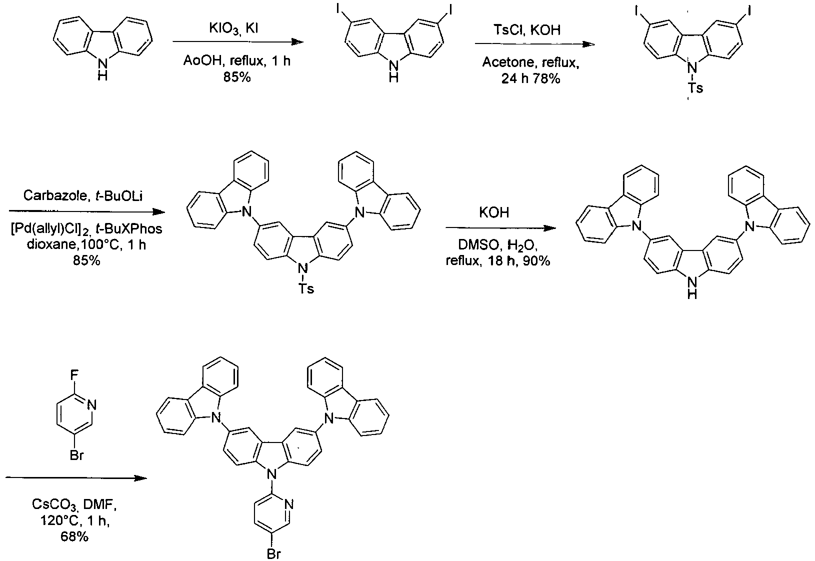

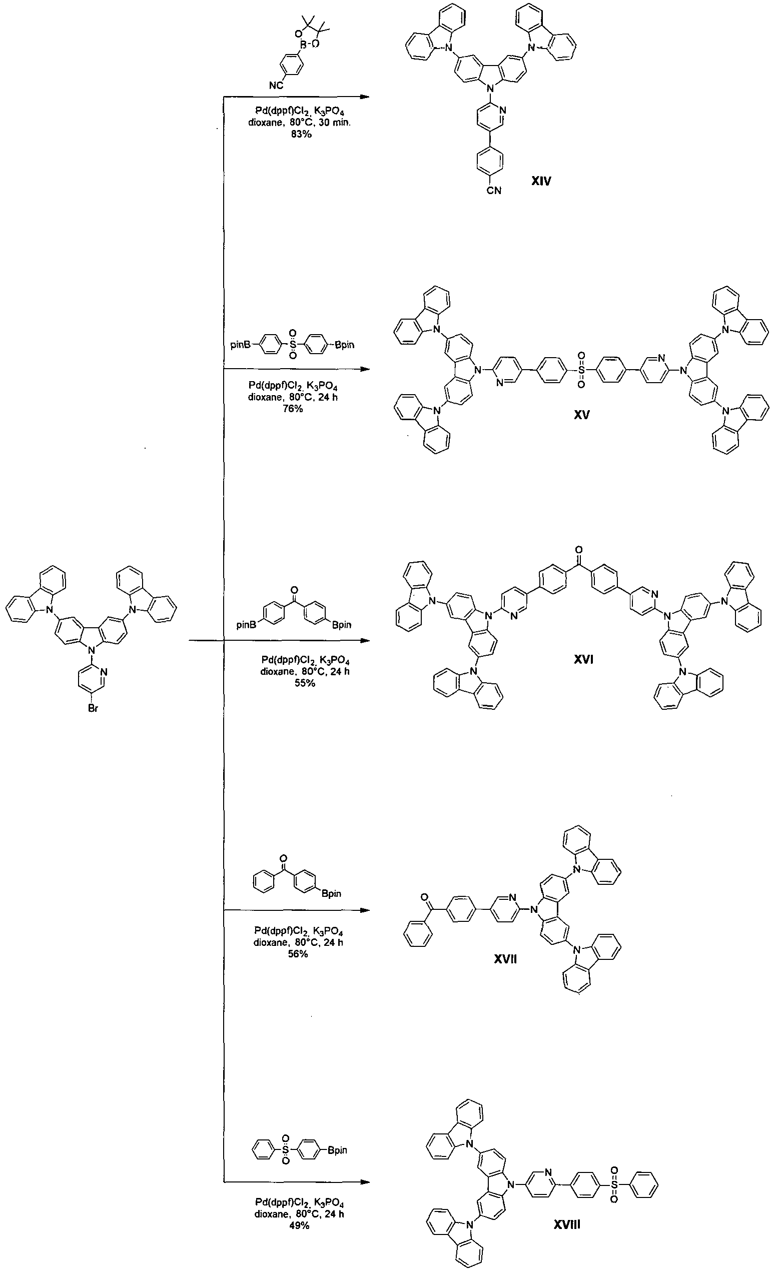

### 3,6-Diiodo-9-tosyl-9H-carbazole:

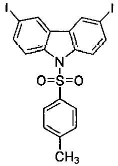

3,6-Diiodo-9*H*-carbazole (17.758 g; 0.04 mol) and KOH (4.03 g; 0.072 mmol) were dissolved in acetone (240 mL). A solution of tosyl chloride (13.37g; 0.07 mol) in acetone (24 mL) was added and the mixture was stirred for 24 h at reflux. Cooled to RT, the resulting precipitate was filtered off, washed with acetone (4×50 mL), water (50 mL), again with acetone (2×50 mL) and left to dry to yield 17.819 g (78%) of the product. mp=240-242°C.

¹H NMR (CDCl3, 700 MHz): δ ppm: 8.17 (s, 2H, 2CH_{Ar}), 8.07 (s, *J*=8.8 Hz, 2H, 2CH_{Ar}), 7.78 (dd, *J*=8.8 Hz, 1.7 Hz, 2H, 2CH_{Ar}), 7.64 (d, *J*=8.3 Hz, 2H, 2CH_{Ar}), 7.13 (d, *J*=8.1 Hz, 2H, 2CH_{Ar}), 2.29 (s, 3H, CH₃).

IR vₘₐₓ cm⁻¹: 3079.36(w), 2913.17(w), 2280.47(w), 2082.37(w), 1871.99(w), 1737.46(m), 1591.19(m), 1567.02(w), 1495.01(w), 1459.84(m), 1420.61(s), 1358.64(s), 1304.54(w), 1275.66(w), 1207.18(w), 1165.16(m), 1127.52(m), 1086.41(m), 1016.36(s), 962.51(s), 866.00(w), 806.90(s), 707.09(s), 669.01(s), 576.42(m), 534.14(m), 472.04(w), 444.60(w). 9'- Tosylo-9'*H*-9,3':6',9"-terkarbazol:

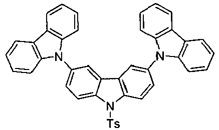

In a 8 mL vial capped septum [Pd(allyl)Cl]₂ (76.86 mg; 0.21 mmol; 0.5 mol%) and *t-*BuXPhos (357 mg; 0.84 mmol; 2 mol%) were mixed in dry 1,4-dioxane (5 mL) and stirred for 5 min. in an inert gas atmosphere. In a separate round-bottomed flask, carbazole (7.014 g; 42 mmol) was dissolved in dry 1,4-dioxane (130 mL), *tert*-BuOLi (1M; 42 mL; 42 mmol) was added and the mixture was stirred for 5 min. in an inert gas atmosphere. 3,6-Diodo-9-tosyl-9*H*-carbazole (11,460 g; 20 mmol) and the catalyst prepared in the vial were added and it was stirred at 100°C for 24 h. Cooled to RT and poured into methanol (250 mL). Precipitate was filtered off, washed with methanol (25 mL), water (25 mL) and air dried to obtain 8.925 g (68%) of the product as a gray solid. mp=306-311°C.

¹H NMR (CDCl3, 700 MHz), δ ppm: 8.60-8.62 (d, *J*=8.9 Hz 2H, 2CH_{Ar}), 8.14-8.17 (d, *J*=7.6 Hz 4H, 4CH_{Ar}), 8.09-8.10 (d, *J*=2.1 Hz 2H, 2CH_{Ar}), 7.93-7.95 (d, *J*=8.4 Hz 2H, 2CH_{Ar}), 7.71-7.73 (dd, *J*=8.9 Hz, 2.1 Hz, 2H, 2CH_{Ar}), 7.35-7.38 (m, 8H, 8CH_{Ar}), 2.40 (s, 3H, CH₃).

IR vₘₐₓ cm⁻¹: 3055(w), 1595(m), 1493(m), 1452(s), 1372(m), 1332(w), 1313(w), 1228(s), 1169(s), 1124(m), 1089(m), 1029(w), 924(m), 823(m), 748(s), 723(m), 677(w), 661(w), 640(w), 583(s), 534(s), 487(m), 423(m). 9'*H*-9,3':6',9"-Terkarbazol:

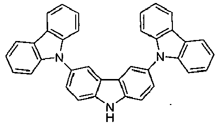

9'-Tosyl-9'*H*-9.3':6',9"-tercarbazole (8.925 g; 13.5 mmol) in a round bottom flask was dissolved in THF (30 mL), DMSO (15 mL) and, water (5 mL) and stirred for 10 min. Potassium hydroxide (9.20 g, 164 mmol) was added and the mixture was heated at reflux for 18 h. The mixture was cooled to RT, water (15 mL) was added and it was neutralized with HCI (2M, 15 mL). The precipitate was filtered off and air dried, yielding 6.039 g (90%) of gray powder. mp=330-333°C.

¹H NMR (CDCl3, 700 MHz), δ ppm: 8.57 (s, 1H, NH), 8.19-8.21 (d, *J*=1.8 Hz, 2H, 2CH_{Ar}), 8.15-8.17 (d, *J*=7.8 Hz, 4H, 4CH_{Ar}), 7.69-7.72 (d, *J*=8.7 Hz, 2H, 2CH_{Ar}), 7.61-7.63 (dd, *J*=8.5 Hz, 2.1 Hz, 2H, 2CH_{Ar}), 7.36-7.41 (m, 8H, 8CH_{Ar}), 7.26-7.29 (m, 4H, 4CH_{Ar}).

IR vₘₐₓ cm⁻¹: 3047(m), 3051(m), 1624(s), 1593(s), 1496(s), 1468(w), 1452(s), 1335(w), 1315(m), 1285(m), 1232(s), 1156(m), 1121(w), 1023(s), 920(m), 883(w), 815(m), 748(s), 723(s), 634(s), 609(m), 572(m), 528(w), 492(w), 424(s). 9'-(5-Bromopyridin-2-yl)-9'*H*-9,3':6',9"-tercarbazole:

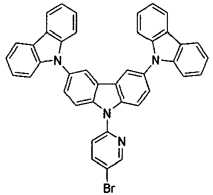

9'*H*-9,3':6',9"-Tercarbazole (9.96 g; 20 mmol), 5-bromo-2-fluoropyridine (6.16 g; 35 mmol) and cesium carbonate (13.04 g; 40 mmol) were mixed in dry DMF (50 mL) and inserted into oil bath of 120°C. After one hour, it was poured into water (50 mL), dichloromethane (100 mL) was added and the layers were separated. The aqueous layer was extracted with dichloromethane (80 mL), the combined chloroform extracts were washed with water (2×100 mL), brine and dried with anhydrous magnesium sulfate. The solvents were removed on a rotary evaporator, yielding 12.20 g of crude product crystallized twice from toluene (50 mL) to obtain 8.96 g (68%) of pure product. mp=180.6°C.

¹H NMR (CDCl₃, 400 MHz), δ ppm: 8.90 (dd, *J*=2.6 Hz, 0.8 Hz,1H,CH_{Ar}), 8.19-8.21 (dd, *J*=2.0 Hz,0.8 Hz,2H, 2CH_{Ar}), 8.17-8.20 (m, 5H, 5CH_{Ar}), 8.10 (dd, *J*=8.7 Hz, *J*=0.4 Hz, 2H, 2CH_{Ar}), 8.10 (dd, *J*=8.5 Hz, *J*=0.7 Hz, 2H, 2CH_{Ar}), 7.77 (dd, *J*=8.5 Hz, 2.0 Hz,1H, CH_{Ar}), 7.70 (dd, *J*=8.7 Hz, 2.0 Hz,1H, CH_{Ar}), 7.40-7.44 (m, 8H, 8CH_{Ar}), 7.27-7.34 (m, 4H, 4CH_{Ar}).

IR vₘₐₓcm⁻¹: 3059(m), 3023.3(m), 1883.6(w), 1595.9(m), 1574(m), 1491.28(s), 1454.05(s), 1381.18(s), 1336.06(s), 1315.33(s), 1288.20(s), 1229.50(s), 1163(s), 1091.8(s), 1006.8(s), 924.66(m), 820.44(s), 739.02(s), 721.32(s), 639.92(s), 563.01(s), 500(s), 425.07(s)

### 4-(2-(9'H-[9,3':6',9"-Tercarbazol]-9'-yl)pyridin-5-yl)benzonitrile:

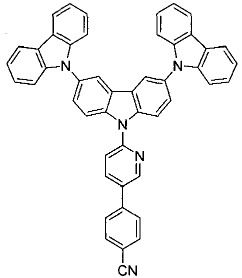

4-(4,4,5,5-Tetramethyl-1,3,2-dioxaborolan-2-yl)benzonitrile (0.194 g; 0.65 mmol), 9'-(5-bromopyridin-2-yl)-9'*H*-9,3':6',9"-tercarbazole (0.330 g; 0.5 mmol) and Pd(dppf)Cl₂·DCM (20.4 mg; 0.025 mmol, 5% mol) were mixed in degassed 1,4-dioxane (5 mL) in an inert atmosphere at RT. K₃PO₄ (3M aqueous 0.5 mL) was added and the mixture was inserted into 80°C oil bath and stirred for 30 min. Pour into water (40 mL), filter, washed with methanol and dried. The crude product was purified by flash chromatography(AcOEt:PE - 1:4) to give 0.280 g (83%) of pure product.

¹H NMR (CDCl3, 400 MHz): δ ppm: 9.07 (dd, *J*=2.5 Hz, *J*=0.7 Hz 1H, CH_{Ar}), 8.30 (d, *J*=1.6 Hz, 2H, 2CH_{Ar}), 8.27 (dd, *J*=8.4 Hz, *J*=2.5 Hz, 1H, CH_{Ar}), 8.20 (m, 6H, 6CH_{Ar}), 7.98 (d, *J*=8.4 Hz, 1H, CH_{Ar}), 7.85-7.90 (m, 4H, 4CH_{Ar}), 7.72 (dd, *J*=8.8 Hz, *J*=2.1 Hz, 2H, 2CH_{Ar}), 7.45-7.42 (m, 8H, 8CH_{Ar}), 7.34-7.26 (m, 4H, 4CH_{Ar}).

### 2,2'-(Sulfonylbis(4,1-phenylene))bis(4,4,5,5-tetramethyl-1,3,2-dioxaborolane):

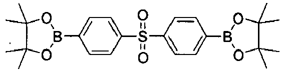

In a Schlenk tube, bis(pinacolato)diboron (3.050 g, 12 mmol), 4,4'-sulfonylbis(chlorobenzene) (1.39 g, 5 mmol) and potassium acetate (1.96 g, 20 mmol) were mixed under argon in degassed dioxane (15 mL). Separately, in septum capped vial, catalyst was prepared by mixing under argon [Pd(allyl)Cl]₂ (37 mg, 0.1 mmol) and XPhos (191 mg, 0.4 mmol) in degassed dioxane (5 mL) at RT for 5 min. It was added to the reaction in Schlenk tube, warmed to 80°C and stirred overnight. Poured onto a 10% aqueous citric acid, extracted with ethyl acetate (2×60 mL), the combined organic extracts were washed with water (2×80 mL), dried with anhydrous magnesium sulfate and evaporated to dryness. The crude product was purified by crystallization from PE/DCM to give 2.12 g (38%) of pure product as a gray solid. mp=274.9° C.

¹H NMR (CDCl3, 700 MHz), δ ppm: 7.90 (s, 8H, 8CH_{Ar}), 1.32 (s, 24H, 8CH₃).

IR vₘₐₓ cm⁻¹ : 3672.6(w), 2985(m), 1598.6(m), 1494.5(m), 1391.51(s), 1355.65(s), 1270.03(s), 1215(m), 1141.82(s), 1079.6(s), 1014.70(s), 963.01(m), 855.42(s), 741.76(s), 718.73(s), 646.92(s),599.01(s), 570.04(s), 431.5(m)

### 9',9""-((Sulfonylbis(4,1-phenylene))bis(pyridine-5,2-diyl))bis((9'H-9,3':6',9"-tercarbazole)):

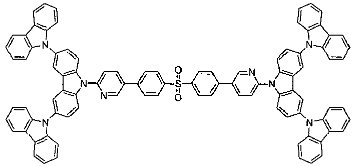

In a Schlenk tube 9'-(2-bromopyridin-5-yl)-9'*H*-9,3':6',9"-tercarbazole (588 mg, 0.9 mmol), 2,2'-(sulfonylbis(4,1-phenylene))bis(4,4,5,5-tetramethyl-1,3,2-dioxaborolane) (141 mg, 0.3 mmol) and Pd(dppf)Cl₂ (50 mg, 0.06 mmol) were mixed in anhydrous degassed dioxane (10 mL) under argon. The 3M aqueous K₃PO₄ solution (1 mL, 3 mmol) was added, inserted into an oil bath at 80°C and stirred overnight. Poured onto 10% aqueous citric acid (50 mL), extracted with ethyl acetate (2×35 mL), the combined organic extracts were washed with water (2×50 mL), dried with anhydrous magnesium sulfate and evaporated to dryness on a rotary evaporator. The residue was purified by flash chromatography (THF/PE 1:4 to 1:1), and crystallized from THF/PE, yielding 313 mg (76%) of pure product as a light yellow powder. mp=321.1°C.

¹H NMR (CDCl₃, 700 MHz), δ ppm: 9.05 (d, J=2.1 Hz, 2H, 2CH_{Ar}); 8.28 (d, J=2.1 Hz, 4H, 4CH_{Ar}); 8.25 (dd, J=2.8 Hz, J=8.4 Hz, 2H, 2CH_{Ar}); 8.24-8.22 (m, 4H, 4CH_{Ar}); 8.19 (d, J=8.4 Hz, 4H, 4CH_{Ar}); 8.18-8.15 (m, 8H, 8CH_{Ar}); 7.96 (d, J=8.4 Hz, 4H, 4CH_{Ar}); 7.92-7.90 (m, 4H, 4CH_{Ar}); 7.69 (dd, J=8.4 Hz, J=2.1 Hz, 4H, 4CH_{Ar}); 7.41-7.40 (m, 16H, 16CHAr); 7.30-7.27 (m, 8H, 8CH_{Ar}).

IR vₘₐₓ cm⁻¹: 3050.58(m), 2960.59(m), 2652.66(w), 2282.97(w), 1885.19(m), 1708.44(m), 1594.08(s), 1462.19(s), 1315.43(s), 1228.63(s), 1107.66(m), 745.86(s), 607.31(s), 400.74(s).

### Bis(4-(4,4,5,5-tetramethyl-1,3,2-dioxaborolan-2-yl)phenyl)methanone:

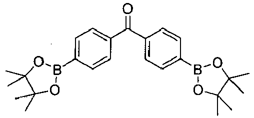

Bis(4-chlorophenyl)methanone (1.255 g, 5 mmol), bis(pinacolato)diboron (3.05 g, 12 mmol) and potassium acetate (1.96 g, 20 mmol) were mixed under argon in dry degassed dioxane (15 mL). Prepared separately catalyst from Pd(allyl)Cl₂ (36.6 mg, 0.1 mmol, 2 mol%) and XPhos (191 mg, 0.4 mmol, 8% mol) in dry degassed dioxane (3 mL) was added and stirred at 100°C for 10 minutes. Cooled to RT, 10% citric acid (50 mL) was added and extracted with ethyl acetate (2×50 mL), washed with water (2×50 mL), brine, dried with anhydrous magnesium sulfate, and the solvents were evaporated on a rotary evaporator. Purified by flash chromatography (10% AcOEt) to give 1.38 g (64%) of a white crystalline solid. 190.0-191.5°C.

¹H NMR (CDCl₃, 400 MHz), δ ppm: 7.94-7.90 (m, 4H, 4CH_{Ar}); 7.80-7.77 (m, 4H, 4CH_{Ar}); 1.39 (s, 24H, 8CH₃).

### Bis(4-(2-(9'H-[9,3':6',9"-tercarbazol]-9'-yl)pyridin-5-yl)phenyl)methanone:

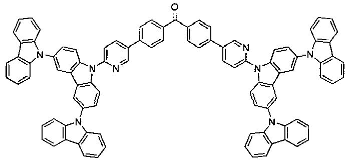

In a Schlenk tube 9'-(2-bromopyridin-5-yl)-9'H-9,3':6',9"-tercarbazole (589 mg, 0.9 mmol), bis(4-(4,4,5,5-tetramethyl-1,3,2-dioxaborolan-2-yl)phenyl)methanone (130 mg, 0.3 mmol) and Pd(dppf)Cl₂ (50 mg, 0.06 mmol) were mixed under argon in anhydrous, degassed dioxane (10 mL), 3M aqueous K₃PO₄ solution (1 mL, 3 mmol) was added and the Schlenk was inserted into a 80°C oil bath at and stirred overnight. Cooled, poured onto 10% aqueous citric acid (50 mL), extracted with ethyl acetate (2×40 mL), the combined organic extracts were washed with water (2×50 mL), dried over anhydrous magnesium sulfate and evaporated to dryness on a rotary evaporator. The crude product was purified by flash chromatography (THF/PE 1:4 to 1:1), and crystallization from THF/PE to give 221 mg (55%) of pure product as a pale yellow powder. mp=284.1°C.

¹H NMR (CDCl₃, 700 MHz), δ ppm: 9.15 (d, J=2.1 Hz, 2H, 2CH_{Ar}); 8.35 (dd, J=8.4 Hz, J=2.8 Hz, 2H, 2CH_{Ar}); 8.30 (d, J=2.1 Hz, 4H, 4CH_{Ar}); 8.21 (d, J=8.4 Hz, 4H, 4CH_{Ar}); 8.19-8.16 (m, 8H, 8CH_{Ar}); 8.12-8.09 (m, 4H, 4CH_{Ar}); 7.99 (d, J=8.4 Hz, 2H, 2CH_{Ar}); 7.94-7.91 (m, 4H, 4CH_{Ar}); 7.71 (dd, J=8.4 Hz, J=2.1 Hz, 4H, 4CH_{Ar}); 7.43-7.40 (m, 16H, 16CH_{Ar}); 7.31-7.28 (m, 8H, 8CHAr)

IR vₘₐₓ cm⁻¹: 3047.28(s), 1911.39(m), 1656.52(s), 1595.96(s), 1549.71(s), 1458.28(s), 1378.49(s), 1332.53(s), 1313.72(s), 1275.40(s), 1226.56(s), 1157.73 (m), 1023.77(m), 1000.34(m), 926.25(s), 816.50(s), 745.77(s), 721.90 (s), 639.41(m), 568.71(m), 494.49(m), 421.22(s)

### Phenyl(4-(4,4,5,5-tetramethyl-1,3,2-dioxaborolan-2-yl)phenyl)methanone

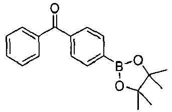

(4-Chlorophenyl)(phenyl)methanone (1.08 g, 5 mmol), bis(pinacolato)diboron (1.52 g, 6 mmol) and potassium acetate (1.18 g, 12 mmol) were mixed under argon in dry degassed dioxane (15 mL) and separately prepared catalyst from Pd(allyl)Cl₂ (36.6 mg, 0.1 mmol, 2% mol) and XPhos (191 mg, 0.4 mmol, 8% mol) in dry degassed dioxane (3 mL) was added and stirred at RT for 30 minutes. Cooled to RT, 10% citric acid (50 mL) was added and extracted with ethyl acetate (2×50 mL). Combined organic extract were washed with water (2×50 mL), brine, dried with anhydrous magnesium sulfate, and evaporated to dryness on a rotary evaporator. Purified by flash chromatography (10% AcOEt) to obtain 1.26 g (82%) of a white crystalline solid. mp=153.8°C.

¹H NMR (CDCl₃, 400 MHz), δ ppm: 7.96-7.92 (m, 2H, 2CH_{Ar}); 7.84-7.77 (m, 4H, 4CH_{Ar}); 7.63-7.58 (m, 1H, CH_{Ar}); 7.52-7.47 (m, 2H, 2CH_{Ar}); 1.39 (s, 12H, 4CH3).

IR vₘₐₓ cm⁻¹ : 3374(w), 3297.3(w), 3069.9(w), 2974(m), 2927.4(m), 2363(w), 1654.17(s), 1505.5(m), 1448(m), 1394.26(s), 1357.85(s), 1323.02(s), 1270.85(s), 1164.44(s), 1137.48(s), 1082.58(s), 960.27(m), 925.30(s), 850.61(s), 754.8(m), 694.79(s), 654.26(s), 431.5(m)

### (4-(2-(9'H-[9,3':6',9"-tercarbazol]-9'-yl)pyridin-5-yl)phenyl)(phenyl)methanone:

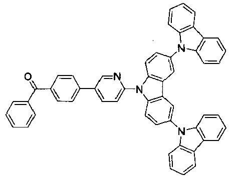

In a Schlenk tube 9'-(2-bromopyridin-5-yl)-9'H-9,3':6',9"-tercarbazole (294 mg, 0.45 mmol), phenyl(4-(4,4,5,5-tetramethyl-1,3,2-dioxaborolan-2-yl)phenyl)methanone (92 mg, 0.3 mmol) and Pd(dppf)Cl₂ (25 mg, 0.03 mmol) were mixed under argon in dry degassed dioxane (10 mL). 3M aqueous K₃PO₄ solution (0.5 mL, 1.5 mmol) was added, inserted into a 80°C oil bath and stirred overnight. Poured onto 10% aqueous citric acid solution (50 mL), extracted with ethyl acetate (2×50 mL), the combined extracts were washed with water (2×50 mL), dried with anhydrous magnesium sulfate and evaporated to dryness on a rotary evaporator. Crude product was purified by flash chromatography (THF/PE 1:4 to 1:1) and crystallization from THF/PE to give 126 mg (56%) of the product as a light yellow powder. mp=315.4° C.

¹H NMR (CDCl₃, 700 MHz), δ ppm: 9.12 (d, J=2.1 Hz, 1H, CH_{Ar}); 8.32 (dd, J=8.4 Hz, J=2.8 Hz, 1H, CH_{Ar}); 8.29 (d, J=2.1 Hz, 2H, 2CH_{Ar}); 8.20 (d, J=8.4 Hz, 2H, 2CH_{Ar}); 8.18-8.16 (m, 4H, 4CH_{Ar}); 8.04-8.02 (m, 2H, 2CH_{Ar}); 7.97 (d, J=8.4 Hz, 1H, CH_{Ar}); 7.90-7.86 (m, 4H, 4CH_{Ar}); 7.70 (dd, J=8.4 Hz, J=2.1 Hz, 2H, 2CH_{Ar}); 7.66-7.64 (m, 1H, CH_{Ar}); 7.56-7.53 (m, 2H, 2CH_{Ar}); 7.42-7.41 (m, 8H, 8CH_{Ar}); 7.31-7.28 (m, 4H, 4CH_{Ar}).

IR vₘₐₓ cm⁻¹: 3051.14(m), 2959.97(m), 1916.59(w), 1864.67(w), 1772.06(w), 1648.68(s), 1593.98(s), 1549.24(s), 1463.30(s), 1416.57(s), 1379.86(s), 1315.19(s), 1277.55(s), 1231.23(s), 1171.67(s), 1119.58(m), 1091.99(m), 1023.91(m), 925.37(s), 884.09(m), 818.46(s), 746.76(s), 724.36(s), 699.85(s), 642.95(s), 574.40 (s), 525.36(m), 497.20(s), 422.21(s)

### 9'-(2-(4-(Phenylsulfonyl)phenyl)pyridin-5-yl)-9'H-9,3':6',9"-tercarbazole:

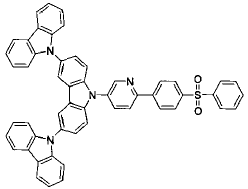

In a Schlenk tube 9'-(4-bromophenyl)-9'H-9,3':6',9"-tercarbazole (294 mg, 0.45 mmol), 4,4,5,5-tetramethyl-2-(4-(phenylsulfonyl)phenyl)-1,3,2-dioxaborolane (103 mg, 0.3 mmol) and Pd(dppf)Cl₂ (25 mg, 0.03 mmol) were mixed under argon in dry degassed dioxane (10 mL). 3M aqueous K₃PO₄ solution (0.5 mL, 1.5 mmol) was added, inserted into a 80°C oil bath and stirred overnight. Poured onto 10% aqueous citric acid solution (50 mL), extracted with ethyl acetate (2×50 mL), the combined extracts were washed with water (2×50 mL), dried with anhydrous magnesium sulfate and evaporated to dryness on a rotary evaporator. Crude product was purified by flash chromatography (THF/PE 1:4 to 1:1) and crystallization from THF/PE to give 116 mg (49%) of the product as a light yellow powder. mp 190.0-191.5°C.

¹H NMR (CDCl₃, 700 MHz), δ ppm: 9.02 (d, J=2.1 Hz, 1H, CH_{Ar}); 8.27 (d, J=2.1 Hz, 2H, 2CH_{Ar}); 8.23 (dd, J=8.4 Hz, J=2.8 Hz, 1H, CH_{Ar}); 8.19-8.13 (m, 8H, 8CH_{Ar}); 8.05-8.02 (m, 2H, 2CH_{Ar}); 7.94 (d, J=8.4 Hz, 1H, CH_{Ar}); 7.87-7.84 (m, 2H, 2CH_{Ar}); 7.69 (dd, J=8.4 Hz, J=2.1 Hz, 2H, 2CH_{Ar}); 7.64-7.61 (m, 1H, CH_{Ar}); 7.58-7.55 (m, 2H, 2CHAr);7.41-7.40 (m, 8H, 8CH_{Ar}); 7.30-7.27 (m, 4H, 4CH_{Ar}).

¹³C NMR (CDCl₃, 75 MHz), δ ppm: 109.5 (4CH_{Ar}); 112.7 (2CH_{Ar}); 118.8 (CH_{Ar}); 119.5 (2CH_{Ar}); 119.7 (4CH_{Ar}); 120.2 (4CH_{Ar}); 123.2 (4C_{Ar}); 125.2 (2C_{Ar}); 125.8 (4CH_{Ar}); 126.4 (2CH_{Ar}); 127.7 (2CH_{Ar}); 127.8 (2CH_{Ar}); 128.6 (2CH_{Ar}); 129.3 (2CH_{Ar}); 131.6 (2C_{Ar}); 132.9 (C_{Ar}); 133.3 (CH_{Ar}); 137.4 (CH_{Ar}); 139.0 (2C_{Ar}); 141.4 (C_{Ar}); 141.5 (C_{Ar}); 141.6 (4C_{Ar}); 141.7 (C_{Ar}); 148.1 (CH_{Ar}); 151.3 (C_{Ar}).

IR vₘₐₓ cm⁻¹: 3051.88(m), 2924.92(m), 2857.19(m), 2108.01(w), 1983.10(w), 1764.14(w), 1699.46(w), 1625.07(m), 1592.98(s), 1465.38(s), 1375.17(s), 1314.03(s), 1230.17(s), 1154.47(s), 1105.35(s), 1063.79(s), 919.20(m), 820.52(s), 784.92(m), 745.43(s), 687.88(s), 641.40 (s), 598.25(s), 568.02(s), 492.51(s), 420.39(s).

### EMBODIMENT 8 - synthesis of 9,9'-((Sulfonylbis(4,1-phenylene))bis(pyridine-5,2-diyl))bis(N³,N³,N⁶,N⁶-tetraphenyl-9H-carbazole-3,6-diamine

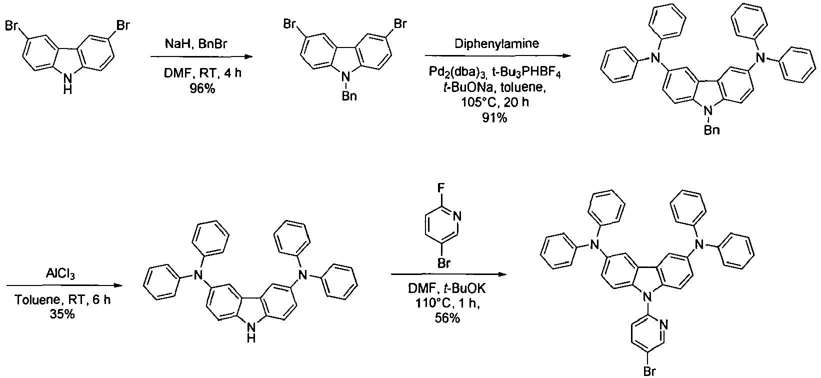

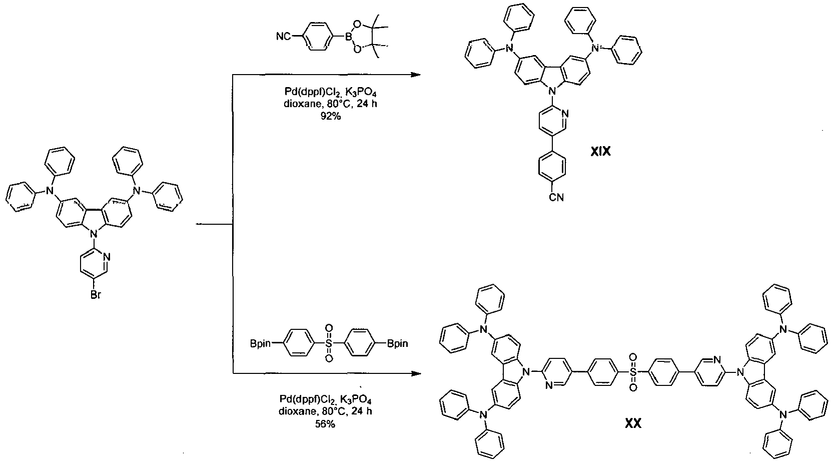

### 9-Benzyl-3,6-dibromo-9H-carbazole:

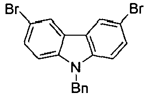

In a 250 ml two-necked flask, equipped with a reflux condenser and dropping funnel, sodium hydride (60% suspension in mineral oil, 1.54 g; 0.04 mol; 1.3 eq.) in dry DMF (50 mL) was placed and 3,6-dibromo-9H-carbazole (10 g; 0.031 mol; 1 eq.) in DMF (60 mL) was added dropwise over 20 minutes. After stirring at RT for 10 minutes, benzyl bromide (5.29 g; 0.031 mol; 1 eq.) was added dropwise and stirring continued for 4 hours. Carefully poured into water (250 mL), stirred for 30 minutes to cool. The product was extracted with ethyl acetate (3×250 mL), the combined organic layers were washed with water (250 mL) and brine (250 mL). The organic extracts were dried over anhydrous magnesium sulfate and the solvent was removed by rotary evaporation. The residue was dispersed in petroleum ether (250 mL), stirred 30 minutes, the product was filtered off with a sintered glass funnel and dried in vacuum exsiccator under P₄O₁₀ to give 12.29 g (96%) of a pale cream product, which was taken to the next step without purification.

¹H NMR (DMSO-de, 700 MHz), δ ppm: 8.50 (d, J=1.9 Hz, 2H, 2CH_{Ar}), 7.63 (s, 1H, CH_{Ar}), 7.62 (s, 1H, CH_{Ar}), 7.59 (d, J=2.0 Hz, 1H, CHAr) 7.58 (d, J=2.0 Hz, 1H, CH_{Ar}), 7.26-7.24 (m, 2H, 2CH_{Ar}), 7.22-7.21 (m, 1H, CH_{Ar}),7.12 (d, J=7.0 Hz, 2H, 2CH_{Ar}), 5.66 (s, 2H, 2CH₂).

### 9-Benzyl-N³,N³,N⁶,N⁶-tetraphenyl-9H-carbazole-3,6-diamine

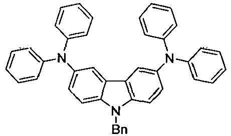

In an oven dried 500 mL single-necked flask, equipped with a reflux condenser, 9-benzyl-3-6-dibromo-9H-carbazole (7.3 g; 18 mmol; 1 eq.) from the previous stage, sodium tert-butoxide (5,096 g; 53 mmol; 2.95 eq.), and diphenylamine (6.76 g; 0.04 mol; 2.2 eq.) were mixed in dry toluene (250 mL) and stirred for 10 minutes at RT. Solution of Pd₂(dba)₃ (0.166 g; 0.18 mmol; 0.01 eq.) and t-Bu₃PHBF₄ (0.104 g; 0.36 mmol; 0.02 eq.) in dry toluene (10 mL) was added, the flask was heated to 105°C and stirred at this temperature overnight. After cooling, the precipitate was filtered off and washed with toluene (50 mL). Combined organic filtrates were washed with water (3×200 mL), brine (200 mL), and dried over anhydrous magnesium sulfate. Solvent was removed by rotary evaporation, methanol (200 mL) was added and stirred for one hour to give a fine precipitate. The product was filtered on a sintered glass funnel to obtain 9.5 g (91%) of light green powder. The product without purification was taken to the next step. mp=98-100°C.

¹H NMR (DMSO-ds, 700 MHz), δ ppm: 7.89 (d, J=2.0Hz, 2H, 2CH_{Ar}), 7.62 (d, J=8.4 Hz, 2H, 2CH_{Ar}), 7.29-6.87 (m, 23H, 23CH_{Ar}), 5.61 (s, 2H, CH2).

IR vₘₐₓ cm⁻¹: 3418.91(m), 3030.28(m), 2972.82(m), 2923.31(m), 2285.32(s), 2048.22(s), 1865.62(s), 1808.42(s), 1585.65(w), 1483.40(w), 1311.23(w), 1274.58(w), 1207.58(w), 1207.58(w), 1151.40(w), 1074.27(m), 1027.97(m), 880.92(m), 804.61(m), 748.70(w), 693.93(w), 632.97(m), 565.85(m), 510.84(m), 425.12(m)

### N³,N³,N⁶,N⁶-Tetraphenyl-9H-carbazole-3,6-diamine

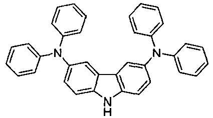

In an oven dried 250 mL two-necked flask, equipped with a magnetic stirrer, reflux condenser, and dropping funnel, aluminum chloride (2.13 g; 16 mmol; 4 eq.) was dispersed in toluene (40 mL) at RT. A solution of 9-benzyl-N³,N³,N⁶,N⁶-tetraphenyl-9H-carbazole-3,6-diamine (2.36 g; 4 mmol; 1 eq.) in toluene (40 mL) was added dropwise at RT. After stirring at RT for 6 hours, ethyl acetate (80 mL) was added followed by a saturated sodium bicarbonate solution (80 mL). Product was extracted with ethyl acetate (3x100 mL), combined organic layers were washed with saturated sodium bicarbonate (2×150 mL), water (2×150 mL), brine (150 mL), and dried over anhydrous magnesium sulfate. Solvent was removed by rotary evaporation to give 1.60 g of crude product. The product was purified on a flash chromatography, (AcOEtPE - 1: 9) to obtain 0.70 g (35%) of pure product. mp=246-248° C.

¹H NMR (DMSO-d₆, 700 MHz), δ ppm: 11.36 (s, 1H, NH), 7.85 (s, 2H, 2CH_{Ar}), 7.50 (d, J=8.3 Hz, 2H, 2CH_{Ar}), 7.20-7.15 (m, 8H, 8CHAr) 6.94-6.87 (m, 10H, 10CH_{Ar}).

IR vₘₐₓ cm⁻¹: 3401.42(m), 2960.90(w), 2924.70(m), 2345.04(w), 2227.21(w), 1983.37(w), 1930.78(w), 1865.36(w), 1831.74(w), 1708.51(m), 1642.75(w), 1584.91(s), 1475.56(s), 1394.82(w), 1270.14(s), 1230.35(s), 1175.15(w), 1149.92(w), 1116.85(w), 1075.10(m), 1039.98(m), 929.26(w), 881.59(w), 847.41(w), 806.99(m), 748.65(s), 692.09(s), 634.87(m), 595.46(w), 576.00(w), 509.26(m), 461.81(m), 429.61(w).

### 9-(5-Bromopyridin-2-yl)-N³,N³,N⁶,N⁶-tetraphenyl-9H-carbazole-3,6-diamine:

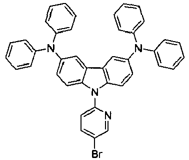

In a 100 mL single-necked flask N³,N³,N⁶,N⁶-tetraphenyl-9H-carbazole-3,6-diamine (3.9 g; 7.8 mmol; 1 eq.) and potassium tert-butoxide (1.74 g; 15.6 mmol; 2 eq.) were mixed in dry DMF (50 mL) and 5-bromo-2-fluoropyridine (2.74 g; 15.6 mmol; 2 eq.) was added. Flask was heated to 110°C and stirred at this temperature for 1 hour. After cooling, the contents of the flask were transferred to a separatory funnel, water (100 mL) was added and the product was extracted with ethyl acetate (3×50 mL). The combined organic extracts were washed with water (3×100 mL), brine (100 mL), dried with anhydrous magnesium sulfate. Solvent was removed, methanol (100 mL) was added to the residue and it was stirred for 30 minutes at RT. The precipitate was filtered off and purified by flash chromatography (chloroform:PE 2:8) to obtain 2.85 g (56%) of pure product as a light yellow powder. mp=140-160°C.

¹H NMR (CDCl₃, 700 MHz), δ ppm: 8.74 (d, J=2.3 Hz, 1H, CH_{Ar}), 8.02 (dd, J₁=8.5 Hz, J₂=2.5 Hz, 1H, CH_{Ar}), 7.74 (d, J=8.6Hz, 2H, 2CH_{Ar}), 7.72(d, J=2.1 Hz,2H, 2CH_{Ar}), 7.56 (d, J=8.5 Hz, 1H, CH_{Ar}), 7.24 (dd, J=8.8 Hz, 2.1 Hz, 2H, 2CH_{Ar}), 7.22-7.19 (m, 8H, 8CH_{Ar}), 7.08-7.06 (m, 8H, 8CH_{Ar}), 6.96-6.93 (m, 4H, 4CH_{Ar}).

IR vₘₐₓ cm⁻¹: 3405.15(w), 2922.80(m), 227.64(w), 1931.81(w), 1833.59(w), 1723.26(w), 1583.25(s), 1483.35(s), 1455.68(s), 1378.09(m), 1312.27(m), 1272.45(m), 1225.21(m), 1176.77(w), 1152.91(m), 1119.30(w), 1082.52(m), 1029.00(m), 923.28(w), 874.97(w), 832.69(w), 807.82(m), 748.19(s), 692.93(s), 633.03(m), 574.09(m), 512. 57(s), 479.01(w), 419.14(m).

### 4-(4,4,5,5-Tetramethyl-1,3,2-dioxaborolan-2-yl)benzonitrile:

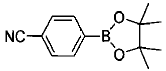

4-Bromobenzonitrile (1.82 g; 0.01 mol), bis(pinacolato)diboron (3.81 g; 0.015 mol), potassium acetate (1.96 g; 0.02 mol), Pd(dppf)Cl₂ (0.22 g; 0.3 mmol) dissolved under argon in dry dioxane (15 mL) and stirred at 100°C for 3 h. Cooled to RT, water (150 mL) was added and extracted with ethyl acetate (3×50 mL). The combined organic layers were washed with water (3×100 mL), brine (150 mL) and dried with anhydrous magnesium sulfate. The solvent was rotary evaporated to yield 3.147 g of crude product which was purified by flash chromatography (AcOEt/PE 5:95) to give 1.98 g (86%) of pure product. mp=93-94°C.

¹H NMR (CDCl₃,400 MHz): δ ppm: 7.90 (d, J=8.3 Hz, 2H, 2CH_{Ar}), 7.66 (d, J=8.3 Hz, 2H, 2CH_{Ar}), 1.37 (s, 12H, 4CH₃).

IR vₘₐₓ cm⁻¹: 3829.40(w), 3735.46(w), 3425.00(m), 3053.28(w), 2976.79(s) 2934.26(w), 2347.10(w), 224.98(m), 2104.29(w), 1946.40(w), 1892.33(w), 1742.34(s), 1676.00(w), 1599.31(w), 1564.69(m), 1453.72(s), 1356.00(s), 1272.53(m), 1215.27(m), 1171.03(w), 1133.58(m), 1086.58(m), 1016.56(m), 958.62(m), 834.79(s), 738.85(s), 651.10(s), 572.00(w), 551.04(m), 452.18(w), 423.48(w).

### 4-(2-(3,6-bis(diphenylamino)-9H-carbazol-9-yl)pyridine-5-yl)benzonitrile

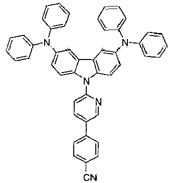

9-(5-Bromopyridin-2-yl)-N³,N³,N⁶,N⁶-tetraphenyl-9H-carbazole-3,6-diamine (0.60 g; 0.9 mmol; 1 eq.), 4-(4,4,5,5-tetramethyl-1,3,2-dioxaborolan-2-yl)benzonitrile (0.23; 1.0 mmol; 1.1 eq.), Pd(dppf)Cl₂·DCM (0.037 g; 0.046 mmol; 0.05 eq), and dioxane (30 mL) were stirred at RT for 5 minutes. 3M aqueous K₃PO₄ solution (0.62 mL; 1.8 mmol; 2 eq.) was added, temperature was increased to 80°C and stirred at this temperature overnight. After cooling, the mixture was transferred to a separatory funnel, water (100 mL) was added, the product was extracted with ethyl acetate (3×50 mL), combined organic extracts were washed with water (3×100 mL), brine (100 mL), dried over anhydrous magnesium sulfate and rotary evaporated. Crude product was purified on a flash column chromatography (AcOEt/PE 1:9→1:1) to give 0.57 g (92%) of pure product as a light yellow powder. mp=130-132 °C.

¹H NMR (CDCl₃, 700 MHz), δ ppm: 8.93 (d, J=2.6Hz, 1H, CH_{Ar}), 8.12 (dd, J₁=8.4Hz, J₂=2.4 Hz, 1H, CH_{Ar}), 7.86 (d, J=8.8 Hz, 2H, 2CH_{Ar}), 7.83-7.82 (m, 2H, 2CH_{Ar}), 7.79-7.77 (m, 3H, 3CH_{Ar}), 7.75 (d, J=2.1Hz, 2H, 2CH_{Ar}), 7.27 (dd, J₁=8.8Hz, J₂=2.1Hz, 2H, 2CH_{Ar}), 7.23-7.21 (m, 8H, 8CH_{Ar}), 7.10-7.08 (m, 8H, 8CH_{Ar}), 6.97-6.95 (m, 4H, 4CH_{Ar}). IR vₘₐₓ cm⁻¹:3671.11(w), 2922.50(m), 2618.92(w), 2299.40(w), 1919.93(w), 1705.15(m), 1661.59(w), 1590.79(s), 1481.84(s), 1456.00(s), 1378.48(m), 1270.55(s), 1175.22(w), 1152.84(w), 114.92(w), 1075.22(m), 1038.87(w), 932.55(w), 830.676(m), 807.31(m), 747.56(s), 691.77(s), 632.41(m), 563.77(m), 510.00(m), 411.78(w).

### 9,9'-((Sulfonylbis(4,1-phenylene))bis(pyridine-5,2-diyl))bis(N³,N³,N⁶,N⁶-tetraphenyl-9H-carbazole-3,6-diamine:

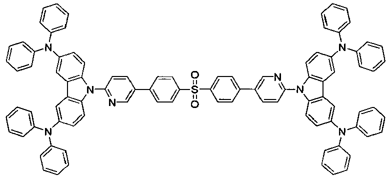

In a 50 mL flask 6-benzhydryl-9-(5-bromopyridin-2-yl)-N,N-diphenyl-9H-carbazol-3-amine (0.292 g; 0.445 mmol; 2.1 eq.), 2,2'-(sulfonylbis(4,1-phenylene))bis(4,4,5,5-tetramethyl-1,3,2-dioxaborolane) (0.10 g; 2.1 mmol; 1 eq.) and Pd(dppf)Cl₂·DCM (0.014 g; 0.0169 mmol; 0.08 eq.) were mixed under argon in degassed dioxane (30 mL) and stirred for 5 minutes at RT. 3M aqueous K₃PO₄ solution (0.3 mL; 0.84 mmol; 4 eq.) was added, the flasks was heated to 80°C and stirred at this temperature overnight. After cooling, the flask content was transferred to a separatory funnel, water (100 mL) was added, and the product was extracted with chloroform (3×50 mL). The combined organic layers were washed with water (3×100 mL), brine (100 mL), and dried over anhydrous magnesium sulfate. The solvent was removed by rotary evaporation, the residue was purified by flash chromatography (THF/PE 1:4→1:1) to give 0.221 g (76%) of product as a light yellow powder. mp=189-195° C.

¹H NMR (CDCl₃, 700 MHz), δ ppm: 9.01 (d, J=2.2 Hz, 2H, 2CH_{Ar}), 8.19 (dd, J₁=3.3Hz, J₂=2.5Hz, 2H, 2CH_{Ar}), 8.04 (d, J=8.3 Hz, 4H, 4CH_{Ar}), 7.88 (d, J=8.8 Hz,4H, 4CH_{Ar}), 7.84 (d, J=8.2 Hz, 4H, 4CH_{Ar}), 7.78 (d, J=8.2 Hz, 2H, 2CH_{Ar}), 7.76 (d, J=2.0 Hz, 4H, 4CHAr), 7.28 (dd, J₁=8.8 Hz, *J*₂=2.1 Hz, 4H, 4CH_{Ar}), 7.23-7.21 (m, 16H, 16CH_{Ar}), 7.11-7.09 (m, 16H, 16CH_{Ar}), 6.97-6.95 (m, 8H, 8CH_{Ar}).

IR vmax cm⁻¹: 3668.45(w), 3405.51(w), 2921.53(m), 2855.28(w), 2226.84(w), 1988.39(w), 1918.63(w), 1711.04(w), 1646.17(m), 1586.32(s), 1482.80(s), 1455.36(s), 1372.13(m), 1270.37(s), 1230.34(m), 1159.17(m), 1115.77(m), 1073.60(m), 1032.35(m), 926.50(m), 868.77(m), 834.83(m), 806.25(m), 748.72(s), 691.34(s), 633.61(m), 574.80(m), 511.61(m), 425.11(m).

### EMBODIMENT 9 - synthesis of XXI and XXII, which are shown below by the semi-structural formulae:

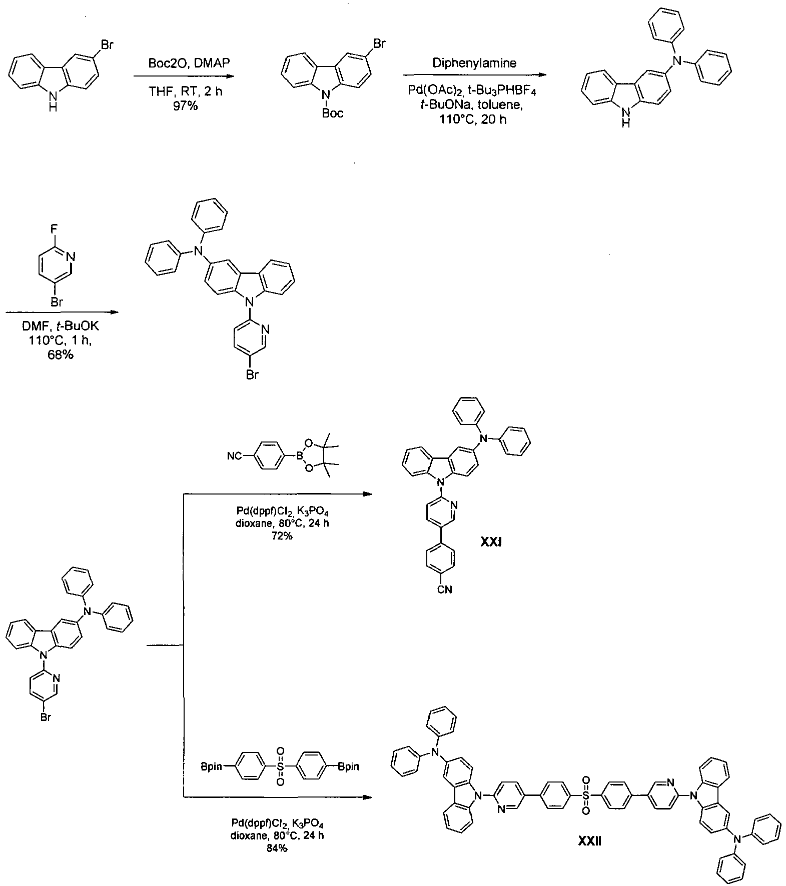

### tert-Butyl 3-bromo-9H-carbazole-9-carboxylate

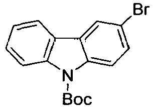

In a 100 mL flask 3-bromo-9H-carbazole (5 g; 20.2 mmol; 1 eq.) and DMAP (0.247; 1 mmol; 0.1 eq) were stirred in dry THF (50 mL), and (Boc)₂O (5.7 g; 26.1 mmol; 1.3 eq.) in THF (10 mL) was added dropwise and the stirring was continued for 2 hours at RT. THF was evaporated, methanol (100 mL) was added to the residue, stirred 10 minutes, the precipitate of the product was filtered off and dried to give 6.80 g (97%) of the product as a creamy powder. mp=66-68°C.

¹H NMR (CDCl₃, 700 MHz), δ ppm: 8.28 (d, J=8.2Hz, 1H, CH_{Ar}), 8.20 (d, J=9.0Hz, 1H, CH_{Ar}), 8.09 (d, J=2.0 Hz, 1H, CH_{Ar}), 7.93 (d, J=8.0 Hz, 1H, CH_{Ar}), 7.55 (dd, J₁=8.9 Hz, J₂=2.1Hz, 1H, CH_{Ar}) 7.51-7.47 (m, 1H, CH_{Ar}), 7.38-7.34 (m, 1H, CH_{Ar}), 1.76 (s, 9H, 3CH₃).

IR vₘₐₓ cm⁻¹: 3403.79(m), 2972.55(m), 2925.88(m), 2227.23(w), 1875.63(w), 1718.88(s), 1580.14(m), 14080.26(s), 1392.52(m), 1362.21(m), 1320.00(m), 1303.34(m), 1266.44(s), 1219.23(m), 1154.73(s)m 1055.44(m), 1028.29(m), 941.57(m), 837.36(m), 804.03(m), 749.07(s), 692.39(m), 644.10(m), 556.98(m), 509.85(w), 468.22(m), 410.47(m)

### tert-Butyl 3-(diphenylamino)-9H-carbazole-9-carboxylate

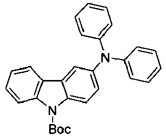

In a 50 mL flask Pd(OAc)₂ (0.027 g, (0.12 mmol; 0.08 eq.), t-Bu₃PHBF₄ (0.0175 g; 0.06 mmol; 0.04 eq.) were mixed in degassed toluene (10 mL) and stirred for 10 minutes at RT. tert-Butyl 3-bromo-9H-carbazole-9-carboxylate (0.52 g; 1.5 mmol; 1 eq.), diphenylamine (0.28 g; 1.65 mmol; 1.1 eq.) and sodium tert-butoxide (0.331 g; 3.6mmol; 2.4eq.) were added, temperature was increased to 110°C and stirred overnight. After cooling, the precipitate was filtered off and washed with toluene (15 mL). Filtrate was washed with water (2×25 mL), brine (25 mL), dried over anhydrous magnesium sulfate. Solvent was removed to give 0.63 g of crude product as a mixture of expected product and the product without Boc group. Crude mixture without purification was taken to the next stage. mp=194-195 °C.

¹H NMR (DMSO-de, 400 MHz), δ ppm: 11.29 (s, 1H, 1NH), 8.02 (d, J=8.0Hz, 1H, CH_{Ar}), 7.91 (d, J=2.0Hz, 1H, CH_{Ar}), 7.49 (t, J=8.1 Hz,2H, 2CH_{Ar}), 7.37(t, J=8.0 Hz, 1H, CH_{Ar}), 7.23 (t, J=8.0 Hz, 4H, 4CH_{Ar}), 7.17 (dd, J₁=8.5 Hz, J₂=2.1Hz, 1H, CH_{Ar}), 7.09(t, J=7.4 Hz, 1H, CH_{Ar}), 6.98 (d, J=7.8Hz, 4H, 4CH_{Ar}), 6.92 (t, J=7.4 Hz, 2H, 2CH_{Ar}).

IR vₘₐₓ cm⁻¹: 3842.43(m), 3402.04(w), 3030.56(m), 2951.47(m), 2611.07(m), 2325.23(s), 2110.05(s), 1983(s), 1940.24(s), 1871.32(s), 1940.24(m), 1738.70(s), 1707.68(m), 1584.00(w), 1738.70(s), 1707.68(m), 1584.00(w), 1483.19(w), 1271.08(w), 1236.81(w), 1174.22(m), 1152.18(m), 1109.29(m), 1072.98(m), 1026.61(m), 998.85(s), 940.17(m), 896.60(m), 870.83(m), 810.09(w), 750.82(w), 694.10(w), 638.72(w), 572.04(w), 504.95(w), 443.55(w)

### 9-(5-Bromopyridin-2-yl)-N,N-diphenyl-9H-carbazol-3-amine

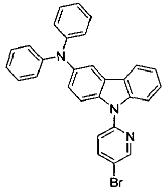

In a 100 mL flask N,N-diphenyl-9H-carbazol-3-amine (1.4 g; 4.2 mmol; 1 eq.) and potassium tert-butoxide (0.94 g; 8.4 mmol; 2 eq.) were placed in dry DMF (30 mL) and 5-bromo-2-fluoropyridine (1.48 g; 8.4 mmol; 2 eq.) was added dropwise. Temperature was increased to 110°C and stirred at this temperature for 1 hour. After cooling it was transferred to a separatory funnel, water (100 mL) was added, and the product was extracted with ethyl acetate (3×50 mL). The combined organic layers were washed with water (3×100 mL), brine (100 mL), and dried over anhydrous magnesium sulfate. Solvent was evaporated, methanol (100 mL) was added and it was stirred for 30 min at RT. The precipitate was filtered off and purified by flash chromatography (chloroform: PE - 3:7) to give 1.4 g of pure product (68%) as a light yellow powder. mp=162-164° C.

¹H NMR (CDCl₃, 700 MHz), δ ppm: 8.75 (d, J=2.5Hz, 1H, CH_{Ar}), 8.03 (dd, J₁=8.5 Hz, J₂=2.5 Hz, 1H, CH_{Ar}), 7.96 (d, J=8.0Hz, 1H, CH_{Ar}), 7.87 (s, 1H, CH_{Ar}), 7.79 (d, J=8.3Hz, 1H, CH_{Ar}), 7.77 (d, J=8.5Hz, 1H, CH_{Ar}), 7.57 (d, J=8.5 Hz, 1H, CH_{Ar}), 7.44-7.42 (m, 1H, CH_{Ar}), 7.29-7.27 (m, 2H, 2CH_{Ar}), 7.25-7.22 (m, 4H, 4CH_{Ar}), 7.12 (d, J=8.2Hz, 4H, 4CH_{Ar}), 6.98-6.96 (m, 2H,2CH_{Ar}).

IR vₘₐₓ cm⁻¹: 2923.59(m), 2857.57(m), 2227.05(w), 1917.03(w), 1833.58(w), 1708.61(m), 1658.44(w), 1589.00(m), 1482.83(s), 1455.29(s), 1377.32(m), 1315.45(m), 1271.92(s), 1219.17(m), 1159.96(m), 1081.39(m), 92868(m), 833.54(m), 809.43(m), 744.02(s), 690.70(m), 642.17(m), 559.73(m), 50-7.09(m),463.71(m), 418.14(m).

### 4-(2-(3-(Diphenyloamino)-9H-carbazol-9yl)pyridine-5-yl)benzonitrile

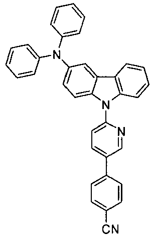

In a 100 mL flask 9-(5-bromopyridin-2-yl)-N,N-diphenyl-9H-carbazol-3-amine (0.6 g; 1.2 mmol; 1 eq.), 4-(4,4,5,5-tetramethyl-1,3,2-dioxaborolan-2-yl)benzonitrilie (0.310; 1.35 mmol; 1.1 eq.) and Pd(dppf)Cl₂·DCM (0.050 g; 0.065 mmol; 0.05 eq.) were mixed in dioxane (30 mL) and stirred at RT for 5 min. 3M aqueous K₃PO₄ solution (0.82 mL; 2.5 mmol; 2 eq.) was added, temperature was increased to 80°C and it was stirred overnight. After cooling, it was transferred to a separatory funnel, water (100 mL) was added, the product was extracted with ethyl acetate (3×50 mL). The combined organic layers were washed with water (3×100 mL), brine (100 mL) and dried over anhydrous magnesium sulfate. Solvent was removed and crude product was purified by flash chromatography (chloroform:PE - 1:9→3:7) to give 0.45 g (72%) of pure product as a light yellow powder. mp=276-278°C.

¹H NMR (CDCl₃, 700 MHz), δ ppm: 8.94 (d, J=2.2Hz, 1H, CH_{Ar}), 8.12 (dd, J₁=8.3Hz, J₂=2.5 Hz, 1H, CH_{Ar}), 7.98 (d, J=7.8 Hz, 1H, CH_{Ar}), 7.91-7.88 (m, 3H, 3CH_{Ar}), 7.83-7.82 (m, 2H, 2CH_{Ar}), 7.79-7.78 (m, 3H, 3CH_{Ar}), 7.47-7.44 (m, 1H, CH_{Ar}), 7.31-7.28 (m, 2H, 2CH_{Ar}), 7.26-7.24 (m, 4H, 4CH_{Ar}), 7.14 (d, J=8.0Hz, 4H, 4CH_{Ar}), 6.99-6.97 (m, 2H,2CH_{Ar})

IR vₘₐₓ cm⁻¹: 3672.02(w), 3404.62(w), 2922.14(m), 2225.00(m), 1982.17(w), 1872.70(w), 1709.78(w), 1588.97(m), 1482.93(s), 1454.93(s), 1376.89(m), 1272.41(s), 1228.68(m), 1151.60(m), 1072.14(m), 1040.77(m), 931.30(w), 830.16(m), 805.16(m), 752.64(s), 693.10(m), 643.03(m), 555.65(m), 507.41(m), 416.85(m).

### 9-(5-(4-((4-(5-(3-(Diphenyloamino)-9H-carbazol-9-yl)pyridine-2-yl)phenyl)sulfonyl)phenyl)pyridin-2-yl)-N,N-diphenyl-9H-carbazol-3-amine

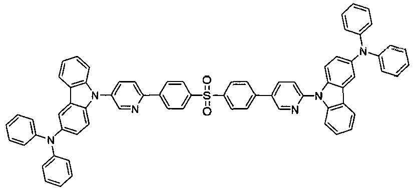

In a 50 mL flask 9-(5-bromopyridin-2-yl)-N,N-diphenyl-9H-carbazol-3-amine (0.216 g; 0.445 mmol; 2.1 eq.), 2,2'-(sulfonylbis(4,1-phenylene))bis(4,4,5,5-tetramethyl-1,3,2-dioxaborolne (0.103 g; 0.21 mmol; 1 eq.) and Pd(dppf)Cl₂×DCM (0.014 g; 16.9 mmol; 0.08 eq.) were mixed in degassed dioxane (30 mL) and stirred for 5 minutes at RT. 3M aqueous K₃PO₄ solution (0.3 mL; 0.84 mmol; 4eq.) was added, temperature was increased to 80°C and it was stirred overnight. After cooling, it was poured onto mixture of water (50 mL) and methanol (100 mL) and stirred for 15 minutes at RT. The precipitate was filtered off, washed with methanol and dried to give 0.184 g (84%) of pure product as a yellow powder. mp=310-315° C. NMR spectra were not recorded because the product is insoluble in methanol, CDCl₃, DMSO.

IR vₘₐₓ cm⁻¹: 3670.53(w), 3403.40(w), 2967.03(m), 2921.22(m), 2227.33(w), 1918.00(w), 1729.97(w), 1645.37(m), 1587.86(s), 1481.41(m), 1451.13(s), 1376.60(m), 1314.90(m), 1271.28(s), 1222.31(m), 1150.28(m), 1111.02(m),

1076.21(m), 103.41(M0, 927.86(m), 832.41(m0, 753.88(s)m 693.06(s), 641.85(m), 558.95(m), 508.54(m), 464.88(w), 408.89(w).

## Claims

1. A thermally activated delayed fluorescence (TADF) compound comprising one structure of donor-acceptor type or donor-acceptor-donor type selected from the group consisting of: 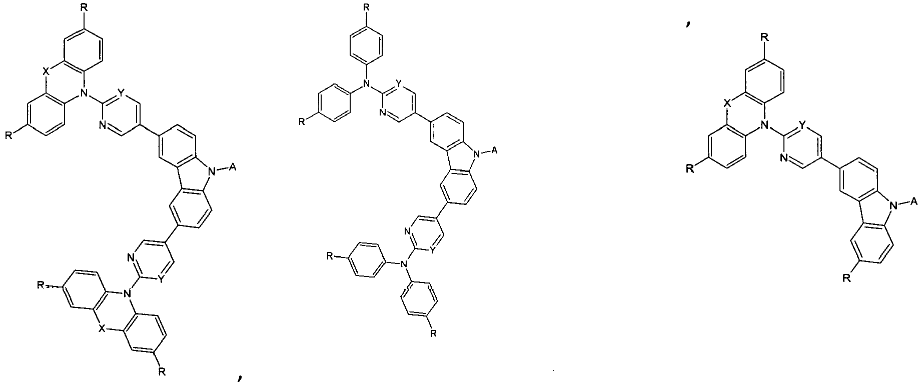 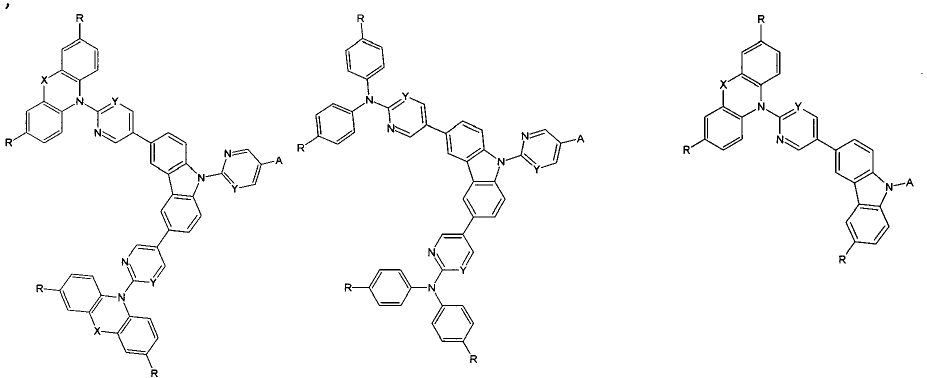 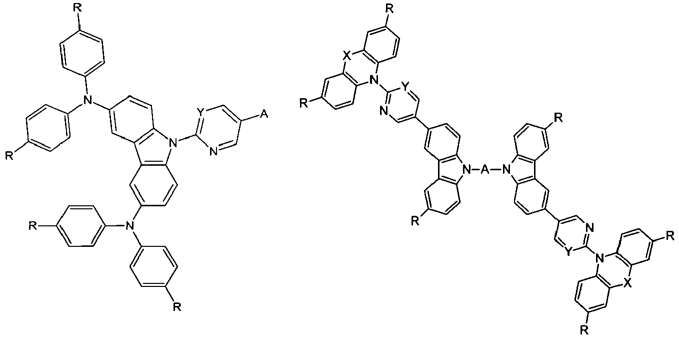 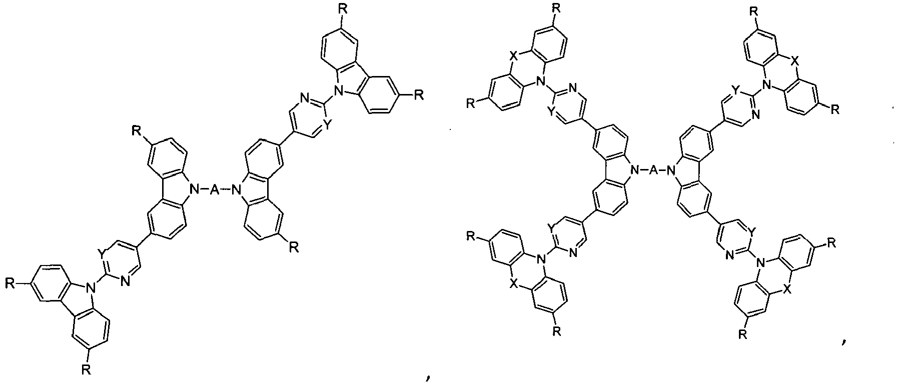 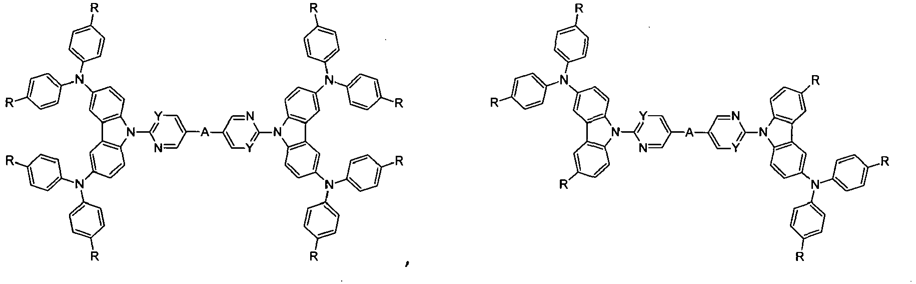 wherein:
Y is a carbon or a nitrogen atom,
X is independently selected from the group consisting of O, S, N(R) and C(CH₃)₂,
R is independently selected from the group consisting of H, alkyl, alkenyl, alkynyl, aryl and heteroaryl,
A is the acceptor moiety A for the donor-acceptor type selected from the group consisting of: cyano (CN) group, benzonitrile, phthalonitrile, isophthalonitrile, terephthalonitrile isonicotinonitrile, benzene-1,3,5-tricarbonitrile, diphenyl sulfone, benzophenone, 1,4-dibenzoylbenzene, 1,3-bis(phenylsulfonyl)benzene, 1,4-bis(phenylsulfonyl)benzene, 2,4,6-triphenyl-1,3,5-triazine, pyrimidine, 2-phenylpyrimidine, 2-methylpyrimidine, pyridine-3,5-dicarbonitrile, 4H-124-triazole, 1,3,4-oxadiazole, 1,3,4-thiadiazole, benzo[d]thiazole, benzo[1,2-d:4,5-d']bis(oxazole), benzo[1,2-d:5,4-d']bis(oxazole), quinoxaline, 1H-benzo[d]imidazole, dibenzo[f,h]quinoxaline, anthracene-9,10-dione, 9H-thioxanthene-9-one 10,10-dioxide, 9,9-dimethyl-9H-thioxanthene 10,10-dioxide, 10,10-dimethylanthracen-9(10H)-one, 5H-cyclopenta[1,2-b:5,4-b']dipyridine, 9H-fluorene-2,7-dicarbonitrile, dibenzo[f,h]quinoxaline-2,3-dicarbonitrile, thianthrene 5,5,10,10-tetraoxide, pyrazino[2,3-f][1,10]phenanthroline-2,3-dicarbonitrile, triphenylborane, tris(2,6-dimethylphenyl)borane and trimesitylborane,
A is the acceptor moiety A for the donor-acceptor-donor type selected from the group consisting of: benzonitrile, phthalonitrile, isophthalonitrile, terephthalonitrile isonicotinonitrile, benzene-1,3,5-tricarbonitrile, diphenyl sulfone, benzophenone, 1,4-dibenzoylbenzene, 1,3-bis(phenylsulfonyl)benzene, 1,4-bis(phenylsulfonyl)benzene, 2,4,6-triphenyl-1,3,5-triazine, pyrimidine, 2-phenylpyrimidine, 2-methylpyrimidine, pyridine-3,5-dicarbonitrile, 4H-124-triazole, 1,3,4-oxadiazole, 1,3,4-thiadiazole, benzo[d]thiazole, benzo[1,2-d:4,5-d']bis(oxazole), benzo[1,2-d:5,4-d']bis(oxazole), quinoxaline, 1H-benzo[d]imidazole, dibenzo[f,h]quinoxaline, anthracene-9,10-dione, 9H-thioxanthene-9-one 10,10-dioxide, 9,9-dimethyl-9H-thioxanthene 10,10-dioxide, 10,10-dimethylanthracen-9(10H)-one, 5H-cyclopenta[1,2-b:5,4-b']dipyridine, 9H-fluorene-2,7-dicarbonitrile, dibenzo[f,h]quinoxaline-2,3-dicarbonitrile, thianthrene 5,5,10,10-tetraoxide, pyrazino[2,3-f][1,10]phenanthroline-2,3-dicarbonitrile, triphenylborane, tris(2,6-dimethylphenyl)borane and trimesitylborane.

2. The TADF compound according to the claim 1, selected from the group consisting of:

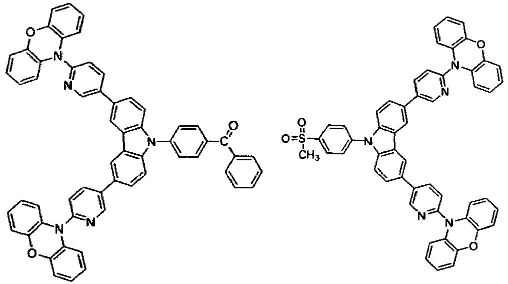

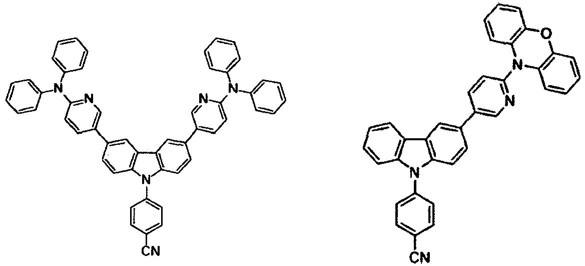

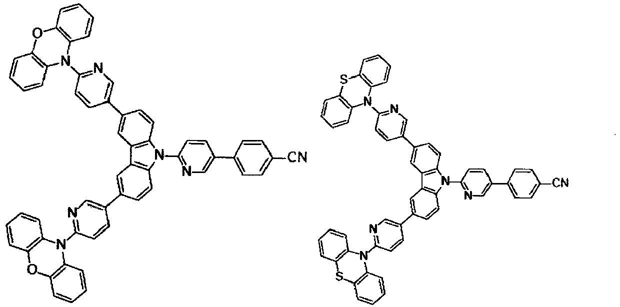

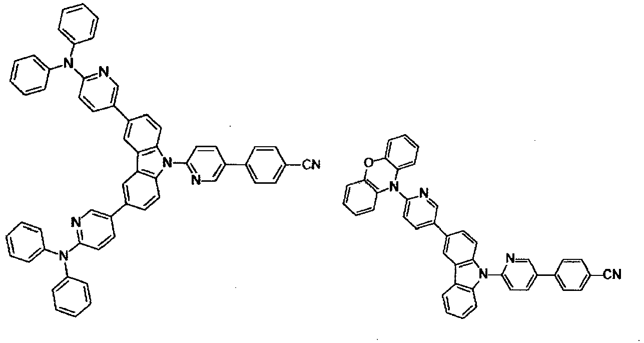

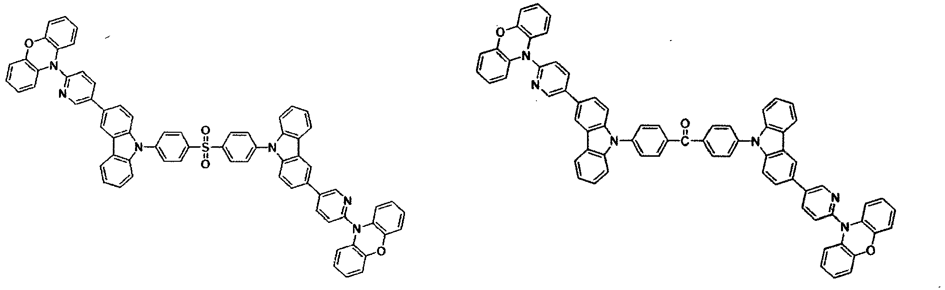

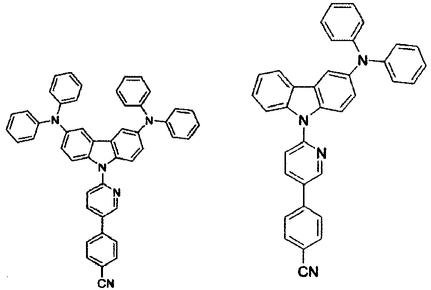

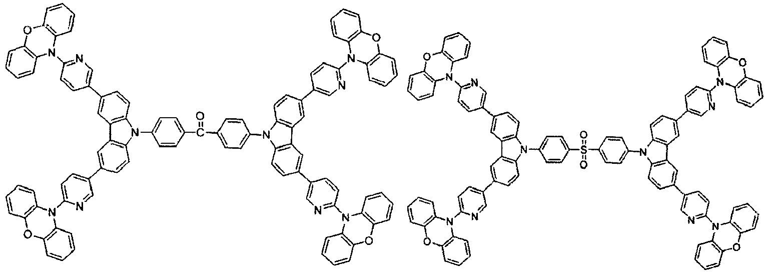

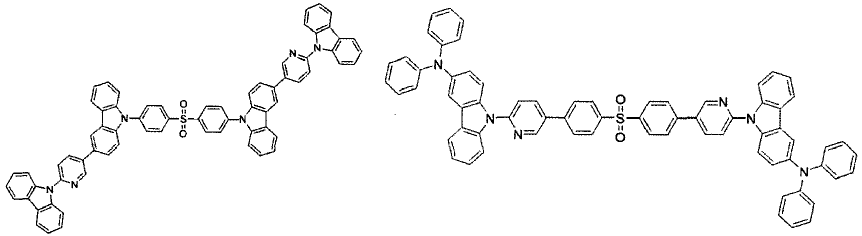

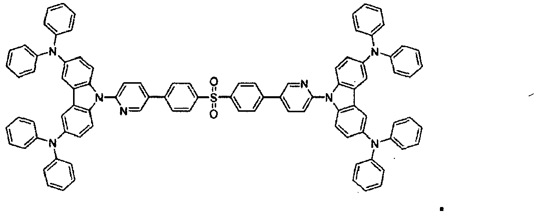

3. A light emitting device comprising the compound according to the claim 1 or 2.

4. The light emitting device according to claim 3 wherein the device comprises an emissive layer for emitting light comprising the compound according to the claim 1 or 2.

5. The light emitting device according to any of the claims 3 - 4 wherein the device comprises an anode, a cathode, an electron transport layer and a hole transport layer, and wherein at least one of the electron transport layer and the hole transport layer comprises the compound according to the claim 1 or 2.

6. The light emitting device according to any of the claims from 3-5 wherein the device comprises at least one layer selected from the group consisting of an electron injection layer, a hole injection layer, a barrier layer, a hole barrier layer and an electron barrier layer, and wherein at least one of said layers comprises the compound according to the claim 1 or 2.

7. The use of the compound according to the claim 1 or 2 as a material for thermally activated delayed fluorescence (TADF) in organic light-emitting devices (OLED).

## Patentansprüche

1. Thermisch aktivierte verzögerte Fluoreszenz (TADF)-Verbindung, umfassend eine Struktur vom Donor-Akzeptor-Typ oder Donor-Akzeptor-Donor-Typ, ausgewählt aus der Gruppe bestehend aus: 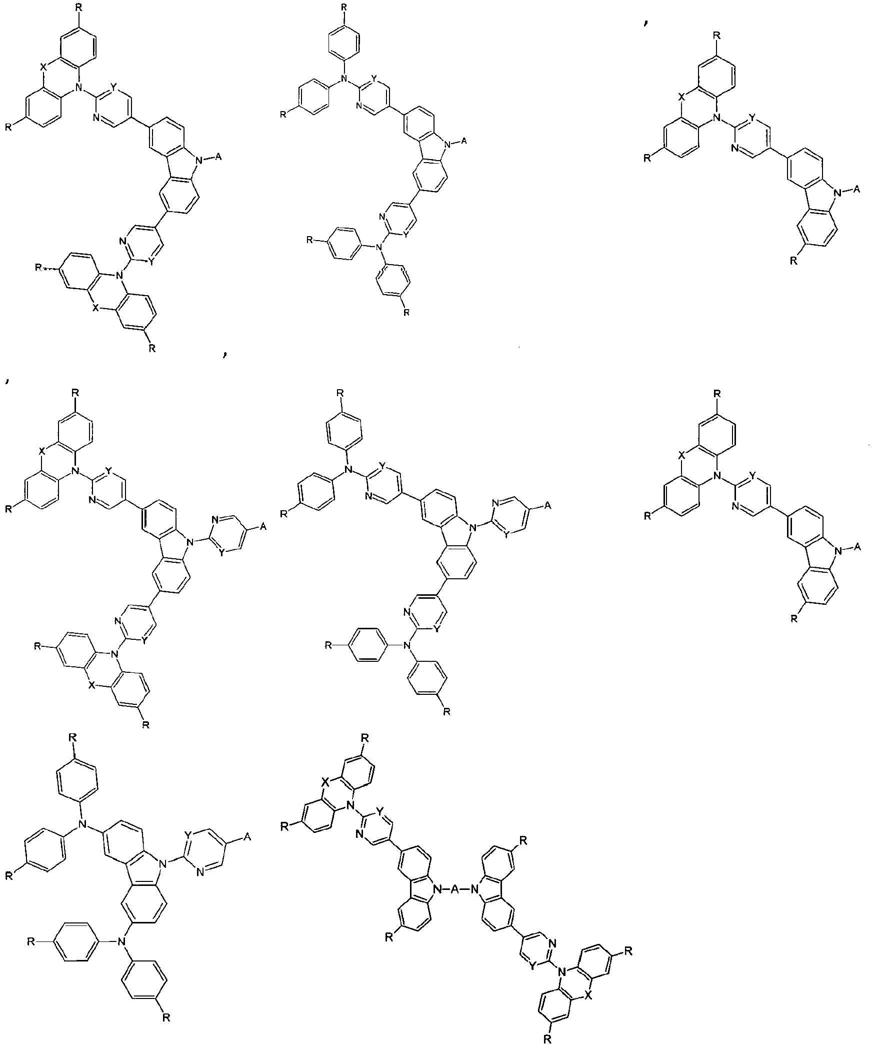 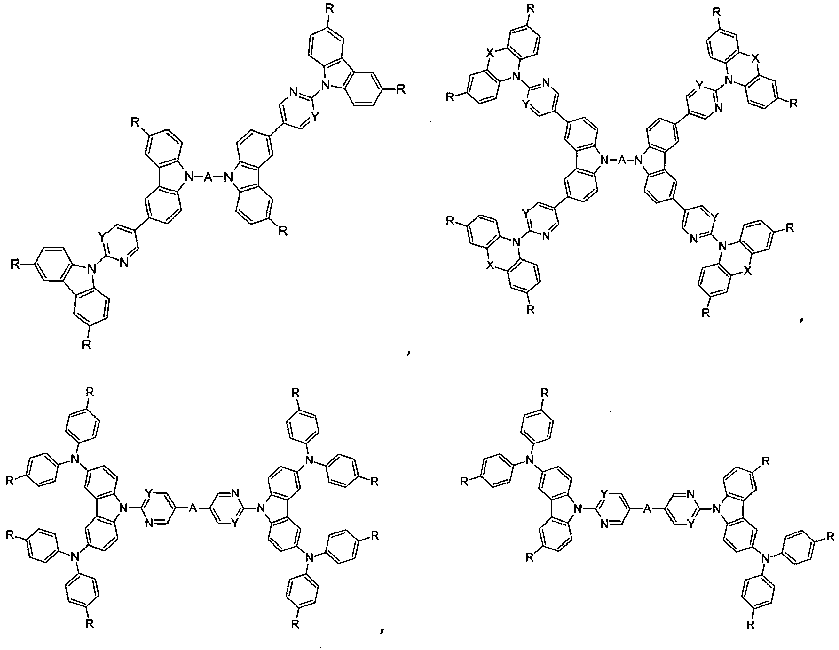 wobei:
Y ein Kohlenstoff- oder ein Stickstoffatom ist,
X unabhängig aus der Gruppe bestehend aus O, S, N(R) and C(CH₃)₂ ausgewählt wird,
R aus der Gruppe bestehend aus H, Alkyl, Alkenyl, Alkinyl, Aryl und Heteroaryl ausgewählt wird,
A ist der Akzeptorteil A für den Donor-Akzeptor-Typ ausgewählt aus der Gruppe bestehend aus: Cyano(CN)-Gruppe, Benzonitril, Phthalonitril, Isophthalonitril, Terephthalonitril, Isonicotinnitril, Benzol-1,3,5-Tricarbonitril, Diphenylsulfon, Benzophenon, 1,4-Dibenzoylbenzol, 1,3-Bis(phenylsulfonyl) benzol, 1,4- bis(phenylsulfonyl) benzol, 2,4,6-Tris(biphenyl-3-yl)-1,3,5-triazin, Pyrimidin, 2-Phenylpyrimidin, 2-Methylpyrimidin, Pyridin-3,5-dicarbonitril, 4H-124-Triazol, 1,3,4-Oxadiazol, 1,3,4-Thiadiazol, Benzo[d]thiazol, Benzo[1,2-d:4,5- d']bis(oxazol), Benzo[1,2-d:5,4-d']bis(oxazol), Chinoxalin, 1H-Benzo[d]imidazol, Dibenzo[f,h]chinoxalin, Anthracen-9,10-dion, 9 H-Thioxanthen-9-on 10,10-dioxid, 9,9-Dimethyl-9H-thioxanthen 10,10-dioxid, 10,10-Dimethylanthracen-9(10H)-on, 5H-Cyclopenta [1,2-b:5,4-b']Dipyridin, 9H- Fluoren-2,7-dicarbonitril, Dibenzo[f,h]chinoxalin-2,3-dicarbonitril, Thianthren 5,5,10,10-tetraoxid, Pyrazino [2,3-f] [1,10] phenanthrolin-2,3-dicarbonitril, Triphenylboran, Tris(2,6 -dimethylphenyl)boran und Trimethylboran,
A ist der Akzeptorteil A für den Donor-Akzeptor-Typ ausgewählt aus der Gruppe bestehend aus: Cyano(CN)-Gruppe, Benzonitril, Phthalonitril, Isophthalonitril, Terephthalonitril, Isonicotinnitril, Benzol-1,3,5-Tricarbonitril, Diphenylsulfon, Benzophenon, 1.4-Dibenzoylbenzol, 1,3-Bis(phenylsulfonyl) benzol, 1,4- bis(phenylsulfonyl) benzol, 2,4,6-Tris(biphenyl-3-yl)-1,3,5-triazin, Pyrimidin, 2-Phenylpyrimidin, 2-Methylpyrimidin, Pyridin-3,5-dicarbonitril, 4H-124-Triazol, 1.3.4-Oxadiazol, 1,3,4-Thiadiazol, Benzo[d]thiazol, Benzo[1,2-d:4,5- d']bis(oxazol), Benzo[1,2-d:5,4-d']bis(oxazol), Chinoxalin, 1H-Benzo[d]imidazol, Dibenzo[f,h]chinoxalin, Anthracen-9,10-dion, 9 H-Thioxanthen-9-on 10,10-dioxid, 9,9-Dimethyl-9H-thioxanthen 10,10-dioxid, 10.10-Dimethylanthracen-9( 10H)-on, 5H-Cyclopenta [1,2-b:5,4-b']Dipyridin, 9H- Fluoren-2,7-dicarbonitril, Dibenzo[f,h]chinoxalin-2,3-dicarbonitril,
Thianthren 5,5,10,10-tetraoxid, Pyrazino [2,3-f] [1,10] phenanthrolin-2,3-dicarbonitril, Triphenylboran, Tris(2,6 -dimethylphenyl)boran und Trimethylboran.

2. TADF-Verbindung nach Anspruch 1, ausgewählt aus der Gruppe bestehend aus:

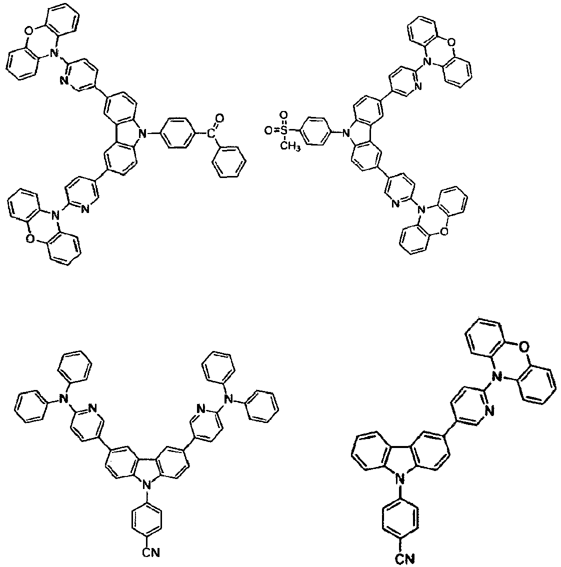

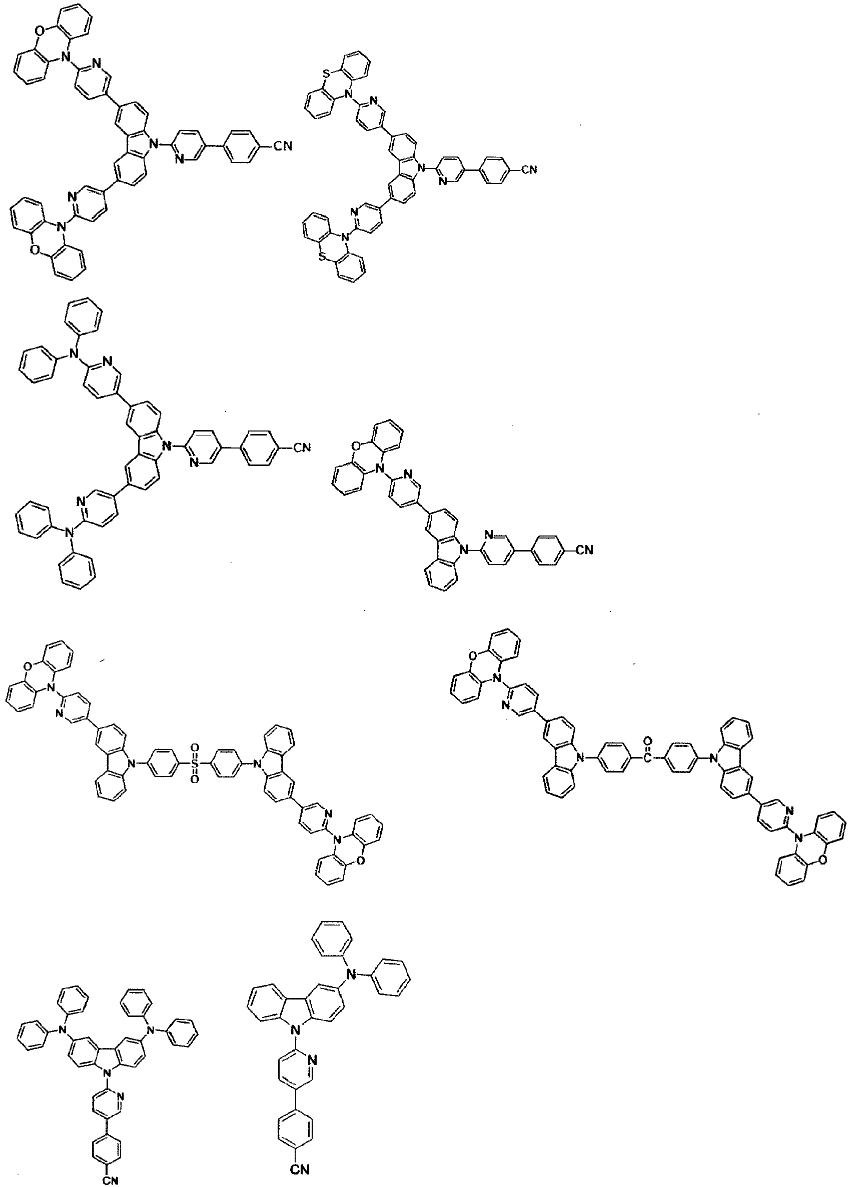

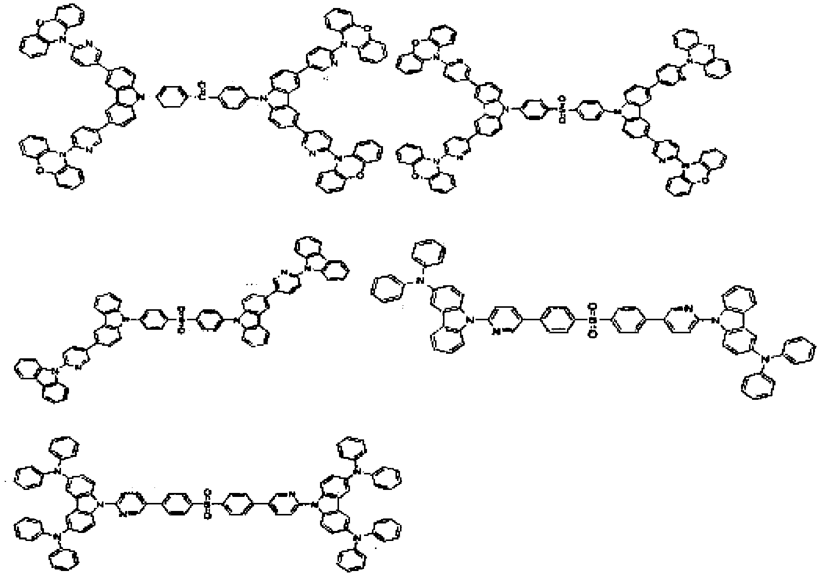

3. Lichtemittierende Vorrichtung, umfassend die Verbindung nach Anspruch 1 oder 2.

4. Lichtemittierende Vorrichtung nach Anspruch 3, wobei die Vorrichtung eine emittierende Schicht zum Emittieren von Licht umfasst, die die Verbindung nach Anspruch 1 oder 2 umfasst.

5. Lichtemittierende Vorrichtung nach einem der Ansprüche 3-4, wobei die Vorrichtung eine Anode, eine Kathode, eine Elektronentransportschicht und eine Lochtransportschicht umfasst, und wobei mindestens eine von der Elektronentransportschicht und der Lochtransportschicht die Verbindung nach Anspruch 1 oder 2 umfasst.

6. Lichtemittierende Vorrichtung nach einem der Ansprüche 3-5, wobei die Vorrichtung mindestens eine Schicht umfasst, die aus der Gruppe ausgewählt ist, die aus einer Elektroneninjektionsschicht, einer Lochinjektionsschicht, einer Barriereschicht, einer Lochbarriereschicht und einer Elektronenbarriereschicht besteht, und wobei mindestens eine der Schichten die Verbindung nach Anspruch 1 oder 2 umfasst.

7. Verwendung der Verbindung nach Anspruch 1 oder 2 als Material für thermisch aktivierte verzögerte Fluoreszenz (TADF) in organischen lichtemittierenden Vorrichtungen (OLED).

## Revendications

1. Un composé à fluorescence retardée thermiquement activée (TADF) comprenant une structure de type donneur-accepteur ou de type donneur-accepteur-donneur, sélectionnée dans le groupe constitué de : 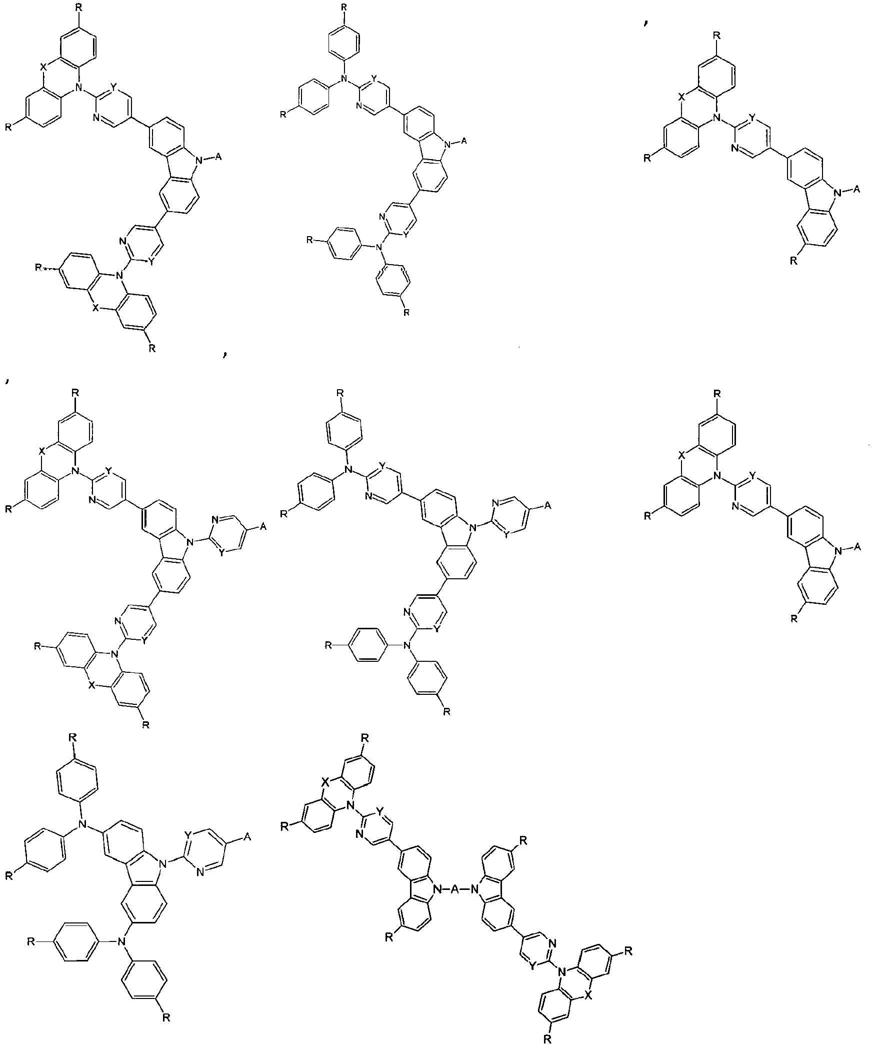 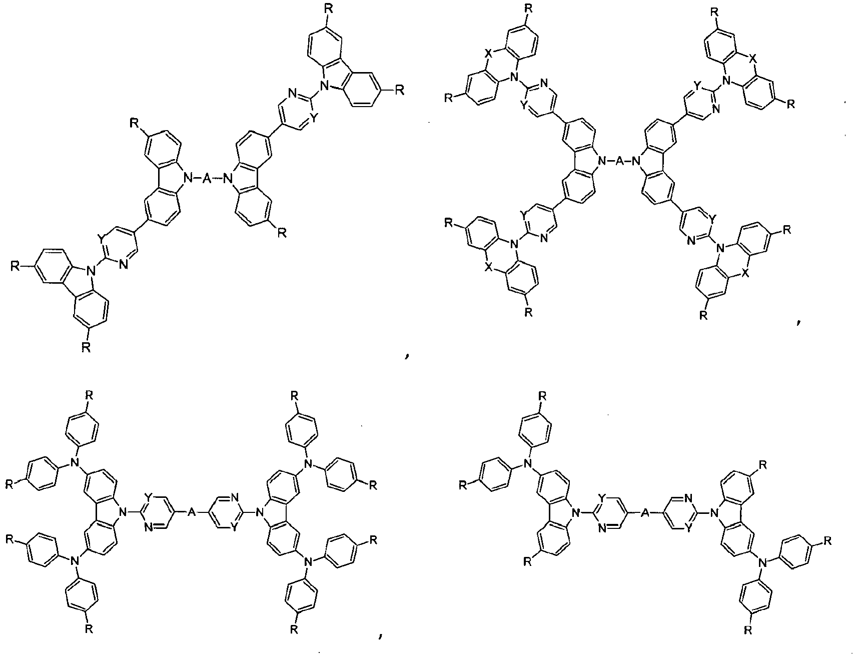 où :
Y est un atome de carbone ou d'azote,
X est indépendamment sélectionné du groupe composé de O, S, N(R) et C(CH₃)₂,
R est indépendamment sélectionné du groupe composé de H, alkyl, alcène, alkynyl, aryle et
hétéro-aryle,
A est le groupement accepteur A pour le type donneur-accepteur sélectionné dans le groupe constitué de : groupe cyano (CN), benzonitrile, phtalonitrile, isophtalonitrile, téréphtalonitrile, isonicotinonitrile, benzène-1,3,5-tricarbonitrile, diphénylsulfone, benzophénone, 1,4-dibenzoylbenzène, 1,3-bis(phénylsulfonyl)benzène, 1,4-bis(phénylsulfonyl)benzène, 2,4,6-triphényl-1,3,5-triazine, pyrimidine, 2-phénylpyrimidine, 2-méthylpyrimidine, pyridine-3,5-dicarbonitrile, 4H-124-triazole, 1,3,4-oxadiazole, 1,3,4-thiadiazole, benzo[d]thiazole, benzo[1,2-d:4,5- d']bis(oxazole), benzo[ 1,2-d:5,4-d']bis(oxazole), quinoxaline, 1H-benzo[d]imidazole, dibenzo[f,h]quinoxaline, anthracène-9,10-dione, 9 H-thioxanthène-9-one 10,10-dioxyde, 9,9-diméthyl-9H-thioxanthène 10,10-dioxyde, 10,10-diméthylanthracène-9(10H)-one, 5H-cyclopenta [1,2-b:5,4-b']dipyridine, 9H-fluorène -2,7-dicarbonitrile, dibenzo[f,h]quinoxaline-2,3-dicarbonitrile, thianthrène 5.5.10.10-tétraoxyde, pyrazino[2,3-f] [1,10]phénanthroline-2,3-dicarbonitrile, triphénylborane, tris(2,6-diméthylphényl)borane et
trimésitylborane,
A est le groupement accepteur A pour le type donneur-accepteur-donneur sélectionné dans le groupe constitué de : benzonitrile, phtalonitrile, isophtalonitrile, téréphtalonitrile, isonicotinonitrile, benzène-1,3,5-tricarbonitrile, diphénylsulfone, benzophénone, 1.4-dibenzoylbenzène, 1,3-bis(phénylsulfonyl)benzène, 1,4-bis(phénylsulfonyl)benzène, 2,4,6-triphényl-1,3,5-triazine, pyrimidine, 2-phénylpyrimidine, 2-méthylpyrimidine, pyridine-3,5-dicarbonitrile, 4H-124-triazole, 1.3.4-oxadiazole, 1,3,4-thiadiazole, benzo[d]thiazole, benzo[1,2-d:4,5- d']bis(oxazole), benzo[ 1,2-d:5,4-d']bis(oxazole), quinoxaline, 1H-benzo[d]imidazole, dibenzo[f,h]quinoxaline, anthracène-9,10-dione, 9 H-thioxanthène-9-one 10,10-dioxyde, 9,9-diméthyl-9H-thioxanthène 10,10-dioxyde, 10.10-diméthylanthracène-9(10/-/)-one, 5H-cyclopenta [1,2-b:5,4-b']dipyridine, 9H- fluorène -2,7-dicarbonitrile, dibenzo[f,h]quinoxaline-2,3-dicarbonitrile, thianthrène 5.5.10.10- tétra-oxyde, pyrazino[2,3-fJ[1,10]phénanthroline-2,3-dicarbonitrile, triphénylborane, tris(2,6-diméthylphényl)borane et
trimésitylborane.

2. Le composé TADF selon la revendication 1, sélectionné parmi le groupe composé de :

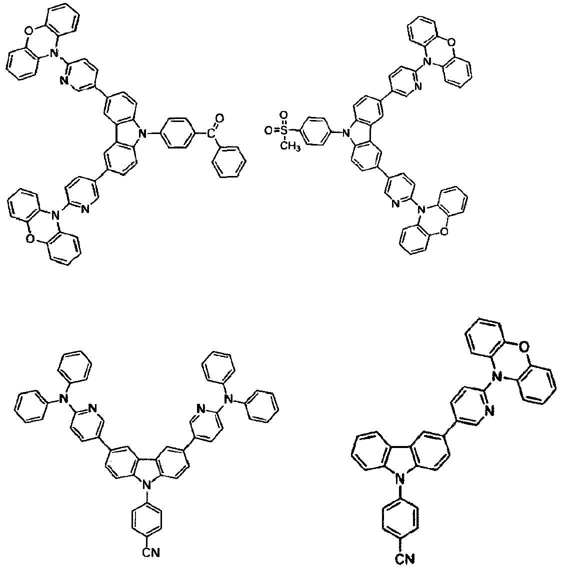

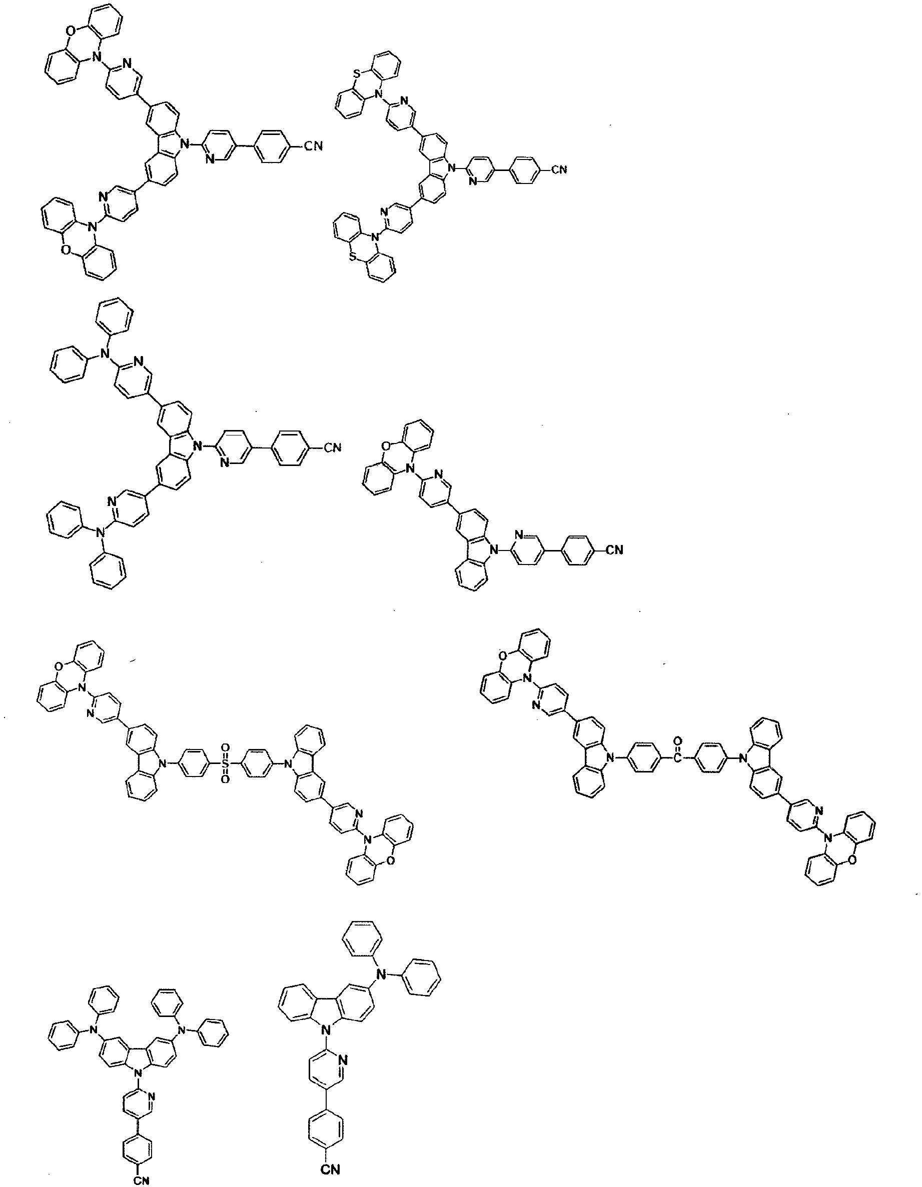

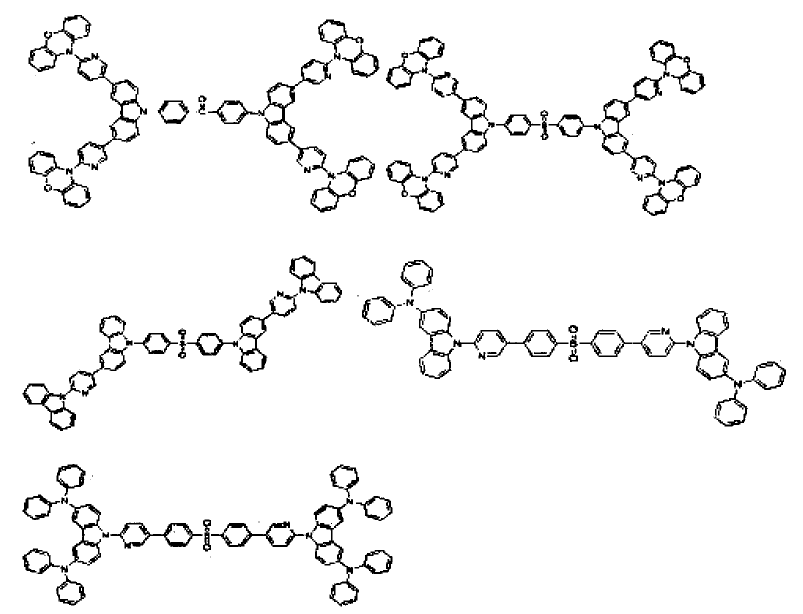

3. Un dispositif émetteur de lumière comprenant le composé selon la revendication 1 ou 2.

4. Le dispositif émetteur de lumière selon la revendication 3 dans laquelle le dispositif comprend une couche émissive pour émettre de la lumière comprenant le composé selon la revendication 1 ou 2.

5. Le dispositif émetteur de lumière selon n'importe laquelle des revendications 3 ou 4 dans laquelle le dispositif comprend une anode, une cathode, une couche de transport d'électrons ainsi qu'une couche de transport de trous, et dans laquelle au moins l'une des couches de transport d'électrons et de transport de trous comprend le composé selon la revendication 1 ou 2.

6. Le dispositif émetteur de lumière selon n'importe laquelle des revendications de 3 à 5 où le dispositif comprend au moins une couche choisie à partir du groupe composé d'une couche d'injection d'électrons, d'une couche d'injection de trous, d'une couche de blocage, d'une couche de blocage de trous ainsi que d'une couche de blocage d'électrons, où au moins l'une des dites couches comprend le composé selon la revendication 1 ou 2.

7. L'utilisation du composé selon la revendication 1 ou 2 comme matériau pour la fluorescence retardée thermiquement activée (TADF) dans les dispositifs électroluminescents organiques (OLED).
